# EUROPEAN PATENT SPECIFICATION

(11) **EP 3 717 020 B1**
(45) Date of publication and mention of the grant of the patent: **21.02.2024**
(21) Application number: 18883293.5
(22) Date of filing: 29.11.2018
(51) Int. Cl.: A61K 47/66, A61K 35/74, A61P 35/00, A61K 38/45, A61K 38/48

(54) **RAS-TARGETED THERAPEUTIC**
AUF RAS ABZIELENDES THERAPEUTIKUM
THÉRAPEUTIQUE CIBLANT RAS

(30) Priority: 30.11.2017 US 201715827595
(43) Date of publication of application: 07.10.2020
(73) Proprietor: The Hospital For Sick Children, Toronto, Ontario M5G 1X8 (CA); Northwestern University, Evanston, IL 60208 (US)
(72) Inventor: MELNYK, Roman A., Oakville Ontario L6J 3S3 (CA); AUGER, Anick, Montreal Québec H8T 1S2 (CA); BEILHARTZ, Greg, Toronto Ontario M4S 1B3 (CA); MINASSIAN, Berge, Toronto Ontario M3B 1M9 (CA); SUGIMAN-MARANGOS, Seiji, Toronto Ontario M4K 1N2 (CA); SATCHELL, Karla Fullner, Evanston Illinois (US); BIANCUCCI, Marco, Potomac Maryland (US)
(74) Representative: Schlich, George
(86) International application number: PCT/CA2018/051521
(87) International publication number: WO 2019/104433

(56) References cited:
- WO-A1-2016/191869
- WO-A1-2016/191869
- US-A1- 2016 030 531
- ANICK AUGER ET AL: "Efficient Delivery of Structurally Diverse Protein Cargo into Mammalian Cells by a Bacterial Toxin", MOLECULAR PHARMACEUTICS, vol. 12, no. 8, 1 January 2015 (2015-01-01), pages 2962-2971, XP055332136, US ISSN: 1543-8384, DOI: 10.1021/acs.molpharmaceut.5b00233
- AMY E. RABIDEAU ET AL: "Delivery of Non-Native Cargo into Mammalian Cells Using Anthrax Lethal Toxin", ACS CHEMICAL BIOLOGY, vol. 11, no. 6, 2 May 2016 (2016-05-02), pages 1490-1501, XP055635299, ISSN: 1554-8929, DOI: 10.1021/acschembio.6b00169
- ANTIC I et al.: "Site-specific processing ofRas and Rap 1 Switch I by a MARTX toxin effector domain", NATURE COMMUNICATIONS, vol. 6, no. 7396, 8 June 2015 (2015-06-08) , pages 1-10, XP055616857,

## Description

### FIELD

The present disclosure relates generally to a RAS-targeted therapeutic. More particularly, the present disclosure relates to a bacterial toxin-based RAS-targeted therapeutic.

### BACKGROUND

In contrast with small-molecule therapeutics and probes, which often readily penetrate biological membranes, larger macromolecules, such as peptides and proteins, are generally excluded from the cell interior. Given the vast array of applications for protein-based tools and therapeutics inside cells, there is great interest in developing safe and efficient protein delivery platforms that direct biologics into cells. To date, numerous approaches have been investigated to facilitate protein entry into the cytoplasm of cells, including cell-penetrating peptides, lipid-based molecules, nanoparticles, encapsulated protein containers, zinc-finger proteins, and super-charged green fluorescent proteins. Though each is capable of delivering protein cargo into cells to varying degrees, general mechanism-based limitations exist for these platforms. Cell-selectivity and/or efficiency-of-delivery remain particularly elusive features for most platforms owing to their shared nonspecific mode of interaction with membranes.

Platforms enabling targeted delivery of proteins into cells are needed to fully realize the potential of protein-based therapeutics with intracellular sites-of-action. As such, there remains a pressing need for delivery platforms with robust capacity that offer the possibility to deliver diverse protein-based therapeutics into specific cells.

Abnormal activity of the RAS subfamily of proteins has be implicated in many diseases, including cancer.
WO2016/191869, in the name of Hospital for Sick Children describes a DT-based cell delivery system having a cargo of a range of polypeptides for various therapeutic treatments. However, this document does not describe the use of the construct in relation to the treatment of cancer.
US 2016/030531, in the name of Satchell, describes RRSP and its potential use in the treatment of cancers with an aberrant Ras activity, via a range of cell delivery systems.

Therefore, there remains a need for therapeutics that effectively target one or more RAS protein.

### SUMMARY

It is an object of the present disclosure to obviate or mitigate at least one disadvantage of previous approaches.

The invention is defined by the appended claims. According to the invention there is provided a recombinant molecule for use in treatment of cancer comprising increase RAS activity in cells compared to a corresponding healthy state, the recombinant molecule comprising a cargo polypeptide (C), a diphtheria toxin enzymatic fragment (DTA), and a diphtheria toxin translocation fragment (DTB)having a general structure:

x-C-y-DTA-DTB,

wherein:
x is a polypeptide or absent,
C comprises a Ras/Rap1-specific endopeptidase (RRSP) from Vibrio vulnificus having the sequence set forth in SEQ ID No: 30,
y comprises a linker comprising (G4S)2 (SEQ ID No: 11),
DTA consists of amino acids having the sequence set forth in SEQ ID No: 26, and
DTB comprises amino acids having sequence set forth in SEQ ID No: 3,
wherein the DTA is linked to the DTB by way of a disulphide linkage.

Described but not claimed is a nucleic acid encoding the above-described recombinant molecule.

Described but not claimed is a recombinant cell comprising at least one above-described nucleic acid.

Described but not claimed is a vector comprising at least one above-described nucleic acid.

Described but not claimed is a cell transformed with the above-described vector.

Described but not claimed is a pharmaceutical composition comprising the above-described recombinant molecule, and a pharmaceutically acceptable carrier.

Described but not claimed is a method of delivering a cargo polypeptide to a cell, comprising contacting the cell with the above-described recombinant molecule.

Described but not claimed is a method of delivery a cargo polypeptide to a cell of a subject, comprising contacting the cell with the above-described recombinant molecule.

Described but not claimed is a method of delivering a cargo polypeptide across the blood brain barrier, comprising administering to a subject the above-described recombinant molecule.

Described but not claimed is a method of increasing enzyme or protein activity in a cell, comprising contacting the cell with the above-described recombinant molecule.

Described but not claimed is a method of alleviating enzyme or protein deficiency in a cell, comprising contacting the cell with the above-described recombinant molecule.

Described but not claimed is a method of treating a disease or disorder caused by enzyme or protein deficiency in a subject, comprising administering to the subject the above-described recombinant molecule.

Described but not claimed is a method of manipulating the genome of a cell, comprising contacting the cell with the above-described recombinant molecule, wherein the cargo polypeptide comprises a genome-modifying protein.

Described but not claimed is a use of the above-described recombinant molecule for delivery, or for preparation of a medicament for delivery, of the cargo polypeptide to a cell.

Described but not claimed is a use of the above-described recombinant molecule for delivery, or for preparation of a medicament for delivery, of the cargo polypeptide to a cell of a subject.

Described but not claimed is a use of the above-described recombinant molecule for delivery, or for preparation of a medicament for delivery, of the cargo polypeptide across the blood brain barrier.

Described but not claimed is a use of the above-described recombinant molecule for increasing, or for preparation of a medicament for increasing, enzyme or protein activity in a cell.

Described but not claimed is a use of the above-described recombinant molecule for alleviating, or for preparation of a medicament for alleviating, enzyme or protein deficiency in a cell.

Described but not claimed is a use of the above-described recombinant molecule for treating, or for preparation of a medicament for treating, a disease or disorder caused by enzyme or protein deficiency in a subject.

Described but not claimed is a use of the above-described recombinant molecule for manipulating the genome of a cell, wherein the cargo polypeptide comprises a genome-modifying protein.

Described but not claimed is a use for treatment of a disease or disorder caused by increased RAS activity in cells compared to a corresponding healthy state, of a recombinant molecule comprising a cargo polypeptide (C); a diphtheria toxin enzymatic fragment (DTA); and a diphtheria toxin translocation fragment (DTB), and having a general structure x-C-y-DTA-DTB, wherein x is a polypeptide or absent, C is the cargo comprises a Ras/Rap1-specific endopeptidase (RRSP) from Vibrio vulnificus, a functional variant thereof, a functional fragment thereof, or a homologue thereof, y is a polypeptide, a linker, or absent, DTA comprises amino acids having the sequence set forth in SEQ ID No: 26, and DTB comprises amino acids having the sequence set forth in SEQ ID No: 4, wherein the diphtheria toxin enzymatic fragment is linked to the diphtheria toxin translocation fragment by way of a disulphide linkage.

Described but not claimed is a use there is provided a use, for preparation of a medicament for treatment of a disease or disorder caused by increased RAS activity in cells compared to a corresponding healthy state, of a recombinant molecule comprising a cargo polypeptide (C); a diphtheria toxin enzymatic fragment (DTA); and a diphtheria toxin translocation fragment (DTB), and having a general structure x-C-y-DTA-DTB, wherein x is a polypeptide or absent, C is the cargo comprises a Ras/Rap1-specific endopeptidase (RRSP) from Vibrio vulnificus, a functional variant thereof, a functional fragment thereof, or a homologue thereof, y is a polypeptide, a linker, or absent, DTA comprises amino acids having the sequence set forth in SEQ ID No: 26, and DTB comprises amino acids having the sequence set forth in SEQ ID No: 4, wherein the diphtheria toxin enzymatic fragment is linked to the diphtheria toxin translocation fragment by way of a disulphide linkage.

Described but not claimed is a recombinant molecule for use in treatment of a disease or disorder caused by increased RAS activity in cells compared to a corresponding healthy state, the recombinant molecule comprising a cargo polypeptide (C); a diphtheria toxin enzymatic fragment (DTA); and a diphtheria toxin translocation fragment (DTB), and having a general structure x-C-y-DTA-DTB, wherein x is a polypeptide or absent, C is the cargo comprises a Ras/Rap1-specific endopeptidase (RRSP) from Vibrio vulnificus, a functional variant thereof, a functional fragment thereof, or a homologue thereof, y is a polypeptide, a linker, or absent, DTA comprises amino acids having the sequence set forth in SEQ ID No: 26, and DTB comprises amino acids having the sequence set forth in SEQ ID No: 4, wherein the diphtheria toxin enzymatic fragment is linked to the diphtheria toxin translocation fragment by way of a disulphide linkage.

Described but not claimed is a method for a method of treating a disease or disorder caused by increased RAS activity in cells compared to a corresponding healthy state comprising administering to a subject a recombinant molecule comprising a cargo polypeptide (C); a diphtheria toxin enzymatic fragment (DTA); and a diphtheria toxin translocation fragment (DTB), and having a general structure x-C-y-DTA-DTB, wherein x is a polypeptide or absent, C is the cargo comprises a Ras/Rap1-specific endopeptidase (RRSP) from Vibrio vulnificus, a functional variant thereof, a functional fragment thereof, or a homologue thereof, y is a polypeptide, a linker, or absent, DTA comprises amino acids having the sequence set forth in SEQ ID No: 26, and DTB comprises amino acids having the sequence set forth in SEQ ID No: 4, wherein the diphtheria toxin enzymatic fragment is linked to the diphtheria toxin translocation fragment by way of a disulphide linkage.

Described but not claimed is a kit for delivering a cargo polypeptide to a cell comprising the above-described recombinant molecule, and instructions for contacting the cell with the recombinant molecule.

Described but not claimed is a kit for delivering a cargo polypeptide to a cell of a subject, comprising the above-described recombinant molecule, and instructions for contacting the cell with the recombinant molecule.

Described but not claimed is a kit for delivering a cargo polypeptide across the blood brain barrier, comprising the above-described recombinant molecule, and instructions for administering the recombinant molecule to a subject.

Described but not claimed is a kit for increasing enzyme or protein activity in a cell, comprising the above-described recombinant molecule, and instructions for contacting the cell with the recombinant molecule.

Described but not claimed is a kit for alleviating enzyme or protein deficiency in a cell, comprising the above-described recombinant molecule, and instructions for contacting the cell with the recombinant molecule.

Described but not claimed is a kit for treating a disease or disorder caused by enzyme or protein deficiency in a subject, comprising the above-described recombinant molecule, and instructions for administering the recombinant molecule to the subject.

Described but not claimed is a kit for manipulating the genome of cell, comprising the above-described recombinant molecule, and instructions for contacting the cell with the recombinant molecule, wherein the cargo polypeptide comprises a genome-modifying protein.

Other aspects and features of the present disclosure will become apparent to those ordinarily skilled in the art upon review of the following description of specific embodiments in conjunction with the accompanying figures.

### BRIEF DESCRIPTION OF THE DRAWINGS

Embodiments of the present disclosure will now be described, by way of example only, with reference to the attached Figures.
**Figure 1** depicts representative structures of the three different passenger proteins: sumo protein; α-amylase; and eGFP. Arrows indicate the C-terminus of each protein.
**Figure 2** depicts a schematic of first generation chimeric fusions of different passenger proteins to the amino terminus of native diphtheria toxin (DT) via a flexible GSG linker. A' represents a catalytically active diphtheria toxin enzymatic fragment (elsewhere termed 'dtA'). 'B' represents a functional the diphtheria toxin translocation fragment (elsewhere termed 'dtB').
**Figure 3** depicts dose titration curves of chimeric constructs on cells with wt-DT, Sumo-DT, Amylase-DT, and eGFP-DT.
**Figure 4** depicts data evaluating the effect of linker size between eGFP and dtA on cells. 'dtA' represents a catalytically active diphtheria toxin enzymatic fragment.
**Figure 5** depicts the results of cell toxicity assays to measure the positional effects of dtA on inhibition of protein synthesis. 'A' (uppercase) indicates a catalytically active diphtheria toxin enzymatic fragment (dtA), while 'a' (lowercase) indicates a catalytically inactive diphtheria toxin enzymatic fragment (dta).
**Figure 6** depicts data in addition to Figure 5 to rule out the possibility that the amino terminal dtA fragment was affecting translocation. 'A' (uppercase) indicates a catalytically active diphtheria toxin enzymatic fragment (dtA), while 'a' (lowercase) indicates a catalytically inactive diphtheria toxin enzymatic fragment (dta).
**Figure 7** depicts data in addition to Figure 5 showing the same positional dependence for dtA when amylase is the passenger protein. 'A' (uppercase) indicates a catalytically active diphtheria toxin enzymatic fragment (dtA), while 'a' (lowercase) indicates a catalytically inactive diphtheria toxin enzymatic fragment (dta).
**Figure 8** depicts the results of cell toxicity assays indicating that passenger proteins reach the cytosol. 'A' (uppercase) indicates a catalytically active diphtheria toxin enzymatic fragment (dtA).
**Figure 9** depicts a time course of inhibition of protein synthesis of three constructs using 1nM of each toxin. A' (uppercase) indicates a catalytically active diphtheria toxin enzymatic fragment (dtA).
**Figure 10** depicts the results of differential scanning fluorimetry at various pH values for eGFP and mCherry.
**Figure 11** depicts pH-induced unfolding of dtA alone using differential scanning fluorimetry, with the transition midpoint of unfolding (Tm) shown across several pH values.
**Figure 12** depicts the results of cell toxicity assays indicating that mCherry is efficiently delivered into cells by DT. 'A' (uppercase) indicates a catalytically active diphtheria toxin enzymatic fragment (dtA), while 'a' (lowercase) indicates a catalytically inactive diphtheria toxin enzymatic fragment (dta).
**Figure 13** depicts the results of cell toxicity assays comparing delivery of dtA by dtB and TAT peptides.
**Figure 14** depicts the results of cell toxicity assays demonstrating that enzymatically inactive DT competes with A-eGFP-a-B, wherein 'A' (uppercase) indicates a catalytically active diphtheria toxin enzymatic fragment (dtA), eGFP indicates enhanced green fluorescent protein, 'a' (lowercase) indicates a catalytically inactive diphtheria toxin enzymatic fragment (dta), and B indicates a functional diphtheria toxin translocation fragment (elsewhere dtB).
**Figure 15** depicts the results of cell toxicity assays examining the effect of a pore-formation formation/translocation-defective mutation (L350K).
**Figure 16** depicts further data indicating that the pore-formation/translocation mutant L350K is unable to enter cells and inhibit protein synthesis.
**Figure 17** depicts data obtained with the EnzChek^{™} Ultra Amylase Assay indicating that amylase fused to DT is folded and functional.
**Figure 18** depicts the experimental design for α-amylase-DT treatment of HEK 293 cells.
**Figure 19** depicts measurements of protein-based glycogen content in HEK cells after 24h or 48h treatment normalized on content in cells treated with either DT alone or amylase alone, respectively (n=1).
**Figure 20** depicts protein-based glycogen content in HEK cells after 24 h treatment with 1.0 uM DT, amylase-DT, or amylase alone.
**Figure 21** depicts results of protein toxicity studies cells indicating proof of delivery of MeCP2e1 into the cytosol of Vero cells.
**Figure 22** depicts results of cell toxicity assays indicating proof of delivery of MeCP2e1 into the cytosol of iPSC-derived neurons from Rett Syndrome patient fibroblasts.
**Figure 23** depicts results of cell toxicity assays indicating proof of delivery of SMN into the cytosol of Vero cells.
**Figure 24** depicts results of cell toxicity assays indicating proof of delivery of FMRP into the cytosol of Vero cells.
**Figure 25** depicts results of ³H-leucine incorporation toxicity assays demonstrating delivery of PNP into the cytosol of Very cells.
**Figure 26** depicts results of ³H-leucine incorporation toxicity assays demonstrating delivery of PNP into the cytosol of two-week old wild type (WT) neurons cells.
**Figure 27** depicts results of ³H-leucine incorporation toxicity assays demonstrating delivery of Cas9 into the cytosol of Vero cells.
**Figure 28** depicts the results of NanoGlo assays demonstrating delivery of eGFP-CPD_{Vc}-DT into the cytosol of Vero-NlucP cells by fusion protein toxicity, wherein eGFP represents enhanced green fluorescent protein, CPD_{Vc} represents a cysteine protease domain from *Vibrio cholera,* and DT represents, and DT represents diphtheria toxin.
**Figure 29** depicts the results of cell viability assays to assess the effects of removing most of the DTA domain.
**Figure 30** depicts results of RRSP delivery into the cytosol of HeLa cells by a non-toxic DT(dta-dtB) as N-terminal fusion, wherein dta represents a catalytically inactive diphtheria toxin enzymatic fragment, and dtB represents a functional diphtheria toxin translocation fragment. RRSP remained active in the context of DT fusion and degraded endogenous RAS proteins at a sub-nanomolar concentration, as measured by immunoblotting with an anti-Ras antibody. At 0.25 nM of RRSP-dta-dtB, 50% of the Ras proteins were cleaved.
**Figure 31** depicts optimization of the DT delivery platform by removing the dta domain. RRSP-Δdta-dtB fusion construct increased potency by approximately 300-fold, as measured by loss of RAS proteins in HeLa cells. At 1 pM of RRSP-Δdta-dtB, 50% of RAS proteins were degraded. In RRSP-Δdta-dtB, RRSP represents Ras/Rap1-specific endopeptidase, Δdta represents a C-terminal (inactivating) deletion of the diphtheria toxin enzymatic fragment corresponding to SEQ ID NO: 26, and dtB represents a functional diphtheria toxin translocation fragment.
**Figure 32** depicts mutant RAS cleavage by RRSP-Δdta-dtB in HCT116 colorectal carcinoma cells expressing a G13D mutation. RRSP-Δdta-dtB cleaved mutant KRAS protein in HCT116 cells with high efficiency. The enzymatically inactive RRSP (H451A) was not able to degrade the RAS proteins, confirming that the loss of RAS signal was solely due to the activity of RRSP. As expected, RRSP-dtR (without the translocation domain) was not able to reach the cytosol to process RAS.
**Figure 33** depicts immunoblot detection of RAS from various cancer cell lines treated with RRSP-Δdta-dtB for 18 h. RRSP-Δdta-dtB efficiently cleaved RAS proteins in various cancer cell lines carrying most common KRAS mutations, demonstrating RRSP-Δdta-dtB as a potential treatment of malignancies.
**Figure 34** depicts effect of RRSP-Δdta-dtB on cell proliferation. BxPC3 cells containing RealTime-Glo pro-substrate and enzyme were incubated with RRSP-Δdta-dtB dilutions for 72 hours. Viable cells reduce the RealTime-Glo pro-substrate and emit luminescence. Luminescence reading was taken at 1, 4, 9, 24, 48, and 72 hr post toxin addition. Increasing concentration of RRSP-Δdta-dtB led to reduced cell proliferation.
**Figure 35** depicts a schematic of a dosing schedule in NCR nu/nu mice using intraperitoneal (i.p.) dosing.
**Figure 36** shows the normalized body weight for all 10 groups of mice (each data point represents the mean of 3 mice per group).
**Figure 37** is a Kaplan-Meier Survival plot for all 10 groups showing percentage of survivors remaining over 30 days.
**Figure 38** depicts a schematic of a xenography study.
**Figure 39** shows the average calculated tumor sizes in mice implanted with SK-N-AS cell xenografts. Each data point represents the average values of tumors measured in three mice per group

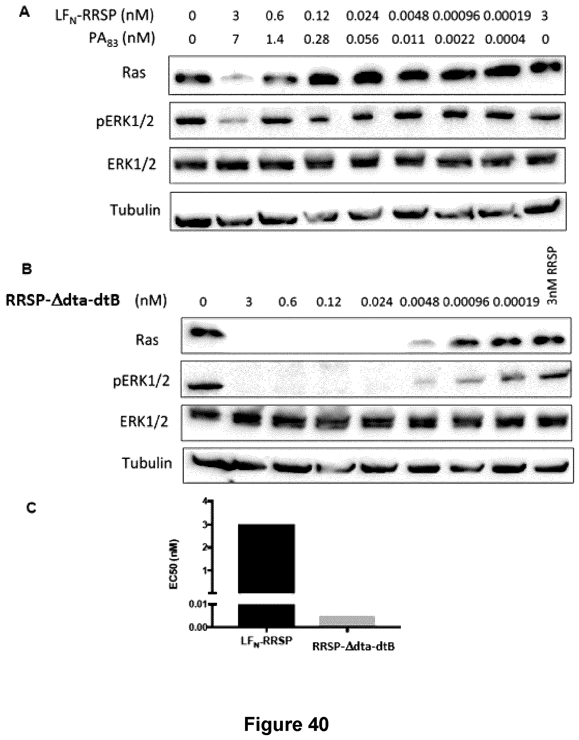

**Figure 40** depicts the result of experiments comparing DT-based delivery of RRSP to a previously described anthrax toxin-based delivery system. Panel A depicts representative immunoblots of lysates prepared from HCT116 cells treated with or without an anthrax toxin lethal factor-RRSP fusion protein (LF_{N}-RRSP) in the absence or the presence of a receptor binding moiety of the toxin (PA83) for 24 h. Panel B depicts representative immunoblots of lysates prepared from HCT116 cells treated with either RRSP-Δdta-dtB or RRSP alone for 24 h. Inactivation of Ras and ERK1/2 is shown by reduced Ras and phospho-ERK1/2 levels, respectively. RRSP delivered into cells by DT platform more effectively degraded Ras and inactivated phosphorylation of ERK1/2 than RRSP delivered by anthrax toxin. Panel C depicts EC50 values of LF_{N}-RRSP and RRSP-Δdta-dtB on HCT116 cells obtained from experiments depicted in Panels A and B.

### DETAILED DESCRIPTION

Generally, the present disclosure provides a platform for delivering cargo polypeptides into cells, which is based on a recombinant molecule comprising: a cargo polypeptide, a diphtheria toxin enzymatic fragment (DTA), and a diphtheria toxin translocation fragment (DTB). The platform may been employed to deliver diverse cargo into cells, including those having low or high molecular weights. Hyper-stable cargo polypeptide may be delivered, as well as proteins of therapeutic significance (e.g. MecP2, SMN, FMRP, PNP, and alpha-amylase). The platform may be useful in delivering genome-modifying proteins, such as the CRISPR protein, Cas9. Associated nucleic acids, pharmaceutical compositions, methods, uses, and kits are also described, including those which may be of therapeutic significance, e.g., for treating diseases or disorders caused by enzyme or protein deficiency.

For example, the present disclosure provides RAS-targeted therapeutics comprising a recombinant molecule of formula x-C-y-DTA-DTB, wherein x is a polypeptide or absent; C is a cargo polypeptide comprising Ras/Rap1-specific endopeptidase (RRSP); y comprises a polypeptide, a linker, or is absent; DTA is a diphtheria toxin enzymatic fragment comprising SEQ ID No: 26; and DTB is a diphtheria toxin translocation fragment comprising SEQ ID No: 4, wherein the DTA is linked to the DTB by a disulphide linkage. Methods and uses for treatment of a disease or disorder caused by increased RAS activity in cells are disclosed.

Protein toxins with intracellular sites-of-action are promising systems to consider as delivery platforms as they have evolved elegant and sophisticated solutions to delivering proteins across membranes and into cells. Bacterial toxins are attractive systems to consider as templates for designing protein transduction systems as they naturally bind and enter specific cells with high efficiency.

Diphtheria toxin (DT) is among the smallest and best characterized toxin of the `AB toxin' family. DT is a single chain 535-amino acid protein composed of an enzymatic A fragment (dtA) and a receptor-binding/translocation B fragment (dtB) linked through an intra-molecular disulfide bond with an intervening furin-like cleavage site. DT binds the heparin-binding epidermal growth factor-like growth factor (HB-EGF; also known as the diphtheria toxin receptor) on target cells via its C-terminal dtB domain triggering endocytosis into clathrin-coated vesicles, which are then converted into early endosomal vesicles. Upon exposure to low pH in the endosome, two hydrophobic α-helical hairpins buried within the translocation domain of dtB unfurl and insert into the endosomal membrane, creating a transmembrane pore that facilitates translocation of the catalytic dtA domain into the cytosol. Once in the cytosol, the dtA domain catalyzes the transfer of the ADP-ribose moiety of NAD+ to eukaryotic elongation factor (eEF-2), which inhibits protein synthesis, and ultimately leads to cell death.

The properties of DT have previously been exploited to make therapeutic fusion proteins; however, in most cases, the receptor-binding region of the dtB-domain was replaced with alternate domains to direct the toxic dtA-fragment to kill specific cells bearing a particular receptor¹⁻³. A fundamentally different concept is investigated herein, namely that of directing foreign proteins attached to the dtA-fragment into target cells using the receptor-binding and translocation properties of the native dtB-domain.

The first piece of evidence that suggested that DT may have the capacity to function as a vehicle for cytosolic delivery of passenger proteins came from a seminal study by Madshus et al., showing that DT could deliver an extra dtA-domain, fused as an amino-terminal extension to the existing dtA, into the cytosol⁴. In subsequent studies, it was shown that short peptides and certain small protein cargo could also be co-delivered with dtA into cells⁴⁻⁷. A recurring - yet unexplained - observation in these studies was that passenger proteins appeared to decrease the efficiency of protein delivery to different extents when the activity of the associated dtA fragment was used to measure translocation. Also, because the passenger proteins used previously have been relatively small (i.e., < 20-kDa) and expected to be largely disordered prior to and during translocation⁸, the extent to which DT could deliver proteins with properties more characteristic of typical proteins and would-be protein therapeutics is not known. Given the importance of cargo size, structure and stability in evaluating the suitability of DT as a universal protein delivery vector, an aim of this study was to resolve these questions using a number of model passenger proteins together with novel construct designs. The data presented here show that DT has great promise as an intracellular protein delivery platform, with some embodiments offering unique advantages of target cell specificity, translocation efficiency and passenger protein versatility.

The capacity of diphtheria toxin to function as an intracellular protein delivery vector is investigated. It is shown that diphtheria toxin can, in some embodiments, deliver an impressive array of passenger proteins spanning a range of sizes, structures and stabilities into cells in a manner that indicates that they are 'invisible' to the translocation machinery. Further, it is shown that α-amylase can be delivered into cells by a detoxified diphtheria toxin chimera, and that it digests intracellular glycogen in live cells, providing evidence that delivered cargo can be folded, active and abundant. The efficiency and versatility of diphtheria toxin over existing systems open numerous possibilities for intracellular delivery of bioactive proteins.

### Recombinant Molecules

In one aspect, there is provided a recombinant molecule comprising a cargo polypeptide, a diphtheria toxin enzymatic fragment (DTA), and a diphtheria toxin translocation fragment (DTB).

`DTA', as used herein, refers to the diphtheria toxin enzymatic A fragment generally, while 'DTB' refers to the receptor-binding/translocation B fragment generally.

By 'fragment' is meant a sequence of amino acids that includes the relevant domain, or a subsequence thereof from which some or all of the relevant domain has been removed. Though terms "enzymatic fragment" or "receptor-binding/translocation" are used by convention, it will be understood that some such fragments are functional, while others may have reduced function or may not be functional. For example, in the case of DTA, a 'fragment' may encompass the entirety of SEQ ID NO: 1 (dtA) or SEQ ID NO: 2 (dta), but is also to be understood as encompassing subsequences thereof.

By 'domain' is meant a particular functional and/or structural unit of a protein, often responsible for a particular function or interaction that contributes to the overall role of a protein. Protein domains may be evolutionarily conserved.

Where 'dtA' is used, it refers to a catalytically active form of DTA, unless otherwise specified. Likewise, and 'dta' is used herein to refer to the catalytically inactive form, unless otherwise specified. 'dtB', as used herein, refers to functional DTB, unless otherwise specified.

By 'catalytically active' is meant that the DTA is enzymatically active, i.e. toxic to the relevant cells. By 'catalytically inactive' is meant that the DTA is enzymatically inactive, i.e. non-toxic to the relevant cells.

The recombinant molecule may be used with a cargo polypeptide of any size. The size can be less than 1 kDa, less than 2 kDa, less than 5 kDa, less than 10 kDa, or greater than 10 kDa. The recombinant molecule may be useful for delivering cargo polypeptides of relatively large size, for example, greater than 10kDa, 20 kDa, 30 kDa, 40 kDa, 50 kDA, 60 kDA, 70 kDa, 80 kDa, 90 kDa, 100 kDa, 110 kDa, 120 kDa, 130 kDa, 140 kDa, 150 kDa, or 160 kDa. For example, the cargo polypeptide may have a molecular weight of greater than 10 kDa. The cargo polypeptide may have a molecular weight greater than 20 kDa. The cargo polypeptide may have a molecular weight greater than 30k Da. The cargo polypeptide may have a molecular weight greater than 50 kDa. The cargo polypeptide may also have a molecular weight of greater than 100 kDa. The cargo polypeptide may also have a molecular weight of greater than 150 kDa. The cargo polypeptide may be positioned at or upstream of the amino terminus of the diphtheria toxin enzymatic fragment.

The cargo polypeptide may be a modified sequence, e.g. containing chemically modified, mutated, or non-natural amino acids. For instance, the cargo polypeptides may be modified to increase stability as compared to, e.g., the unmodified or natural counterpart sequence.

In one embodiment, the recombinant molecule has a general structure: x-C-y-DTA-DTB, wherein: x is a polypeptide or absent, C is the cargo polypeptide, and y is a polypeptide, a linker, or absent. DTA can, for instance, be linked to the DTB by way of a disulphide linkage. This may be formed via a cysteine residue corresponding to the cysteine at position 186 of SEQ ID NOs: 1 or 2; and a cysteine residue corresponding to the cysteine at position 2 of SEQ ID NO: 3.

In one embodiment, y is an autoprocessing domain. Autoprocessing domains are those that effect their own cleavage. In one embodiment, an autoprocessing domain that cleaves at or near its own N-terminus, e.g. to "self clear" is desirable. Using an autoprocessing domain of this sort, cargo polypeptide may be released into the cytosol. The autoprocessing domain may comprise a cysteine protease domain (CPD). This protein family is well known. The CPD may be derived from a bacterium, such as *Vibrio cholerae* or *Clostridium difficile.* These cysteine protease domains may comprises an amino acid sequence as set forth in SEQ ID No: 20 or 21, respectively. In one embodiment, the polypeptide of y additionally comprises one or more linker.

In one embodiment, y is a linker. The linker may be an amino acid linker. When placed between a cargo polypeptide and DTA or DTB, the linker may be of sufficient length so as not to inhibit (or reduce or minimize inhibit) DTA or DTB. The linker may comprise at least 1, 2, 3, or 4 amino acid residues. The linker may comprises, e.g. at least five amino acid residues. The amino acid linker may comprise (G4S)ₙ, wherein n is 1 or greater, for instance 1 to 3. In one embodiment, n is 3.

In one embodiment, x is absent.

DTB may comprise an amino acid sequence as set forth in SEQ ID No: 3.

DTA may be catalytically active (dtA) or catalytically inactive (dta). An example of a catalytically active DTA is one comprising an amino acid sequence as set forth in SEQ ID No: 1. An example of a catalytically inactive DTA is one bearing the mutations K51E and E148K, as numbered with respect to wild type sequence. For instance, an inactive DTA may comprise an amino acid sequence as set forth in SEQ ID No: 2.

The cargo polypeptide may comprise any polypeptide for which cellular delivery is desired.

The cargo polypeptide may comprise an enzyme, or an active fragment thereof having substantially the same activity. By 'substantially the same activity' is meant that a core function of the enzyme is substantially unaltered in the fragment.

The cargo polypeptide may comprise a stably folded, or hyper stable polypeptide. By `hyper stable' is meant a polypeptide that is not susceptible to unfolding. mCherry is one example of a stably folded protein. mCherry is not susceptible to unfolding at high temperatures, i.e. of 80 degrees Celsius. The cargo polypeptide may accordingly be a polypeptide that resists unfolding up to 60, 70, 80, 90, or 100 degrees Celsius. mCherry is also stable down to pH 4. The cargo polypeptide may accordingly be a polypeptide that resists unfolding down to pH 5.0, 4.5, 4.0, 3.5, 3.0, 2.5, 2.0, 1.5, or 1.0.

The cargo polypeptide may comprise a therapeutic protein. By 'therapeutic polypeptide' is meant any protein, the cellular delivery of which could be used for a therapeutic purpose. It is well known, for example, that many human diseases or disorders are caused by or characterized by protein deficiency. Therapeutic proteins encompass proteins, the delivery of which could ameliorate or correct such a deficiency. A therapeutic protein may act to replace a protein that is deficient in the disease or disorder. A therapeutic protein may be the protein that is deficient in the disease or disorder. However, a therapeutic protein need not necessarily be identical to the protein that is deficient in the disease or disorder. For instance, a therapeutic protein may be an active fragment or modified form of a deficient protein. A therapeutic protein may also partially or fully functionally compensate for the protein deficiency underlying the disease or disorder. A therapeutic protein may also ameliorate or correct downstream or secondary effects of the cellular deficiency in a particular protein. As an example, while Lafora disease is caused e.g. by mutations in EPM2A or NHLRC1 (EPM2B), it is envisaged that delivery of an amylase, such as an alpha-amylase, as a therapeutic protein could help to reduce or clear Lafora bodies. The cargo polypeptide may comprise MecP2 (e.g. SEQ ID No: 16 or 17), SMN (e.g. SEQ ID No: 19), FMRP (e.g. SEQ ID No: 18), PNP (e.g. SEQ ID No: 24), or alpha-amylase (e.g. SEQ ID No: 15).

The modified form may comprise, e.g., a functional variant comprising one or more sequence changes that do not substantially impact function of the parent cargo or protein.

In one embodiment, the cargo protein comprises RRSP (Ras/Rap1-specific endopeptidase) from *Vibrio vulnificus,* a functional variant, a functional fragment, or a homologue thereof. In one embodiment, the cargo protein comprises RRSP (Ras/Rap1-specific endopeptidase) from *Vibrio vulnificus.* As referred to herein, the RRSP may be as encoded by SEQ ID NO: 29. The RRSP may comprise amino acids having the sequence of SEQ ID NO: 30. The RRSP may consist of amino acids having the sequence of SEQ ID NO: 30. The cargo protein may comprise a functional variant of RRSP having substantially the same function as RRSP comprising amino acids having the sequence of SEQ ID NO: 30. The cargo protein may comprise of a functional variant of RRSP having substantially the same function as RRSP consisting of amino acids having the sequence of SEQ ID NO: 30. Functional variants of RRSP, as referred to herein, may comprise sequence changes that do not substantially impact function. The variant may comprise 80%, 85%, 90%, 95%, 96%, 97%, 98%, or 99% sequence identity to SEQ ID NO: 30. The cargo protein may comprise a functional fragment of RRSP. The cargo protein may consist of a functional fragment of RRSP. Such fragments will be understood as N- or C-terminal truncations of RRSP that substantially maintain function. The cargo protein may comprise a homologue of RRSP having a homologous function in another species. Some such homologues are as disclosed in reference 29. However, homologues of RRSP, as referred to herein, could also be readily identified, e.g. by BLAST searching using SEQ ID NO: 30. Putative homologues could be tested for the ability to cleave Ras using methods described, e.g. in reference 29. Homologues, as referred to herein, may comprise proteins having amino acid sequences that are 80%, 85%, 90%, 95%, 96%, 97%, 98%, or 99% sequence identity to SEQ ID NO: 30.

An example of a recombinant molecule according to on embodiment is x-RRSP-y-DTA-DTB, wherein the constituents are as defined herein. A further Example is x-RRSP-y-dta-dtB, wherein dta comprises amino acids having SEQ ID NO: 26. A further Example is x-RRSP-y-dta-dtB, wherein dta consists of amino acids having SEQ ID NO: 26. In these constructs, x may be absent in some embodiments. In one embodiment, y is a polypeptide comprising an autoprocessing domain, e.g., as described herein. In one embodiment, y is a polypeptide comprising one or more linker, e.g., as described herein. In one embodiment, y is a polypeptide comprising both an autoprocessing domain and a linker. The linker may comprise a (G4S)₂ linker. The recombinant molecule may be the construct termed "RRSP-Δdta-dtB" and described in Example 15.

In one embodiment, there is provided a recombinant molecule comprising a cargo polypeptide (C); a diphtheria toxin enzymatic fragment (DTA); and a diphtheria toxin translocation fragment (DTB), and having a general structure

x-C-y-DTA-DTB,

wherein
x is a polypeptide or absent,
C is the cargo comprises a Ras/Rap1-specific endopeptidase (RRSP) from *Vibrio vulnificus,* a functional variant thereof, a functional fragment thereof, or a homologue thereof,
y is a polypeptide, a linker, or absent,
DTA comprises the amino acid sequence set forth in SEQ ID No: 26, and
DTB comprises the amino acid sequence set forth in SEQ ID No: 4,
wherein the diphtheria toxin enzymatic fragment is linked to the diphtheria toxin translocation fragment by way of a disulphide linkage.

In some embodiments, the DTA is catalytically inactive. In some embodiments, the DTA comprises mutations K51E and E148K, as numbered with respect to wild type sequence SEQ ID No: 1. In some embodiments, the DTA is a C-terminal fragment of SEQ ID No: 1 comprising the cysteine corresponding to position 186 of SEQ ID No: 1.

In some embodiments, the DTA consists of the amino acid sequence CAGNRVRRSVGSSL (SEQ ID No: 26).

In some embodiments, the DTB comprises the amino acid sequence set forth in SEQ ID No: 3.

In some embodiments, the DTB consists of the amino acid sequence set forth in SEQ ID No: 4.

In one embodiment, y is an autoprocessing domain. In some embodiments, the autoprocessing domain comprises a cysteine protease domain from *Clostridium difficile* or *Vibrio cholerae,* having the amino acid sequence as set forth in SEQ ID No: 20 or 21, respectively.

In one embodiment, y is a linker. The linker may be an amino acid linker. When placed between a cargo polypeptide and DTA or DTB, the linker may be of sufficient length so as not to inhibit (or reduce or minimize inhibit) DTA or DTB. The linker may comprise at least 1, 2, 3, or 4 amino acid residues. The linker may comprises, e.g. at least five amino acid residues. The amino acid linker may comprise (G4S)ₙ, wherein n is 1 or greater, for instance 1 to 3. In one embodiment, n is 3.

In some embodiments, the recombinant molecule is RRSP-Δdta-dtB, which refers to SEQ ID No: 30 - y - SEQ ID No: 25 - SEQ ID No: 3, wherein y comprises a (G4S)₂ linker. In other embodiment, y may comprise the autoprocessing domain, as described above. For example, y may comprise SEQ ID No: 20 or 21.

In some embodiments, the recombinant molecule is RRSP-Δdta-dtT, which refers to SEQ ID No: 30 - y - SEQ ID No: 25 - SEQ ID No: 4, wherein y comprises a (G4S)₂ linker. In other embodiment, y may comprise the autoprocessing domain, as described above. For example, y may comprise SEQ ID No: 20 or 21.

The cargo polypeptide comprises a genome-modifying protein. The genome-modifying protein comprises a zinc finger nuclease (ZFN), a transcription activator-like effector nuclease (TALEN), or a CRISPR (clustered regularly interspaced short palindromic repeat) protein. The CRISPR protein may be Cas9 (e.g. SEQ ID No: 22).The cargo polypeptide may comprise a complex of the genome-modifying protein and a nucleic acid, such as a guide nucleic acid. For instance, Cas9 may be complexed with a nucleic acid (such as a guide RNA), such as crRNA, trRNA, and/or sgRNA.

The amino acid sequences referred to herein encompass sequence differences compared to the references sequences (such as those set forth in Table 1, below). These may be variants, mutations, insertions, or deletions. In some applications, it may be important to ensure that the primary function of the protein is not substantially altered or abrogated, but this can be readily tested, e.g. using assays described herein. The amino acid sequences described herein may comprise a sequence of 75% or greater, 80% or greater, 85% or greater, 90% or greater, 95% or greater, or 99% or greater identity to the references sequences. The amino acid sequences may encompass conservative amino substitutions. Conservative amino acid substitutions which are known in the art are as follows with conservative substitutable candidate amino acids showing in parentheses: Ala (Gly, Ser); Arg (Gly, Gln); Asn (Gln; His); Asp (Glu); Cys (Ser); Gln (Asn, Lys); Glu (Asp); Gly (Ala, Pro); His (Asn; Gln); Ile (Leu; Val); Leu (Ile; Val); Lys (Arg; Gln); Met (Leu, Ile); Phe (Met, Leu, Tyr); Ser (Thr; Gly); Thr (Ser; Val); Trp (Tyr); Tyr (Trp; Phe); Val (Ile; Leu). Some so-called 'functional' variants, mutations, insertions, or deletions encompass sequences in which the function is substantially the same as that of the reference sequence, e.g. from which it is derived. This can be readily tested using assays similar to those described herein.

The amino acid sequences referred to herein, in particular the DT sequences may be modified for some applications. It may be desirable, for instance, to reduce the antigenicity of the fusion protein or the DT domains. They may be accomplished in a number of ways. For example, an amino acid sequence could be PEGylated. The amino acid sequence may also be mutated, e.g. to reduce antigenicity, for example by removing B- and/or T-cell epitopes. Humanization is one example mode of sequence modification.

In one embodiment, the cargo comprises an ubiquitin or a variant thereof. In one embodiment, the cargo comprises ubiquitin.

As mentioned previously, a 'variant' may encompass sequences that encompasses sequence differences with respect to a reference sequence, e.g. which may have 75% or greater, 80% or greater, 85% or greater, 90% or greater, 95% or greater, or 99% or greater identity to the references sequence. A 'variant' may also encompass amino acid substitutions, such as aforementioned conservative amino substitutions. Variants may also encompass sequence changes aimed at humanizing and/or reducing antigenicity.

In one embodiment, the cargo polypeptide comprises a therapeutic polypeptide. By 'therapeutic peptide' is meant any amino acid sequence that is delivered for a therapeutic purpose, e.g. to treat, prevent, or ameliorate a disease or pathological state.

In one embodiment, y comprises a ligation site. By 'ligation site' is meant the product of a ligation reaction. This could encompass, e.g., a particular sequence or a chemical structures that is the product of a ligation reaction. In one embodiment, the ligation site is a sortase ligation site.

In some embodiments, it may be advantageous to reduce the size of the recombinant molecule, i.e. to provide a smaller construct or lower antigenicity.

The DTA may be a subsequence of dtA or dta in some embodiments. In one embodiment, the DTA is a C-terminal fragment comprising a cysteine corresponding to the cysteine at position 186 of SEQ ID NO: 1. By 'corresponding to' is meant a position at the equivalent or cognate position when, e.g., two sequences are compared or aligned.

In one embodiment, the C-terminal fragment comprises a polypeptide having a sequence CAGNRVRRSVGSSL (SEQ ID NO: 26). In one embodiment, the C-terminal fragment consists of a polypeptide having a sequence CAGNRVRRSVGSSL (SEQ ID NO: 26). However, in some embodiments, DTA may be a different C-terminal fragment longer than SEQ ID NO: 26 but shorter than SEQ ID NOs: 1 or 2.

### Nucleic Acids, Vectors, and Cells

In one aspect, there is provided a nucleic acid encoding the above-described recombinant molecule. It will be appreciated that DTA and DTB, being separate polypeptides in the wild type diphtheria toxic linked by a disulphide bridge, may be separately encoded. Accordingly, in the nucleic acid, the DTA and DTB may be separately encoded. Separate nucleic acids encoding each of DTA and DTB may also be provided.

A skilled person would readily appreciate there are many ways to encode the above-described recombinant molecule (e.g. due to degeneracy of the genetic code), all of which are encompassed. Deletions, insertions, and substitutions may also be permitted if protein function remains substantially intact. For instance, nucleic acids may have 75% or greater, 80% or greater, 85% or greater, 90% or greater, 95% or greater, or 99% or greater identity to wild-type or references sequences may be encompassed. The above-noted nucleic acids could also be codon optimized depending on the organism or expression system in which it is intended to be expressed.

In one aspect, there is provided a recombinant cell comprising the above-described nucleic acid.

In one aspect, there is provided a vector comprising the above-described nucleic acid. Vectors suitable for propagated nucleic acid in bacterial and/or eukaryotic cells are well known in the art.

In one aspect, there is provided a cell transformed with the above-described vector. Transformation methods for obtaining such cells are well known.

### Pharmaceutical Compositions and Dosage Forms

In one aspect, there is provided a pharmaceutical composition comprising the above-described recombinant molecule and a pharmaceutically acceptable carrier. In some applications, a recombinant molecule comprising non-toxic, catalytically inactive DTA (dta) may be preferred. For example, a DTA having K51E and E148K mutations may be useful in such applications. A skilled person could generate and test other mutations, e.g. using cellular assays such as those described herein, to determine which have desirable properties in this regard. The DTA may comprise a sequence as set forth in SEQ ID No: 2. The DTA may comprise variants or modification of this sequence, such as those discussed above.

For some therapeutic applications, it may be desirable to reduce the antigenicity of the fusion protein or the DT domains. They may be accomplished in a number of ways. For example, an amino acid sequence could be PEGylated. The amino acid sequence may also be mutated, e.g. to reduce antigenicity, for example by removing B- and/or T-cell epitopes. Humanization is one example mode of sequence modification.

Pharmaceutically acceptable carriers include solvents, diluents, liquid vehicles, dispersion or suspension aids, surface active agents, isotonic agents, thickening or emulsifying agents, preservatives, solid binders, or lubricants. Carriers may be selected to prolong dwell time for sustained release appropriate to the selected route of administration. Exemplary carriers include sugars such as glucose and sucrose, starches such as corn starch and potato starch, fibers such as cellulose and its derivatives, sodium carboxymethyl cellulose, ethyl cellulose, cellulose acetate, powdered tragacanth, malt, gelatin, talc, cocoa butter, suppository waxes, oils such as peanut oil, cottonseed oil, safflower oil, sesame oil, olive oil, corn oil, and soybean oil; glycols such as propylene glycol, esters such as ethyl oleate and ethyl laurate, agar, buffering agents such as magnesium hydroxide and aluminum hydroxide, alginic acid, pyrogen-free water, isotonic saline, Ringer's solution, ethyl alcohol, phosphate buffer solutions, non-toxic compatible lubricants such as sodium lauryl sulfate and magnesium stearate, coloring agents, releasing agents, coating agents, sweeteners, flavors, perfuming agents, preservatives, and antioxidants.

Compositions can be administered to subjects through any acceptable route, such as topically (as by powders, ointments, or drops), orally, rectally, mucosally, sublingually, parenterally, intracisternally, intravaginally, intraperitoneally, bucally, ocularly, or intranasally.

Liquid dosage forms for oral administration may include emulsions, microemulsions, solutions, suspensions, syrups and elixirs. Liquid dosage forms may contain inert diluents such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils such as cottonseed, groundnut, corn, germ, olive, castor, and sesame oils, glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Dosage forms for topical or transdermal administration of an inventive pharmaceutical composition include ointments, pastes, creams, lotions, gels, powders, solutions, sprays, inhalants, or patches. The active agent is admixed under sterile conditions with a pharmaceutically acceptable carrier and any needed preservatives or buffers as may be required.

Injectable preparations, such as sterile injectable aqueous or oleaginous suspensions may be formulated according to the known art using suitable dispersing or wetting agents and suspending agents. The sterile injectable preparation may also be a sterile injectable solution, suspension or emulsion in a non-toxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized prior to addition of spores, for example, by filtration through a bacterial-retaining filter, or by incorporating sterilizing agents in the form of sterile solid compositions which can be dissolved or dispersed in sterile water or other sterile injectable medium prior to use.

It is often desirable to slow the absorption of the agent from subcutaneous or intramuscular injection. Delayed absorption of a parenterally administered active agent may be accomplished by dissolving or suspending the agent in an oil vehicle. Injectable depot forms are made by forming microencapsule matrices of the agent in biodegradable polymers such as polylactide-polyglycolide. Depending upon the ratio of active agent to polymer and the nature of the particular polymer employed, the rate of active agent release can be controlled. Examples of other biodegradable polymers include poly(orthoesters) and poly(anhydrides). Depot injectable formulations are also prepared by entrapping the agent in liposomes or microemulsions which are compatible with body tissues.

Compositions for rectal or vaginal administration are preferably suppositories which can be prepared by mixing the active agent(s) of this invention with suitable non-irritating excipients or carriers such as cocoa butter, polyethylene glycol or a suppository wax which are solid at ambient temperature but liquid at body temperature and therefore melt in the rectum or vaginal cavity and release the active agent(s).

Solid dosage forms for oral, mucosal or sublingual administration include capsules, tablets, pills, powders, and granules. In such solid dosage forms, the active agent is mixed with at least one inert, pharmaceutically acceptable excipient or carrier such as sodium citrate or dicalcium phosphate, fillers or extenders such as starches, sucrose, glucose, mannitol, and silicic acid, binders such as, for example, carboxymethylcellulose, alginates, gelatin, polyvinylpyrrolidinone, sucrose, and acacia, humectants such as glycerol, disintegrating agents such as agar-agar, calcium carbonate, potato or tapioca starch, alginic acid, certain silicates, and sodium carbonate, solution retarding agents such as paraffin, absorption accelerators such as quaternary ammonium compounds, wetting agents such as, for example, cetyl alcohol and glycerol monostearate, absorbents such as kaolin and bentonite clay, and lubricants such as talc, calcium stearate, magnesium stearate, solid polyethylene glycols, sodium lauryl sulfate, and mixtures thereof.

Solid compositions of a similar type may also be employed as fillers in soft and hard-filled gelatin capsules using such excipients as milk sugar as well as high molecular weight polyethylene glycols and the like. The solid dosage forms of tablets, capsules, pills, and granules can be prepared with coatings and shells such as enteric coatings, release controlling coatings and other coatings well known in the pharmaceutical formulating art. In such solid dosage forms the active agent(s) may be admixed with at least one inert diluent such as sucrose or starch. Such dosage forms may also comprise, as is normal practice, additional substances other than inert diluents, such as tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active agent(s) only, or preferentially, in a certain part of the intestinal tract, optionally, in a delayed manner. Examples of embedding compositions which can be used include polymeric substances and waxes.

The therapeutically effective amount may be determined on an individual basis or on the basis of the established amount necessary. The dosage for an individual subject is chosen in view of the subject to be treated. Dosage and administration may be adjusted to provide sufficient levels of the active agent(s) or to maintain the desired effect. Factors which may be taken into account include the severity of the disease state, contact with infectious agent in the past, potential future contact; age, weight, gender of the subject, diet, time and frequency of administration, drug combinations, reaction sensitivities, and tolerance/response to therapy. Sustained release compositions might be administered less frequently than fast-acting compositions.

### Methods

In one aspect, there is provided a method of delivering a cargo polypeptide to a cell, comprising contacting the cell with the above-described recombinant molecule.

In one aspect, there is provided a method of delivery a cargo polypeptide to a cell of a subject, comprising contacting the cell with the above-described recombinant molecule.

In one aspect, there is provided a method of delivering a cargo polypeptide across the blood brain barrier, comprising administering to a subject the above-described recombinant molecule.

In one aspect, there is provided a method of increasing enzyme or protein activity in a cell, comprising contacting the cell with the above-described recombinant molecule.

In one aspect, there is provided a method of alleviating enzyme or protein deficiency in a cell, comprising contacting the above-described recombinant molecule. In one embodiment, the cargo polypeptide comprises the enzyme or protein, or an active fragment thereof having substantially the same activity. In another embodiment, the cargo polypeptide compensates for the enzyme or protein deficiency.

By 'compensate', as used herein, is meant that the cargo polypeptide corrects or at least partially ameliorates the protein or enzyme deficiency, an aspect of the deficient protein or enzyme's function, or one or more of its downstream or secondary cellular effects or consequences.

In one aspect, there is provided a method of treating a disease or disorder caused by enzyme or protein deficiency in a subject, comprising administering to the subject the above-described recombinant molecule. In one embodiment, the cargo polypeptide comprises the enzyme or protein, or an active fragment thereof having substantially the same activity. In another embodiment, the cargo polypeptide compensates for the enzyme or protein deficiency. The disease or disorder may be Rett syndrome, and the cargo polypeptide may comprise MecP2 (e.g. SEQ ID No: 16 or 17). The disease or disorder may be Spinal Muscular Atrophy syndrome, and the cargo polypeptide may comprise SMN (e.g. SEQ ID No: 19). The disease or disorder may be Fragile X syndrome, and the cargo polypeptide may comprise FMRP (e.g. SEQ ID No: 18). The disease or disorder may be PNP-deficiency, and the cargo polypeptide may comprise PNP (e.g. SEQ ID No: 24). The disease or disorder may be Lafora Disease, and the cargo polypeptide may comprise alpha-amylase.

In one aspect, there is provided a method of treating a disease or disorder caused by protein over-expression, comprising administering to the subject the above-described recombinant molecule. Here, an aim may be e.g., to reduce expression of said protein, to inactivate said protein, or to increase degradation said protein.

In one embodiment the disease or disorder may be cancer. In one embodiment the cancer may be characterized by cells over-expressing one or more Ras protein (e.g., relative to comparable healthy cells). The one or more Ras protein may comprises one or more mutant Ras protein. In some embodiments, the one or more mutant Ras protein may comprise mutant forms of KRas, NRas, and/or HRas. In one embodiment, the cargo may comprise RRSP, a functional variant, a functional fragment, or a homologue thereof, as defined herein. The cargo may comprise RRSP. The cargo may consist of RRSP. A nucleic acid sequence encoding RRSP is depicted in SEQ ID NO: 29. A encoded amino acid sequence is depicted in SEQ ID NO: 30.

In one embodiment, there is provided a method of delivering RRSP, a functional variant, a functional fragment, or a homologue thereof to a cell, comprising contacting the cell with the above-described recombinant molecule, wherein the recombinant molecule comprises RRSP, a functional variant, a functional fragment or a homologue thereof. The recombinant molecule may comprise RRSP. The method may be carried out *in vitro.* The method may be carried out *in vivo.*

In one embodiment, there is provided a method of reducing levels of one or more mutant Ras protein in a cell, comprising contacting the cell with the above-described recombinant molecule, wherein the recombinant molecule comprises RRSP, a functional variant, a functional fragment, or a homologue thereof. The recombinant molecule may comprise RRSP. In some embodiments, the one or more mutant Ras protein may comprise mutant forms of KRas, NRas, and/or HRas. The method may be carried out *in vitro.* The method may be carried out *in vivo.*

In one embodiment, there is provided a method of inhibiting or reducing cell division of cells comprising increased levels of one or more mutant Ras protein, comprising contacting the cell with the above-described recombinant molecule, wherein the recombinant molecule comprises RRSP, a functional variant, a functional fragment, or a homologue thereof. The recombinant molecule may comprise RRSP. In some embodiments, the one or more mutant Ras protein may comprise mutant forms of KRas, NRas, and/or HRas. The method may be carried out *in vitro.* The method may be carried out *in vivo.*

In the methods described herein, the cargo polypeptide may have a molecular weight of less than 10 kDa, greater than 10 kDa, greater than 20 kDa, greater than 30 kDa, greater than 50 kDa, greater than 100 kDa, or greater than 150 kDa.

In one aspect, there is provided a method of manipulating the genome of a cell, comprising contacting the cell with the above-described recombinant molecule, wherein the cargo polypeptide comprises a genome-modifying protein. Genome-modifying proteins for genetic engineering are widely known. The genome-modifying protein may be, for example, a zinc finger nuclease (ZFN), a transcription activator-like effector nuclease (TALEN), or a CRISPR clustered regularly interspaced short palindromic repeat) protein. For example, the CRISPR protein may be Cas9 (e.g. SEQ ID No: 22). In some embodiments, these nucleic acids, such as guide RNAs, may be separately delivered to cells. In others, a pre-complex of protein and nucleic acid may be formed for delivery into a cell.

For applications of the above methods involving subjects or therapy, a recombinant molecule comprising non-toxic, catalytically inactive DTA (dta) may be preferred. For example, a DTA having K51E and E148K mutations may be useful in such applications. A skilled person could generate and test other mutations, e.g. using cellular assays such as those described herein, to determine which have desirable properties in this regard. The DTA may comprise a sequence as set forth in SEQ ID No: 2. The DTA may comprise variants or modification of this sequence, such as those discussed above.

For some therapeutic applications, it may be desirable to reduce the antigenicity of the fusion protein or the DT domains. They may be accomplished in a number of ways. For example, an amino acid sequence could be PEGylated. The amino acid sequence may also be mutated, e.g. to reduce antigenicity, for example by removing B- and/or T-cell epitopes. Humanization is one example mode of sequence modification.

In one embodiment, there is provided a method of treating a disease or disorder caused by increased RAS activity in cells compared to a corresponding healthy state comprising administering to a subject a recombinant molecule comprising a cargo polypeptide (C); a diphtheria toxin enzymatic fragment (DTA); and a diphtheria toxin translocation fragment (DTB), and having a general structure

x-C-y-DTA-DTB,

wherein
x is a polypeptide or absent,
C is the cargo comprises a Ras/Rap1-specific endopeptidase (RRSP) from *Vibrio vulnificus,* a functional variant thereof, a functional fragment thereof, or a homologue thereof,
y is a polypeptide, a linker, or absent,
DTA comprises amino acids having the sequence set forth in SEQ ID No: 26, and
DTB comprises amino acids having the sequence set forth in SEQ ID No: 4,
wherein the diphtheria toxin enzymatic fragment is linked to the diphtheria toxin translocation fragment by way of a disulphide linkage.

By 'disease or disorder caused by increased RAS activity in cells compared to a corresponding healthy state' will be understood any one of a number of pathological states caused by abnormal activity of a RAS subfamily member that activates a RAS/MAPK pathway. They have been termed `RASopathies'. RASopathies may arise due to inherited / germ-line mutations, or due to acquired / somatic mutations. Some diseases or disorders are discussed, for example, in C. Niemeyer (2014) Ras disease in children. Haematologica. 99(11): 1653-1662; and Prior, I.A., Lewis, P.D.; and C. Mattos (2012) (hereinafter 'Mattos 2012'). A comprehensive survey of Ras mutations in cancer. Cancer Research. 72(10): 2457-2467, both of which are incorporation by reference. A 'healthy state' may be characteristic of cell of a corresponding normal or diseased tissue.

In the context of embodiments in which the cargo protein comprises RRSP, it will be understood that the intended diseases and disorders are those caused by abnormal up-regulation or activation of a RAS subfamily member that is targeted for degradation by RRSP, for example, KRas, HRas, or NRas.

In some embodiments, said increased RAS activity is caused by an activating mutation. In some embodiment, the activating mutation is a germline mutation. In some embodiments, the activating mutation is a somatic mutation. Activation and up-regulation may be assessed vis-à-vis corresponding healthy cells.

In some embodiments, the method achieves a therapeutically effective intracellular level of RRSP in cells to which RRSP is delivered. In some embodiments, the method is for reducing the RAS activity to a normal level. A 'normal level', in this context, would be understood as a reference level from corresponding healthy cell, e.g. of the same type.

In some embodiments, the method is for inhibition of cellular proliferation.

In some embodiments, the method is for treatment of a myeloproliferative or lymphoproliferative disease or disorder. In some embodiments, the method is for treatment of a myeloproliferative or lymphoproliferative disease or disorder. In some embodiments, the method is for treatment of a dysplasia or neoplasia. In some embodiments, the method is for treatment of a cancer comprising said increased RAS activity. The cancer may be any of those that comprise activation of RAS targetable by RRSP. For example, the cancer may be any one of those described by Mattos (2012).

In some embodiments, the cancer comprises pancreatic cancer.

In some embodiments, the DTA is catalytically inactive. In some embodiments, the DTA comprises mutations K51E and E148K, as numbered with respect to wild type sequence SEQ ID No: 1. In some embodiments, the DTA comprises amino acids having the sequence set forth in SEQ ID No: 2. In some embodiments, the DTA comprises amino acids that are a C-terminal fragment of SEQ ID No: 1 or 2 and comprising the cysteine corresponding to position 186 of SEQ ID No: 1.

In some embodiments, the DTA consists of amino acids having the sequence CAGNRVRRSVGSSL (SEQ ID No: 26).

In some embodiments, the DTB comprises amino acids having the sequence set forth in SEQ ID No: 3.

In some embodiments, the DTB consists of amino acids having the sequence set forth in SEQ ID No: 4.

In one embodiment, y is an autoprocessing domain. In some embodiments, the autoprocessing domain comprises a cysteine protease domain from *Clostridium difficile* or *Vibrio cholerae,* having the amino acid sequence as set forth in SEQ ID No: 20 or 21, respectively.

In one embodiment, y is a linker. The linker may be an amino acid linker. When placed between a cargo polypeptide and DTA or DTB, the linker may be of sufficient length so as not to inhibit (or reduce or minimize inhibit) DTA or DTB. The linker may comprise at least 1, 2, 3, or 4 amino acid residues. The linker may comprises, e.g. at least five amino acid residues. The amino acid linker may comprise (G4S)ₙ, wherein n is 1 or greater, for instance 1 to 3. In one embodiment, n is 3.

In some embodiments, the recombinant molecule is RRSP-Δdta-dtB, which refers to SEQ ID No: 30 - y - SEQ ID No: 25 - SEQ ID No: 3, wherein y comprises a (G4S)₂ linker. In other embodiment, y may comprise the autoprocessing domain, as described above. For example, y may comprise SEQ ID No: 20 or 21.

In some embodiments, the recombinant molecule is RRSP-Δdta-dtT, which refers to SEQ ID No: 30 - y - SEQ ID No: 25 - SEQ ID No: 4, wherein y comprises a (G4S)₂ linker. In other embodiment, y may comprise the autoprocessing domain, as described above. For example, y may comprise SEQ ID No: 20 or 21.

In some embodiments, the step of administering comprises intraperitoneal administration.

In some embodiments, the recombinant molecule is formulated for intraperitoneal administration.

In some embodiments, the step of administering is carried out as a series of daily doses.

In some embodiments, the recombinant molecules is capable of reducing the RAS to less than 10 picomolar concentration in HCT116 cells grown *in vitro.* In some embodiments, the experimental conditions are as set forth in Example 15.

In some embodiments, the recombinant molecule is capable of producing at least 500-fold more cleavage of RAS in HCT116 cells grown *in vitro* than an anthrax toxin lethal factor-RRSP fusion protein (LF_{N}-RRSP). In some embodiments, the recombinant molecule is capable of producing at least 600-fold more cleavage of RAS in HCT116 cells grown *in vitro* than LF_{N}-RRSP. In some embodiments, the recombinant molecule is capable of producing at least 700-fold more cleavage of RAS in HCT116 cells grown *in vitro* than LF_{N}-RRSP. In some embodiments, the recombinant molecule is capable of producing at least 800-fold more cleavage of RAS in HCT116 cells grown *in vitro* than LF_{N}-RRSP. In some embodiments, the recombinant molecule is capable of producing at least 900-fold more cleavage of RAS in HCT116 cells grown *in vitro* than LF-_{N}-RRSP. In some embodiments, the recombinant molecule is capable of producing about 1000-fold more cleavage of RAS in HCT116 cells grown *in vitro* than an anthrax toxin lethal factor-RRSP fusion protein (LF_{N}-RRSP). In some embodiments, the experimental conditions are as set forth in Example 15.

### Uses

In one aspect, there is provided a use of the above-described recombinant molecule for delivery, or for preparation of a medicament for delivery, of the cargo polypeptide to a cell.

In one aspect, there is provided a use of the above-described recombinant molecule, or for preparation of a medicament for delivery, of the cargo polypeptide to a cell of a subject

In one aspect, there is provided a use of the above-described recombinant molecule for delivery, or for preparation of a medicament for delivery, of the cargo polypeptide across the blood brain barrier.

In one aspect, there is provided a use of the above-described recombinant molecule for increasing, or for preparation of a medicament for increasing, enzyme or protein activity in a cell.

In one aspect, there is provided a use of the above-described recombinant molecule for alleviating, or for preparation of a medicament for alleviating, enzyme or protein deficiency in a cell. In one embodiment, the cargo polypeptide comprises the enzyme or protein, or an active fragment thereof having substantially the same activity. In another embodiment, the cargo polypeptide compensates for the enzyme or protein deficiency.

In one aspect, there is provided a use of the above-described recombinant molecule, or for preparation of a medicament for treating, a disease or disorder caused by enzyme or protein deficiency in a subject. In one embodiment, the cargo polypeptide comprises the enzyme or protein, or an active fragment thereof having substantially the same activity. In another embodiment, the cargo polypeptide compensates for the enzyme or protein deficiency. The disease or disorder may be Rett syndrome, and the cargo polypeptide may comprise MecP2 (e.g. SEQ ID No: 16 or 17). The disease or disorder may be Spinal Muscular Atrophy syndrome, and the cargo polypeptide may comprise SMN (e.g. SEQ ID No: 19). The disease or disorder may be Fragile X syndrome, and the cargo polypeptide may comprise FMRP (e.g. SEQ ID No: 18). The disease or disorder may be PNP-deficiency, and the cargo polypeptide may comprise PNP (e.g. SEQ ID No: 24). The disease or disorder may be Lafora Disease, and the cargo polypeptide may comprise alpha-amylase (e.g. SEQ ID No: 15).

In one aspect, there is provided a use of the above-described recombinant molecule for preparation of a medicament for treatment of a disease or disorder caused by enzyme or protein over-expression. In one aspect, there is provided a use of the above-described recombinant molecule for treatment of a disease or disorder caused by enzyme or protein over-expression. In one aspect, there is provided the above-described recombinant molecule for use in treatment of a disease or disorder caused by enzyme or protein over-expression. The protein over-expressed may be a mutant form, e.g. which may not normally be present in corresponding healthy cells. The protein may be an oncogene.

In one embodiment the disease or disorder may be cancer. In one embodiment the cancer may be characterized by cells over-expressing one or more protein (e.g., relative to comparable healthy cells). The protein may be an oncogene. The oncogene may be a Ras protein. The one or more Ras protein may comprises one or more mutant Ras protein. In some embodiments, the one or more mutant Ras protein may comprise mutant KRas, NRas, and/or HRas. In one embodiment, the cargo may comprise RRSP, a functional variant, a functional fragment, or a homologue thereof. In one embodiment, the cargo protein may comprise RRSP.

In one embodiment, there is provided a use of the above-described recombinant molecule for preparation of a medicament for delivery of RRSP, a functional variant, a functional fragment, or a homologue thereof to a cell. The use may be for the delivery of RRSP. The use may be *in vitro.* The use may be *in vivo.* In one embodiment, there is provided a use of the above-described recombinant molecule for delivery of RRSP, a functional variant, a functional fragment, or a homologue thereof to a cell. The use may be for delivery of RRSP. The use may be *in vitro.* The use may be *in vivo.* In one embodiment, there is provided the above-described recombinant molecule for use in delivery of RRSP, a functional variant, a functional fragment, or a homologue thereof to a cell. The recombinant molecular may be for use in delivery of RRSP. The recombinant molecule may be for use be *in vitro.* The recombinant molecule may be for use may be *in vivo.* The delivery may provide the cargo (e.g. RRSP) at a therapeutically efficacious level.

In one embodiment, there is provided a use of the above-described recombinant molecule for preparation of a medicament for reduction of the levels of one or more mutant Ras protein in a cell, wherein the recombinant molecule comprises RRSP, a functional variant, a functional fragment, or a homologue thereof. The recombinant molecule may comprise RRSP. In one embodiment, there is provided a use of the above-described recombinant molecule for reduction of the levels of one or more mutant Ras protein in a cell, wherein the recombinant molecule comprises RRSP, a functional variant, a functional fragment, or a homologue thereof. The recombinant molecule may comprise RRSP. The use may be *in vitro.* The use may be *in vivo.* In one embodiment, there is provided the above-described recombinant molecule for use in reduction of the levels of one or more mutant Ras protein in a cell, wherein the recombinant molecule comprises RRSP, a functional variant, a functional fragment, or a homologue thereof. The recombinant molecule may comprise RRSP. The recombinant molecule may be for use *in vitro.* The recombinant molecule may be for use *in vivo.* In some embodiments, the one or more mutant Ras protein may comprise mutant KRas, NRas, and/or HRas.

In one embodiment, there is provided a use of the above-described recombinant molecule for preparation of a medicament for inhibition or reduction of cell division of cells comprising increased levels of one or more mutant Ras protein, wherein the recombinant molecule comprises RRSP, a functional variant, a functional fragment, or a homologue thereof. The recombinant molecular may comprise RRSP. In one embodiment, there is provided a use of the above-described recombinant molecule for inhibition or reduction of cell division of cells comprising increased levels of one or more mutant Ras protein, wherein the recombinant molecule comprises RRSP, a functional variant, a functional fragment, or a homologue thereof. The recombinant molecule may comprise RRSP. The use may be *in vitro.* The use may be *in vivo.* In one embodiment, there is provided the above-described recombinant molecule for use in inhibition or reduction of cell division of cells comprising increased levels of one or more mutant Ras protein, wherein the recombinant molecule comprises RRSP, a functional variant, a functional fragment, or a homologue thereof. The recombinant molecular may comprise RRSP. The recombinant molecule may be for use *in vitro.* The recombinant molecule may be for use *in vivo.* In some embodiments, the one or more mutant Ras protein may comprise mutant KRas, NRas, and/or HRas.

In the uses described herein, the cargo polypeptide may have a molecular weight of less than 10 kDa, greater than 10 kDa, greater than 20 kDa, greater than 30 kDa, greater than 50 kDa, greater than 100 kDa, or greater than 150 kDa.

In one aspect, there is provided a use of the above-described recombinant molecule for manipulating the genome of a cell, wherein the cargo polypeptide comprises a genome-modifying protein. Genome-modifying proteins for genetic engineering are widely known. The genome-modifying protein may be, for example, a zinc finger nuclease (ZFN), a transcription activator-like effector nuclease (TALEN), or a CRISPR clustered regularly interspaced short palindromic repeat) protein. For example, the CRISPR protein may be Cas9 (e.g. SEQ ID No: 22). In some embodiments, these nucleic acids, such as guide RNAs, may be separately delivered to cells. In others, a pre-complex of protein and nucleic acid may be formed for delivery into a cell.

For applications of the above uses involving subjects or therapy, a recombinant molecule comprising non-toxic, catalytically inactive DTA (dta) may be preferred. For example, a DTA having K51E and E148K mutations may be useful in such applications. A skilled person could generate and test other mutations, e.g. using cellular assays such as those described herein, to determine which have desirable properties in this regard. The DTA may comprise a sequence as set forth in SEQ ID No: 2. The DTA may comprise variants or modification of this sequence, such as those discussed above.

For some therapeutic applications, it may be desirable to reduce the antigenicity of the fusion protein or the DT domains. They may be accomplished in a number of ways. For example, an amino acid sequence could be PEGylated. The amino acid sequence may also be mutated, e.g. to reduce antigenicity, for example by removing B- and/or T-cell epitopes. Humanization is one example mode of sequence modification.

In one embodiment, there is provided a use, for treatment of a disease or disorder caused by increased RAS activity in cells compared to a corresponding healthy state, of a recombinant molecule comprising a cargo polypeptide (C); a diphtheria toxin enzymatic fragment (DTA); and a diphtheria toxin translocation fragment (DTB), and having a general structure

x-C-y-DTA-DTB,

wherein
x is a polypeptide or absent,
C is the cargo comprises a Ras/Rap1-specific endopeptidase (RRSP) from *Vibrio vulnificus,* a functional variant thereof, a functional fragment thereof, or a homologue thereof,
y is a polypeptide, a linker, or absent,
DTA comprises amino acids having the sequence set forth in SEQ ID No: 26, and
DTB comprises amino acids having the sequence set forth in SEQ ID No: 4,
wherein the diphtheria toxin enzymatic fragment is linked to the diphtheria toxin translocation fragment by way of a disulphide linkage.

In one embodiment, there is provided a use, for preparation of a medicament for treatment of a disease or disorder caused by increased RAS activity in cells compared to a corresponding healthy state, of a recombinant molecule comprising a cargo polypeptide (C); a diphtheria toxin enzymatic fragment (DTA); and a diphtheria toxin translocation fragment (DTB), and having a general structure

x-C-y-DTA-DTB,

wherein
x is a polypeptide or absent,
C is the cargo comprises a Ras/Rap1-specific endopeptidase (RRSP) from *Vibrio vulnificus,* a functional variant thereof, a functional fragment thereof, or a homologue thereof,
y is a polypeptide, a linker, or absent,
DTA comprises amino acids having the sequence set forth in SEQ ID No: 26, and
DTB comprises amino acids having the sequence set forth in SEQ ID No: 4,
wherein the diphtheria toxin enzymatic fragment is linked to the diphtheria toxin translocation fragment by way of a disulphide linkage.

In one embodiment, there is provided a recombinant molecule for use in treatment of a disease or disorder caused by increased RAS activity in cells compared to a corresponding healthy state, the recombinant molecule comprising a cargo polypeptide (C); a diphtheria toxin enzymatic fragment (DTA); and a diphtheria toxin translocation fragment (DTB), and having a general structure

x-C-y-DTA-DTB,

wherein
x is a polypeptide or absent,
C is the cargo comprises a Ras/Rap1-specific endopeptidase (RRSP) from *Vibrio vulnificus,* a functional variant thereof, a functional fragment thereof, or a homologue thereof,
y is a polypeptide, a linker, or absent,
DTA comprises amino acids having the sequence set forth in SEQ ID No: 26, and
DTB comprises amino acids having the sequence set forth in SEQ ID No: 4,
wherein the diphtheria toxin enzymatic fragment is linked to the diphtheria toxin translocation fragment by way of a disulphide linkage.

By 'disease or disorder caused by increased RAS activity in cells compared to a corresponding healthy state' will be understood any one of a number of pathological states caused by abnormal activity of a RAS subfamily member that activates a RAS/MAPK pathway. They have been termed `RASopathies'. RASopathies may arise due to inherited / germ-line mutations, or due to acquired / somatic mutations. Some diseases or disorders are discussed, for example, in C. Niemeyer (2014) Ras disease in children. Haematologica. 99(11): 1653-1662; and Prior, I.A., Lewis, P.D.; and C. Mattos (2012) (hereinafter 'Mattos 2012'). A comprehensive survey of Ras mutations in cancer. Cancer Research. 72(10): 2457-2467, both of which are incorporation by reference. A 'healthy state' may be characteristic of cell of a corresponding normal or diseased tissue.

In the context of embodiments in which the cargo protein comprises RRSP, it will be understood that the intended diseases and disorders are those caused by abnormal up-regulation or activation of a RAS subfamily member that is targeted for degradation by RRSP, for example, KRas, HRas, or NRas.

In some embodiments, said increased RAS activity is caused by an activating mutation. In some embodiment, the activating mutation is a germline mutation. In some embodiments, the activating mutation is a somatic mutation. Activation and up-regulation may be assessed vis-a-vis corresponding healthy cells.

In some embodiments, the use achieves a therapeutically effective intracellular level of RRSP in cells to which RRSP is delivered. In some embodiments, the use is for reducing the RAS activity to a normal level. A 'normal level', in this context, would be understood as a reference level from corresponding healthy cell, e.g. of the same type.

In some embodiments, the use is for inhibition of cellular proliferation.

In some embodiments, the use is for treatment of a myeloproliferative or lymphoproliferative disease or disorder. In some embodiments, the use is for treatment of a myeloproliferative or lymphoproliferative disease or disorder. In some embodiments, the use is for treatment of a dysplasia or neoplasia. In some embodiments, the use is for treatment of a cancer comprising said increased RAS activity. The cancer may be any of those that comprise activation of RAS targetable by RRSP. For example, the cancer may be any one of those described by Mattos (2012).

In some embodiments, the cancer comprises pancreatic cancer.

In some embodiments, the RAS is KRas, NRas, or HRas.

In some embodiments, the DTA is catalytically inactive. In some embodiments, the DTA comprises mutations K51E and E148K, as numbered with respect to wild type sequence SEQ ID No: 1. In some embodiments, the DTA comprises amino acids having the sequence set forth in SEQ ID No: 2. In some embodiments, the DTA comprises amino acids that are a C-terminal fragment of SEQ ID No: 1 or 2 and comprising the cysteine corresponding to position 186 of SEQ ID No: 1.

In some embodiments, the DTA consists of amino acids having the sequence CAGNRVRRSVGSSL (SEQ ID No: 26).

In some embodiments, the DTB comprises amino acids having the sequence set forth in SEQ ID No: 3.

In some embodiments, the DTB consists of amino acids having the sequence set forth in SEQ ID No: 4.

In one embodiment, y is an autoprocessing domain. In some embodiments, the autoprocessing domain comprises a cysteine protease domain from *Clostridium difficile* or *Vibrio cholerae,* having the amino acid sequence as set forth in SEQ ID No: 20 or 21, respectively.

In one embodiment, y is a linker. The linker may be an amino acid linker. When placed between a cargo polypeptide and DTA or DTB, the linker may be of sufficient length so as not to inhibit (or reduce or minimize inhibit) DTA or DTB. The linker may comprise at least 1, 2, 3, or 4 amino acid residues. The linker may comprises, e.g. at least five amino acid residues. The amino acid linker may comprise (G4S)ₙ, wherein n is 1 or greater, for instance 1 to 3. In one embodiment, n is 3.

In some embodiments, the recombinant molecule is RRSP-Δdta-dtB, which refers to SEQ ID No: 30 - y - SEQ ID No: 25 - SEQ ID No: 3, wherein y comprises a (G4S)₂ linker. In other embodiment, y may comprise the autoprocessing domain, as described above. For example, y may comprise SEQ ID No: 20 or 21.

In some embodiments, the recombinant molecule is RRSP-Δdta-dtT, which refers to SEQ ID No: 30 - y - SEQ ID No: 25 - SEQ ID No: 4, wherein y comprises a (G4S)₂ linker. In other embodiment, y may comprise the autoprocessing domain, as described above. For example, y may comprise SEQ ID No: 20 or 21.

In some embodiments, the use is intraperitoneal.

In some embodiments, the recombinant molecule is formulated for intraperitoneal administration.

In some embodiments, the use comprises a series of daily doses.

In some embodiments, the recombinant molecules is capable of reducing the RAS to less than 10 picomolar concentration in HCT116 cells grown *in vitro.* In some embodiments, the experimental conditions are as set forth in Example 15.

In some embodiments, the recombinant molecule is capable of producing at least 500-fold more cleavage of RAS in HCT116 cells grown *in vitro* than an anthrax toxin lethal factor-RRSP fusion protein (LF_{N}-RRSP). In some embodiments, the recombinant molecule is capable of producing at least 600-fold more cleavage of RAS in HCT116 cells grown *in vitro* than LF_{N}-RRSP. In some embodiments, the recombinant molecule is capable of producing at least 700-fold more cleavage of RAS in HCT116 cells grown *in vitro* than LF_{N}-RRSP. In some embodiments, the recombinant molecule is capable of producing at least 800-fold more cleavage of RAS in HCT116 cells grown *in vitro* than LF_{N}-RRSP. In some embodiments, the recombinant molecule is capable of producing at least 900-fold more cleavage of RAS in HCT116 cells grown *in vitro* than LF-_{N}-RRSP. In some embodiments, the recombinant molecule is capable of producing about 1000-fold more cleavage of RAS in HCT116 cells grown *in vitro* than an anthrax toxin lethal factor-RRSP fusion protein (LF_{N}-RRSP). In some embodiments, the experimental conditions are as set forth in Example 15.

### Kits

In one aspect, there is provided a kit for delivering a cargo polypeptide to a cell comprising the above-described recombinant molecule, and instructions for contacting the cell with the recombinant molecule.

In one aspect, there is provided a kit for delivering a cargo polypeptide to a cell of a subject, comprising the above-described recombinant molecule, and instructions for contacting the cell with the recombinant molecule.

In one aspect, there is provided a kit for delivering a cargo polypeptide across the blood brain barrier, comprising the above-described recombinant molecule, and instructions for administering the recombinant molecule to a subject.

In one aspect, there is provided a kit for increasing enzyme or protein activity in a cell, comprising the above-described recombinant molecule, and instructions for contacting the cell with the recombinant molecule.

In one aspect, there is provided a kit for alleviating enzyme or protein deficiency in a cell, comprising the above-described recombinant molecule, and instructions for contacting the cell with the recombinant molecule. In one embodiment, the cargo polypeptide comprises the enzyme or protein, or an active fragment thereof having substantially the same activity. In another embodiment, the cargo polypeptide compensates for the enzyme or protein deficiency.

In one aspect, there is provided a kit for treating a disease or disorder caused by enzyme or protein deficiency in a subject, comprising the above-described recombinant molecule, and instructions for administering the recombinant molecule to the subject. In one embodiment, the cargo polypeptide comprises the enzyme or protein, or an active fragment thereof having substantially the same activity. In another embodiment, the cargo polypeptide compensates for the enzyme or protein deficiency. The disease or disorder may be Rett syndrome, and the cargo polypeptide may comprise MecP2 (e.g. SEQ ID No: 16 or 17). The disease or disorder may be Spinal Muscular Atrophy syndrome, and the cargo polypeptide may comprise SMN (e.g. SEQ ID No: 19). The disease or disorder may be Fragile X syndrome, and the cargo polypeptide may comprise FMRP (e.g. SEQ ID No: 18). The disease or disorder may be PNP-deficiency, and the cargo polypeptide may comprise PNP (e.g. SEQ ID No: 24). The disease or disorder may be Lafora Disease, and the cargo polypeptide may comprise alpha-amylase (e.g. SEQ ID No: 15).

In one embodiment, the cargo protein comprises RRSP (Ras/Rap1-specific endopeptidase) from *Vibrio vulnificus,* a functional variant, or a homologue thereof. The RRSP may comprise SEQ ID NO: 30.

In the kits described herein, the cargo polypeptide may have a molecular weight of less than 10 kDa, greater than 10 kDa, greater than 20 kDa, greater than 30 kDa, greater than 50 kDa, greater than 100 kDa, or greater than 150 kDa.

In one aspect, there is provided a kit for manipulating the genome of cell, comprising the above-described recombinant molecule, and instructions for contacting the cell with the recombinant molecule, wherein the cargo polypeptide comprises a genome-modifying protein. Genome-modifying proteins for genetic engineering are widely known. The genome-modifying protein may be, for example, a zinc finger nuclease (ZFN), a transcription activator-like effector nuclease (TALEN), or a CRISPR clustered regularly interspaced short palindromic repeat) protein. For example, the CRISPR protein may be Cas9 (e.g. SEQ ID No: 22). In some embodiments, these nucleic acids, such as guide RNAs, may be separately delivered to cells. In others, a pre-complex of protein and nucleic acid may be formed for delivery into a cell.

For applications of the above kits involving subjects or therapy, a recombinant molecule comprising non-toxic, catalytically inactive DTA (dta) may be preferred. For example, a DTA having K51E and E148K mutations may be useful in such applications. A skilled person could generate and test other mutations, e.g. using cellular assays such as those described herein, to determine which have desirable properties in this regard. The DTA may comprise a sequence as set forth in SEQ ID No: 2. The DTA may comprise variants or modification of this sequence, such as those discussed above.

For some therapeutic applications, it may be desirable to reduce the antigenicity of the fusion protein or the DT domains. They may be accomplished in a number of ways. For example, an amino acid sequence could be PEGylated. The amino acid sequence may also be mutated, e.g. to reduce antigenicity, for example by removing B- and/or T-cell epitopes. Humanization is one example mode of sequence modification.

### EXAMPLE 1

### Generation of Cargo-DT Chimera

DT plasmid carrying the E148S mutation was a gift Dr. R. John Collier (Harvard Medical School, Boston, MA). Point mutations were made in the DT E148S plasmid using QuikChange^{™} lightning multi-mutagenesis kit (Agilent Technologies) to prepare wt-DT (E148), catalytically inactive DT (K51E/E148K), and the pore-formation defective DT (L350K). Cargo proteins were fused to different DT variants using the In-Fusion^{™} HD Cloning Kits (Clontech).

As referred to herein, dtA refers to the wildtype DTA sequence, whereas dta refers to the DTA sequence containing the inactivating mutations K51E and E148K.

Various fluorescent fusion proteins were created as DT fusion proteins. Both enhanced green fluorescent protein (eGFP) and monomeric cherry (mCherry) proteins were used in various constructs. Both eGFP-dtA-dtB and mCherry-dtA-dtB were created. EGFP and dtA were linked via a GSG linker, while mCherry and dtA were linked via a (G4S)2 linker. Further, both eGFP and mCherry were created as dtA-eGFP-dta-dtB and dtA-mCherry-dta-dtB fusion proteins, where dta contains the inactivating mutations K51E and E148K. In both cases, the dtA and cargo are linked via a GSG linker, and cargo and dta are also linked via a GSG linker. Both eGFP and mCherry contain the mutation V1G to enhance cleavage by the SUMO protease during purification in all constructs except dtA-mCherry-dta-dtB, where first residue is the native valine.

The alpha-amylase enzyme from *Bacillus megaterium* was linked to dtA via a GSG linker. A mutation was made in the alpha-amylase sequence (V1G) to enhance cleavage by the SUMO protease for purification purposes. Another construct, dtA-Amylase-dta-dtB was also made. In this case, dtA is linked to amylase via a GSG linker, and amylase is linked to dta via a GSG linker.

**Table 1** lists sequences of domains, linkers, and cargo.

**Table 1**

| **Diphtheria Toxin Sequences (Full-length DT = dtA-dtB; dtB = dtT + dtR)** | | |
|---|---|---|
| | SEQ ID | |
| dtA Domain | 1 | GADDVVDSSKSFVMENFSSYHGTKPGYVDSIQKGIQKPKSGTQGNYDDDWKGFYS TDNKYDAAGYSVDNENPLSGKAGGVVKVTYPGLTKVLALKVDNAETIKKELGLSL TEPLMEQVGTEEFIKRFGDGASRVVLSLPFAEGSSSVEYINNWEQAKALSVELEI NFETRGKRGQDAMYEYMAQACAGNRVRRSVGSSL |
| dta Domain (K51E, E148K) | 2 | GADDVVDSSKSFVMENFSSYHGTKPGYVDSIQKGIQKPKSGTQGNYDDDWEGFYS TDNKYDAAGYSVDNENPLSGKAGGVVKVTYPGLTKVLALKVDNAETIKKELGLSL TEPLMEQVGTEEFIKRFGDGASRVVLSLPFAEGSSSVKYINNWEQAKALSVELEI NFETRGKRGQDAMYEYMAQACAGNRVRRSVGSSL |
| dtB Domain | 3 | SCINLDWDVIRDKTKTKIESLKEHGPIKNKMSESPNKTVSEEKAKQYLEEFHQTA LEHPELSELKTVTGTNPVFAGANYAAWAVNVAQVIDSETADNLEKTTAALSILPG IGSVMGIADGAVHHNTEEIVAQSIALSSLMVAQAIPLVGELVDIGFAAYNFVESI INLFQVVHNSYNRPAYSPGHKTQPFLHDGYAVSWNTVEDSIIRTGFQGESGHDIK ITAENTPLPIAGVLLPTIPGKLDVNKSKTHISVNGRKIRMRCRAIDGDVTFCRPK SPVYVGNGVHANLHVAFHRSSSEKIHSNEISSDSIGVLGYQKTVDHTKVNSKLSL FFEIKSRQA |
| dtT (dtB Translocation Domain) | 4 | SCINLDWDVIRDKTKTKIESLKEHGPIKNKMSESPNKTVSEEKAKQYLEEFHQTA LEHPELSELKTVTGTNPVFAGANYAAWAVNVAQVIDSETADNLEKTTAALSILPG IGSVMGIADGAVHHNTEEIVAQSIALSSLMVAQAIPLVGELVDIGFAAYNFVESI INLFQVVHNSYNRP |
| Translocation-deficient dtT (L350K) | 5 | SCINLDWDVIRDKTKTKIESLKEHGPIKNKMSESPNKTVSEEKAKQYLEEFHQTA LEHPELSELKTVTGTNPVFAGANYAAWAVNVAQVIDSETADNLEKTTAALSILPG IGSVMGIADGAVHHNTEEIVAQSIALSSLMVAQAIPLVGEKVDIGFAAYNFVESI INLFQVVHNSYNRP |
| dtR (dtB Receptor-binding Domain) | 6 | AYSPGHKTQPFLHDGYAVSWNTVEDSIIRTGFQGESGHDIKITAENTPLPIAGVL LPTIPGKLDVNKSKTHISVNGRKIRMRCRAIDGDVTFCRPKSPVYVGNGVHANLH VAFHRSSSEKIHSNEISSDSIGVLGYQKTVDHTKVNSKLSLFFEIKSRQA |
| Δdta | 26 | CAGNRVRRSVGSSL |

| **Tag and Linker Sequences** | | |
|---|---|---|
| | SEQ ID | |
| Polyhistidine-SUMO | 7 | MGSSHHHHHHGSGLVPRGSASMSDSEVNQEAKPEVKPEVKPETHINLKVSDGSSE IFFKIKKTTPLRRLMEAFAKRQGKEMDSLRFLYDGIRIQADQTPEDLDMEDNDII EAHREQIGG |
| MYC | 8 | EQKLISEEDL |
| SV40 NLS | 9 | SPPKKKRKV |
| (G4S) linker | 10 | GGGGS |
| (G4S)₂ linker | 11 | GGGGSGGGGS |
| (G4S)₃ linker | 12 | GGGGS GGGGS GGGGS |
| GSG Linker | n/a | GSG |
| Strep Tag^{™} II | 27 | LVPRGSAWSHPQFEK |

| **Cargo Sequences** | | |
|---|---|---|
| | SEQ ID | |
| Enhanced Green Fluorescent Protein (eGFP) | 13 | GSKGEELFTGVVPILVELDGDVNGHKFSVSGEGEGDATYGKLTLKFICTTGKLPV PWPTLVTTLTYGVQCFSRYPDHMKQHDFFKSAMPEGYVQERTIFFKDDGNYKTRA EVKFEGDTLVNRIELKGIDFKEDGNILGHKLEYNYNSHNVYIMADKQKNGIKVNF KIRHNIEDGSVQLADHYQQNTPIGDGPVLLPDNHYLSTQSALSKDPNEKRDHMVL LEFVTAAGITLGMDELYK |
| Monomeric Cherry (mCherry) | 14 | GSKGEEDNMAIIKEFMRFKVHMEGSVNGHEFEIEGEGEGRPYEGTQTAKLKVTKG GPLPFAWDILSPQFMYGSKAYVKHPADIPDYLKLSFPEGFKWERVMNFEDGGVVT VTQDSSLQDGEFIYKVKLRGTNFPSDGPVMQKKTMGWEASSERMYPEDGALKGEI KQRLKLKDGGHYDAEVKTTYKAKKPVQLPGAYNVNIKLDITSHNEDYTIVEQYER AEGRHSTGGMDELYK |
| α-amylase (*B*. *megaterium)* | 15 | GHKGKSPTADKNGVFYEVYVNSFYDANKDGHGDLKGLTQKLDYLNDGNSHTKNDL QVNGIWMMPVNPSPSYHKYDVTDYYNIDPQYGNLQDFRKLMKEADKRDVKVIMDL VVNHTSSEHPWFQAALKDKNSKYRDYYIWADKNTDLNEKGSWGQQVWHKAPNGEY FYGTFWEGMPDLNYDNPEVRKEMINVGKFWLNQGVDGFRLDAALHIFKGQTPEGA KKNILWWNEFRDAMKKENPNVYLTGEVWDQPEVVAPYYQSLDSLFNFDLAGKIVS SVKAGNDQGIATAAAATDELFKSYNPNKIDGIFLTNHDQNRVMSELSGDVNKAKS AASILLTLPGNPYIYYGEEIGMTGEKPDELIREPFRWYEGNGLGQTSWETPIYNK GGNGVSIEAQTKQKDSLLNHYREMIRVRQQHEELVKGTLQSISLDQKEVVAYSRT YKGKSISVYHNISNQPIKVSVAAKGKLIFSSEKGVKKVKNQLVIPANTTILIK |
| MeCP2 (e1 isoform) | 16 | AAAAAAAPSGGGGGGEEERLEEKSEDQDLQGLKDKPLKFKKVKKDKKEEKEGKHE PVQPSAHHSAEPAEAGKAETSEGSGSAPAVPEASASPKQRRSIIRDRGPMYDDPT LPEGWTRKLKQRKSGRSAGKYDVYLINPQGKAFRSKVELIAYFEKVGDTSLDPND FDFTVTGRGSPSRREQKPPKKPKSPKAPGTGRGRGRPKGSGTTRPKAATSEGVQV KRVLEKSPGKLLVKMPFQTSPGGKAEGGGATTSTQVMVIKRPGRKRKAEADPQAI PKKRGRKPGSVVAAAAAEAKKKAVKESSIRSVQETVLPIKKRKTRETVSIEVKEV VKPLLVSTLGEKSGKGLKTCKSPGRKSKESSPKGRSSSASSPPKKEHHHHHHHSE SPKAPVPLLPPLPPPPPEPESSEDPTSPPEPQDLSSSVCKEEKMPRGGSLESDGC PKEPAKTQPAVATAATAAEKYKHRGEGERKDIVSSSMPRPNREEPVDSRTPVTER VS |
| MeCP2 (e2 isoform) | 17 | VAGMLGLREEKSEDQDLQGLKDKPLKFKKVKKDKKEEKEGKHEPVQPSAHHSAEP AEAGKAETSEGSGSAPAVPEASASPKQRRSIIRDRGPMYDDPTLPEGWTRKLKQR KSGRSAGKYDVYLINPQGKAFRSKVELIAYFEKVGDTSLDPNDFDFTVTGRGSPS RREQKPPKKPKSPKAPGTGRGRGRPKGSGTTRPKAATSEGVQVKRVLEKSPGKLL VKMPFQTSPGGKAEGGGATTSTQVMVIKRPGRKRKAEADPQAIPKKRGRKPGSVV AAAAAEAKKKAVKESSIRSVQETVLPIKKRKTRETVSIEVKEVVKPLLVSTLGEK SGKGLKTCKSPGRKSKESSPKGRSSSASSPPKKEHHHHHHHSESPKAPVPLLPPL PPPPPEPESSEDPTSPPEPQDLSSSVCKEEKMPRGGSLESDGCPKEPAKTQPAVA TAATAAEKYKHRGEGERKDIVSSSMPRPNREEPVDSRTPVTERVS |
| FMRP | 18 | EELVVEVRGSNGAFYKAFVKDVHEDSITVAFENNWQPDRQIPFHDVRFPPPVGYN KDINESDEVEVYSRANEKEPCCWWLAKVRMIKGEFYVIEYAACDATYNEIVTIER LRSVNPNKPATKDTFHKIKLDVPEDLRQMCAKEAAHKDFKKAVGAFSVTYDPENY QLVILSINEVTSKRAHMLIDMHFRSLRTKLSLIMRNEEASKQLESSRQLASRFHE QFIVREDLMGLAIGTHGANIQQARKVPGVTAIDLDEDTCTFHIYGEDQDAVKKAR SFLEFAEDVIQVPRNLVGKVIGKNGKLIQEIVDKSGVVRVRIEAENEKNVPQEEE IMPPNSLPSNNSRVGPNAPEEKKHLDIKENSTHFSQPNSTKVQRGMVPFVFVGTK DSIANATVLLDYHLNYLKEVDQLRLERLQIDEQLRQIGASSRPPPNRTDKEKSYV TDDGQGMGRGSRPYRNRGHGRRGPGYTSAPTEEERESFLRRGDGRRRGGGRGQG GRGRGGGFKGNDDHSRTDNRPRNPREAKGRTTDGSLQIRVDCNNERSVHTKTLQN |
| | | TSSEGSRLRTGKDRNQKKEKPDSVDGQQPLVNGVP |
| SMN | 19 | MAMSSGGSGGGVPEQEDSVLFRRGTGQSDDSDIWDDTALIKAYDKAVASFKHALK NGDICETSGKPKTTPKRKPAKKNKSQKKNTAASLQQWKVGDKCSAIWSEDGCIYP ATIASIDFKRETCVVVYTGYGNREEQNLSDLLSPICEVANNIEQNAQENENESQV STDESENSRSPGNKSDNIKPKSAPWNSFLPPPPPMPGPRLGPGKPGLKFNGPPPP PPPPPPHLLSCWLPPFPSGPPIIPPPPPICPDSLDDADALGSMLISWYMSGYHTG YYMGFRQNQKEGRCSHSLN |
| CPD (C. *difficile)* | 20 | EGSLGEDDNLDFSQNIVVDKEYLLEKISSLARSSERGYIHYIVQLQGDKISYEAA CNLFAKTPYDSVLFQKNIEDSEIAYYYNPGDGEIQEIDKYKIPSIISDRPKIKLT FIGHGKDEFNTDIFAGFDVDSLSTEIEAAIDLAKEDISPKSIEINLLGCNMFSYS INVEETYPGKLLLKVKDKISELMPSISQDSIIVSANQYEVRINSEGRRELLDHSG EWINKEESIIKDISSKEYISFNPKENKITVKSKNLPELSTL |
| CPD (*V. cholera*) | 21 | KEALADGKILHNQNVNSWGPITVTPTTDGGETRFDGQIIVQMENDPVVAKAAANL AGKHAESSVVVQLDSDGNYRVVYGDPSKLDGKLRWQLVGHGRDHSETNNTRLSGY SADELAVKLAKFQQSFNQAENINNKPDHISIVGCSLVSDDKQKGFGHQFINAMDA NGLRVDVSVRSSELAVDEAGRKHTKDANGDWVQKAENNKVSLSWDAQ |
| Cas9 (*S*. *pyogenes*) | 22 | MDKKYSIGLDIGTNSVGWAVITDDYKVPSKKFKVLGNTDRHSIKKNLIGALLFGS GETAEATRLKRTARRRYTRRKNRICYLQEIFSNEMAKVDDSFFHRLEESFLVEED KKHERHPIFGNIVDEVAYHEKYPTIYHLRKKLADSTDKADLRLIYLALAHMIKFR GHFLIEGDLNPDNSDVDKLFIQLVQIYNQLFEENPINASRVDAKAILSARLSKSR RLENLIAQLPGEKRNGLFGNLIALSLGLTPNFKSNFDLAEDAKLQLSKDTYDDDL DNLLAQIGDQYADLFLAAKNLSDAILLSDILRVNSEITKAPLSASMIKRYDEHHQ DLTLLKALVRQQLPEKYKEIFFDQSKNGYAGYIDGGASQEEFYKFIKPILEKMDG TEELLVKLNREDLLRKQRTFDNGSIPHQIHLGELHAILRRQEDFYPFLKDNREKI EKILTFRIPYYVGPLARGNSRFAWMTRKSEETITPWNFEEVVDKGASAQSFIERM TNFDKNLPNEKVLPKHSLLYEYFTVYNELTKVKYVTEGMRKPAFLSGEQKKAIVD LLFKTNRKVTVKQLKEDYFKKIECFDSVEISGVEDRFNASLGAYHDLLKIIKDKD FLDNEENEDILEDIVLTLTLFEDRGMIEERLKTYAHLFDDKVMKQLKRRRYTGWG RLSRKLINGIRDKQSGKTILDFLKSDGFANRNFMQLIHDDSLTFKEDIQKAQVSG QGHSLHEQIANLAGSPAIKKGILQTVKIVDELVKVMGHKPENIVIEMARENQTTQ KGQKNSRERMKRIEEGIKELGSQILKEHPVENTQLQNEKLYLYYLQNGRDMYVDQ ELDINRLSDYDVDHIVPQSFIKDDSIDNKVLTRSDKNRGKSDNVPSEEVVKKMKN YWRQLLNAKLITQRKFDNLTKAERGGLSELDKAGFIKRQLVETRQITKHVAQILD SRMNTKYDENDKLIREVKVITLKSKLVSDFRKDFQFYKVREINNYHHAHDAYLNA VVGTALIKKYPKLESEFVYGDYKVYDVRKMIAKSEQEIGKATAKYFFYSNIMNFF KTEITLANGEIRKRPLIETNGETGEIVWDKGRDFATVRKVLSMPQVNIVKKTEVQ TGGFSKESILPKRNSDKLIARKKDWDPKKYGGFDSPTVAYSVLVVAKVEKGKSKK LKSVKELLGITIMERSSFEKNPIDFLEAKGYKEVKKDLIIKLPKYSLFELENGRK RMLASAGELQKGNELALPSKYVNFLYLASHYEKLKGSPEDNEQKQLFVEQHKHYL DEIIEQISEFSKRVILADANLDKVLSAYNKHRDKPIREQAENIIHLFTLTNLGAP AAFKYFDTTIDRKRYTSTKEVLDATLIHQSITGLYETRIDLSQLGGDSPVR |
| Cas9 (*S*. *pyogenes*) with N-terminal His, SV40 and C-terminal SV40 sequences | 23 | HHHHHHGSGATMASPPKKKRKVGSMDKKYSIGLDIGTNSVGWAVITDDYKVPSKK FKVLGNTDRHSIKKNLIGALLFGSGETAEATRLKRTARRRYTRRKNRICYLQEIF SNEMAKVDDSFFHRLEESFLVEEDKKHERHPIFGNIVDEVAYHEKYPTIYHLRKK LADSTDKADLRLIYLALAHMIKFRGHFLIEGDLNPDNSDVDKLFIQLVQIYNQLF EENPINASRVDAKAILSARLSKSRRLENLIAQLPGEKRNGLFGNLIALSLGLTPN FKSNFDLAEDAKLQLSKDTYDDDLDNLLAQIGDQYADLFLAAKNLSDAILLSDIL RVNSEITKAPLSASMIKRYDEHHQDLTLLKALVRQQLPEKYKEIFFDQSKNGYAG YIDGGASQEEFYKFIKPILEKMDGTEELLVKLNREDLLRKQRTFDNGSIPHQIHL GELHAILRRQEDFYPFLKDNREKIEKILTFRIPYYVGPLARGNSRFAWMTRKSEE TITPWNFEEVVDKGASAQSFIERMTNFDKNLPNEKVLPKHSLLYEYFTVYNELTK VKYVTEGMRKPAFLSGEQKKAIVDLLFKTNRKVTVKQLKEDYFKKIECFDSVEIS GVEDRFNASLGAYHDLLKIIKDKDFLDNEENEDILEDIVLTLTLFEDRGMIEERL KTYAHLFDDKVMKQLKRRRYTGWGRLSRKLINGIRDKQSGKTILDFLKSDGFANR NFMQLIHDDSLTFKEDIQKAQVSGQGHSLHEQIANLAGSPAIKKGILQTVKIVDE LVKVMGHKPENIVIEMARENQTTQKGQKNSRERMKRIEEGIKELGSQILKEHPVE NTQLQNEKLYLYYLQNGRDMYVDQELDINRLSDYDVDHIVPQSFIKDDSIDNKVL TRSDKNRGKSDNVPSEEVVKKMKNYWRQLLNAKLITQRKFDNLTKAERGGLSELD |
| | | KAGFIKRQLVETRQITKHVAQILDSRMNTKYDENDKLIREVKVITLKSKLVSDFR KDFQFYKVREINNYHHAHDAYLNAVVGTALIKKYPKLESEFVYGDYKVYDVRKMI AKSEQEIGKATAKYFFYSNIMNFFKTEITLANGEIRKRPLIETNGETGEIVWDKG RDFATVRKVLSMPQVNIVKKTEVQTGGFSKESILPKRNSDKLIARKKDWDPKKYG GFDSPTVAYSVLVVAKVEKGKSKKLKSVKELLGITIMERSSFEKNPIDFLEAKGY KEVKKDLIIKLPKYSLFELENGRKRMLASAGELQKGNELALPSKYVNFLYLASHY EKLKGSPEDNEQKQLFVEQHKHYLDEIIEQISEFSKRVILADANLDKVLSAYNKH RDKPIREQAENIIHLFTLTNLGAPAAFKYFDTTIDRKRYTSTKEVLDATLIHQSI TGLYETRIDLSQLGGDSPVRSPKKKRKV |
| PNP | 24 | MENGYTYEDYKNTAEWLLSHTKHRPQVAIICGSGLGGLTDKLTQAQIFDYSEIPN FPRSTVPGHAGRLVFGFLNGRACVMMQGRFHMYEGYPLWKVTFPVRVFHLLGVDT LVVTNAAGGLNPKFEVGDIMLIRDHINLPGFSGQNPLRGPNDERFGDRFPAMSDA YDRTMRQRALSTWKQMGEQRELQEGTYVMVAGPSFETVAECRVLQKLGADAVGMS TVPEVIVARHCGLRVFGFSLITNKVIMDYESLEKANHEEVLAAGKQAAQKLEQFV SILMASIPLPDKAS |
| SUMO | 25 | MSDSEVNQEAKPEVKPEVKPETHINLKVSDGSSEIFFKIKKTTPLRRLMEAFAKR QGKEMDSLRFLYDGIRIQADQTPEDLDMEDNDIIEAHREQIGG |
| GTD | 28 | MSLVNRKQLEKMANVRFRTQEDEYVAILDALEEYHNMSENTVVEKYLKLKDINSL TDIYIDTYKKSGRNKALKKFKEYLVTEVLELKNNNLTPVEKNLHFVWIGGQINDT AINYINQWKDVNSDYNVNVFYDSNAFLINTLKKTVVESAINDTLESFRENLNDPR FDYNKFFRKRMEIIYDKQKNFINYYKAQREENPELIIDDIVKTYLSNEYSKEIDE LNTYIEESLNKITQNSGNDVRNFEEFKNGESFNLYEQELVERWNLAAASDILRIS ALKEIGGMYLDVDMLPGIQPDLFESIEKPSSVTVDFWEMTKLEAIMKYKEYIPEY TSEHFDMLDEEVQSSFESVLASKSDKSEIFSSLGDMEASPLEVKIAFNSKGIINQ GLISVKDSYCSNLIVKQIENRYKILNNSLNPAISEDNDFNTTTNTFIDSIMAEAN ADNGRFMMELGKYLRVGFFPDVKTTINLSGPEAYAAAYQDLLMFKEGSMNIHLIE ADLRNFEISKTNISQSTEQEMASLWSFDDARAKAQFEEYKRNYFEGSL |
| RRSP (DNA) | 29 | GGTGATAAAACCAAGGTCGTGGTCGATTTAGCGCAAATCTTTACGGTGCAAGAGC TGAAAGAAAGAGCAAAAGTTTTTGCTAAACCGATTGGCGCATCCTACCAAGGTAT TCTCGATCAACTCGACCTTGTGCATCAGGCTAAAGGCCGCGATCAAATCGCAGCG AGCTTTGAGCTTAATAAGAAGATTAATGACTACATCGCTGAACATCCAACTTCGG GGCGTAATCAAGCGCTAACGCAGTTGAAAGAGCAGGTCACCAGTGCGTTGTTTAT CGGTAAGATGCAAGTTGCCCAAGCGGGTATTGATGCAATCGCACAAACAAGACCG GAGCTTGCCGCTCGTATCTTTATGGTCGCGATTGAAGAAGCCAACGGTAAACACG TAGGTTTGACGGACATGATGGTTCGTTGGGCCAATGAAGACCCATACTTGGCACC GAAGCATGGTTACAAAGGCGAAACGCCAAGTGACCTTGGTTTTGATGCGAAGTAC CACGTAGATCTAGGTGAGCATTACGCTGATTTCAAACAGTGGTTAGAAACGTCCC AGTCGAACGGGTTGTTGAGTAAAGCGACGTTGGATGAATCCACTAAAACGGTTCA TCTTGGCTATAGCTATCAAGAACTTCAGGATTTGACGGGTGCTGAATCGGTGCAA ATGGCGTTCTACTTCCTGAAAGAAGCGGCGAAGAAAGCGGATCCGATTTCTGGTG ATTCAGCTGAAATGATACTGCTGAAGAAATTTGCAGATCAAAGCTACTTATCTCA ACTTGATTCCGACCGAATGGATCAAATTGAAGGTATCTACCGCAGTAGCCATGAG ACGGATATTGACGCTTGGGATCGTCGTTACTCTGGTACAGGCTATGATGAGCTGA CGAATAAGCTTGCTAGTGCAACGGGCGTTGACGAGCAGCTTGCGGTTCTTCTGGA TGATCGTAAAGGCCTCTTGATTGGTGAAGTGCATGGCAGCGACGTCAACGGCCTA CGCTTTGTTAATGAACAGATGGATGCACTGAAAAAACAGGGAGTCACAGTCATTG GCCTTGAGCATTTACGCTCAGACCTTGCGCAACCGCTGATTGATCGCTACCTAGC TACGGGTGTGATGTCGAGTGAACTAAGCGCAATGCTGAAAACAAAGCATCTCGAT GTCACTCTTTTTGAAAACGCACGTGCTAACGGTATGCGCATCGTCGCGCTGGATG CAAACAGCTCTGCGCGTCCAAATGTTCAGGGAACAGAACATGGTCTGATGTACCG TGCTGGTGCTGCGAACAACATTGCGGTGGAAGTATTACAAAATCTGCCTGATGGC GAAAAGTTCGTTGCTATCTACGGTAAAGCGCATTTGCAGTCTCACAAAGGGATTG AAGGGTTCGTTCCTGGTATCACGCACCGTCTCGATCTTCCTGCGCTTAAAGTCAG TGACTCGAACCAGTTCACAGTTGAACAAGACGATGTAAGTCTACGTGTTGTCTAC GATGATGTTGCTAACAAACCGAAGATCACGTTCAAGGGCAGTTTG |
| RRSP (amino acid) | 30 | GDKTKVVVDLAQIFTVQELKERAKVFAKPIGASYQGILDQLDLVHQAKGRDQIAA SFELNKKINDYIAEHPTSGRNQALTQLKEQVTSALFIGKMQVAQAGIDAIAQTRP ELAARIFMVAIEEANGKHVGLTDMMVRWANEDPYLAPKHGYKGETPSDLGFDAKY |
| | | HVDLGEHYADFKQWLETSQSNGLLSKATLDESTKTVHLGYSYQELQDLTGAESVQ MAFYFLKEAAKKADPISGDSAEMILLKKFADQSYLSQLDSDRMDQIEGIYRSSHE TDIDAWDRRYSGTGYDELTNKLASATGVDEQLAVLLDDRKGLLIGEVHGSDVNGL RFVNEQMDALKKQGVTVIGLEHLRSDLAQPLIDRYLATGVMSSELSAMLKTKHLD VTLFENARANGMRIVALDANSSARPNVQGTEHGLMYRAGAANNIAVEVLQNLPDG EKFVAIYGKAHLQSHKGIEGFVPGITHRLDLPALKVSDSNQFTVEQDDVSLRVVY DDVANKPKITFKGSL |

**Table 2** contains a non-exhaustive list of constructs generated and tested in ensuing Examples.

**Table 2**

| **Cargo family** | **Delivered cargo** | **Cargo MW (kDa)** |
|---|---|---|
| **DT-based** | (wildtype) = dtA | 21 |
| | (K51E/E148K) = dta | 22 |
| | (L350K) = dtb | 21 |
| | dtA alone | 21 |
| **Sumo-based** | Sumo-dtA | 35 |
| **eGFP-based** | eGFP-dtA | 49 |
| | eGFP-(G4S)1-dtA | 49 |
| | eGFP-(G4S)2-dtA | 49 |
| | eGFP-(G4S)3-dtA | 49 |
| | dtA-eGFP-dta | 71 |
| | dta-eGFP-dtA | 71 |
| | Sumo-eGFP-dtA | 62 |
| | Sumo-dtA-eGFP-dta | 84 |
| | dtA-eGFP-dtA-dtb | 71 |
| **mCherry-based** | mCherry-dtA | 48 |
| | dtA-mCherry-dta | 70 |
| | Sumo-dtA-mCherry-dta | 83 |
| **Ubiquitin-based** | Sumo-Ub-dtA | 43 |
| | Sumo-Ub-eGFP-dtA | 70 |
| | Sumo-Ub-dtA-eGFP-dta | 92 |
| **α-amylase-based** | α-amylase-dtA | 78 |
| | dtA-α-amylase-dta | 100 |
| | Sumo-dtA-α-amylase-dta | 113 |
| | α-amylase-dta | 78 |
| | α-amylase | 57 |
| **TAT-based** | TAT-dta | 21 |
| | dta-TAT | 21 |

### EXAMPLE 2

### Expression and Purification of Recombinant Diphtheria Toxin (DT)

Recombinant DT and cargo-DT chimeras were expressed as N-terminal His-tagged proteins in *E*. *coli* BL21 (DE3) cells, induced with 1 mM isopropyl-β-d-1-thiogalactopyranoside (IPTG) for 4 hours at 37°C (DT) or 21°C (cargo-DT), using the Champion^{™} pet-SUMO expression system (Invitrogen). Cells were harvested by centrifugation, re-suspended in lysis buffer (20 mM Tris-HCl pH 8.0, 0.5 M NaCl, 20 mM imidazole, benzonase, lysozyme and Protease inhibitor cocktail) and lysed by an EmulsiFlex C3 microfluidizer (Avestin) at 15,000 psi. The lysates were centrifuged at 18,000 × g for 20 minutes. His-Sumo-tagged proteins were purified by Ni-affinity chromatography using a His-Trap FF column (GE-Healthcare). After purification, the His-Sumo tag was removed by adding 1U of Sumo protease (Life Sensor) to 90 µg of purified protein in 20 mM Tris-HCl pH 8.0 containing 150 mM NaCl and 2 mM DTT. The cleavage reaction mixture was incubated at 30°C for 1 hour followed by purification using His-Pure^{™} Ni-NTA resin (Thermo Scientific) to remove the His-Sumo protease and His-Sumo tag from the purified DT and cargo-DT samples.

### EXAMPLE 3

### Cellular DTA Intoxication Assay

Protein synthesis inhibition was used to measure the ability of DT and cargo-DT chimera to deliver DTA to the cytosol. VERO cells (6,000 cells per well in a 96 well plate) were exposed to 3-fold serial dilutions of DT or cargo-DT. The cells were incubated with the toxin overnight (17 hours) at 37°C. The next day, toxin-containing medium was removed, and the cells were incubated for 2 hours at 37°C in leucine-deficient medium supplemented with 1 µCi of [³H]leucine/ml (PerkinElmer). The cells were washed twice with cold phosphate-buffered saline (PBS) before precipitation of cellular protein with 10% trichloroacetic acid (TCA). Protein synthesis was measured by the amount of [³H]leucine incorporated into total cellular protein, as determined by scintillation counting with a TopCount NXT^{™} (Perkin Elmer). Percent protein synthesis was plotted versus the log concentration of DT or cargo-DT. Protein synthesis kinetic experiments were performed as described above except that the toxin concentration was fixed at 1 nM and the toxin was exposed to cells for 1, 2, 3, 4, 5 or 17 hrs. Protein synthesis competition experiments were performed as described above but a fixed concentration (1 nM) of a non-toxic variant of DT (a-B or DT_K51E/E148K) was also added to cells to compete with the toxic DT variant (A-EGFP-a-B) which was added to cells using a 3-fold serial dilution pattern (starting concentration 1 nM).

### EXAMPLE 4

### Real-time protein unfolding using Differential Scanning Fluorimetry

Purified eGFP (40 ng) and mCherry (7 µg) (20 mM Tris-HCl, 150 mM NaCl, pH8.0) were diluted in citrate-phosphate buffer at pH's that ranged from 3.6 to 7.6 in a 96-well PCR plate. The proteins at various pH's were placed in a Real-Time PCR Detection System (BioRad CFX96^{™}) to measure protein unfolding. Intrinsic fluorescence of eGFP (494 - 519 nm) and mCherry (595 - 613 nm) were captured over a wide range of temperature (15 to 95°C). The melting temperatures of eGFP and mCherry were calculated by the software provided with the detection system (Bio-Rad CFX Manager^{™} 3.1).

### EXAMPLE 5

### Amylase delivery of intracellular glycogen

24h prior to treatment HEK293 cells were seeded on 6-well plates (BD amine coated) at 0.5 × 10⁶ (for 24 h incubation) or 0.25 × 10⁶ (for 48 h incubation) cells per well and cultivated at 37 °C and 5% CO₂ in DMEM (10% FBS, 1% Penicillin/Streptomycin). For treatment the medium was changed, the new medium containing additionally 0.1 or 1.0 uM of DT, DT-amylase, or amylase protein. After incubation as indicated for harvesting cells were put on ice, washed once with ice-cold PBS, and in a cold room (4 °C) transferred to 1.5 mL vials subsequent to scraping and resuspending them in 1 mL ice-cold PBS. The cells, being henceforth kept on ice or at 4 °C, were pelleted, the supernatant being removed. After washing the cells again with 1 mL PBS the cell sample was split for separate glycogen and protein determination.

For protein determination cells were lysed on ice in RIPA buffer (150 mM NaCl, 20 mM Tris, 12.1 mM deoxycholate, 1% triton X-100, 0.1% SDS). Following centrifugation (14,000 x g, 4 °C, 15 min) the supernatant was subjected to protein determination using the DC^{™} protein assay (Bio-Rad) following the manufacturer's instructions.

For glycogen determination cells were incubated for 45 min in 0.5 M KOH at 98 °C with intermittent mixing to lyse cells and extract glycogen. Following neutralization with 2 M acetic acid glycogen was digested in an aliquot overnight at 55 °C with 0.5 U amyloglucosidase (Sigma) and subsequently determined as free glucose according to O.H. Lowry and J.V. Passonneau (1972) A Flexible System of Enzymatic Analysis. New York, New York: Academic Press, with an enzymatic assay that detects NADPH by incubating the sample with hexokinase (Roche), glucose 6-phosphate dehydrogenase (Roche), ATP (Sigma), and NADP (Roche). Glucose in undigested extracts was consistently below the limit of detection.

Glycogen was based on protein levels to account for cell loss or growth variances due to treatment. Protein-based glycogen levels were normalized to controls treated with identical amounts of either DT or amylase protein (as indicated). Significance was tested using a T-test (two-tailed, homoscedastic since variances between sample populations were not significantly different). Significance levels: 0.05>p≥0.01 (*), 0.01>p≥0.001 (**), p<0.001 (***).

### EXAMPLE 6

### Amino-terminal protein fusions dramatically decrease the apparent cytotoxicity of DT

To evaluate the ability of the diphtheria toxin translocation apparatus to co-deliver proteins into mammalian cells, a series of model passenger proteins were cloned, in accordance with Example 1, as amino terminal fusions to DT with an intervening Gly-Ser-Gly linker.

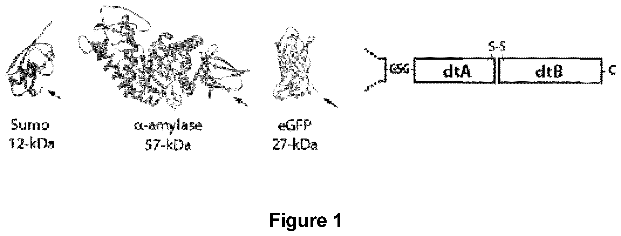

**Figure 1** depicts these constructs. Initially, three distinct passenger proteins were chosen, spanning a range of sizes, structures and physical properties with which to evaluate intracellular delivery: the 13-kDa globular Small Ubiquitin-like Modifier (SUMO; PDB: 3pge) protein; the 27-kDa enhanced green fluorescent protein (eGFP; PDB: 1gfl); and the 57-kDa α-amylase enzyme from *B.megaterium* (in Figure 1, the structure of alpha-amylase of *H. orenni* - PDB: 1wza - Is shown an *example* structure from the alpha-amylase family). The proteins were fused to DT via a GSG linker. These constructs were expressed and purified in accordance with Example 2. To quantify delivery of the chimeric constructs to the cytosol, the intracellular action of the co-delivered A-chain of DT (dtA), which catalyzes the ADP-ribosylation of EF-2 and inhibits protein synthesis (i.e., incorporation of ³H-Leu in the cellular proteome), was measured over a 2h period in VERO cells that had been treated overnight with the chimeric toxins, in general accordance with Example 3.

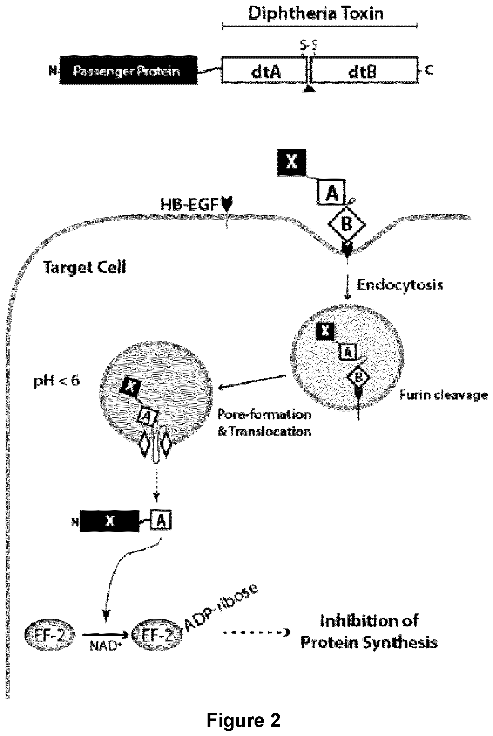

**Figure 2** depicts a schematic of first generation chimeric fusions of different passenger proteins to the amino terminus of native diphtheria toxin (DT) via a flexible GSG linker. The enzymatic A domain (dtA) and translocation/ receptor-binding B domain (dtB) have an intervening furin-like recognition site (black triangle) and are further joined by an intra-molecular disulfide bond. DT is internalized into endocytic vesicles by a receptor-mediated process. Within endosomes, a membrane-bound furin-like protease cleaves between dtA and dtB. Upon vesicular acidification, dtB undergoes a major conformational change, resulting in the formation of a membrane-spanning pore. dtA (and any associated passenger proteins) would then translocate into the cytosol starting with dtA, followed by any amino-terminal passenger proteins. Once in the cytosol, the dtA fragment catalyzes ADP-ribosylation of EF-2, resulting in the inhibition of protein synthesis. This straightforward measure of delivery is well established and provides a universal readout of delivery across different studies and different passenger proteins.

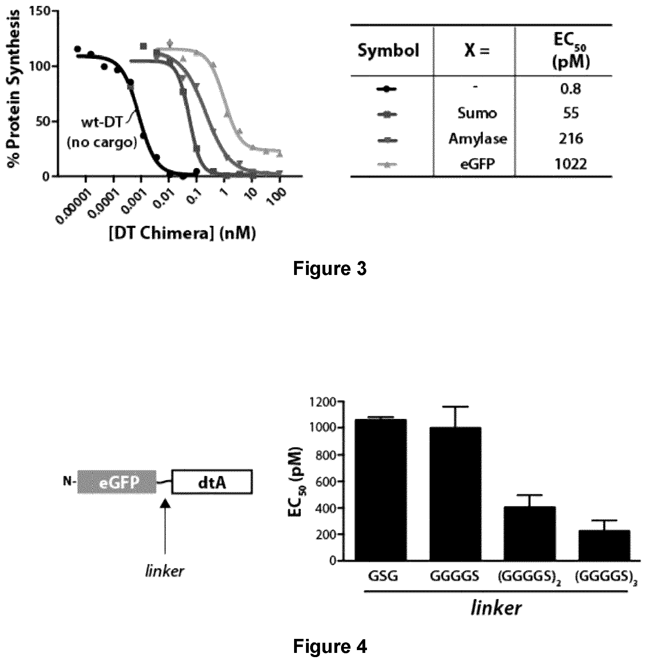

**Figure 3** depicts dose titration curves of chimeric constructs on cells with wt-DT, Sumo-DT, Amylase-DT, and eGFP-DT (EC₅₀ values are at the right), and shows that, in the absence of passenger proteins, (i.e., wildtype DT), protein synthesis was dose-dependently inhibited with an EC₅₀ = 1.3 ± 0.7 pM. Figure 3 further shows that, when Sumo, eGFP and α-amylase fusions were tested for intracellular delivery, protein synthesis was dose-dependently inhibited in all cases indicating that passenger proteins were delivered into the cytosol. Comparing the doses at which protein synthesis was inhibited by 50% (EC₅₀) for each chimera however, revealed significant shifts in their relative abilities to inhibit protein synthesis: 65-fold for Sumo; 260-fold for α-amylase; and 1200-fold for eGFP. These shifts, which are consistent with what has been observed previously with smaller cargo^{4,6,7,9}, suggest that passenger proteins disrupt the natural process of cellular intoxication somehow. Two fundamentally linked questions remain: at what exact step do passenger proteins disrupt intoxication; and, do these observed shifts directly correspond to reduced efficiency of intracellular delivery by DT.

It was hypothesized that the observed decreases in apparent potency might be due to the cargo differentially affecting the intracellular enzymatic activity of dtA after the chimeras had already entered the cytosol, rather than due to affecting upstream phenomena such as receptor binding or translocation *per se.* Support for this hypothesis came from a set of experiments that had been designed to investigate the effect of increasing the linker size between Cargo and dtA on expression and stability.

**Figure 4** shows the effect of linker size between eGFP and dtA on cells, with error bars, SD (n=2). The consequent effects on the potency of inhibition of protein synthesis for each construct are shown on the right. With eGFP as the passenger protein, increasing the linker size GSG to GGGGS (i.e., G₄S) to (G₄S)₂ to (G₄S)₃, resulted in increases in potency on cells, consistent with the idea that the passenger protein was affecting a step other than translocation.

### EXAMPLE 7

### Passenger proteins are 'invisible' to the translocation machinery of DT

To explore the hypothesis that the passenger cargo was indirectly impacting dtA by proximity effects in a more direct way, a new construct was generated per Example 1 in which the active dtA reporter was placed upstream of eGFP (with a free amino terminus as it is in the WT toxin). The existing dtA attached to dtB was rendered catalytically inactive by the double mutation, K₅₁E/E₁₄₈K¹⁰, signified as dta, to yield the final construct: dtA-eGFP-dta-dtB; or for simplification: A-eGFP-a-B. Cellular assays were carried out per Example 3 to study positional effects of passenger proteins on dtA activity in cells.

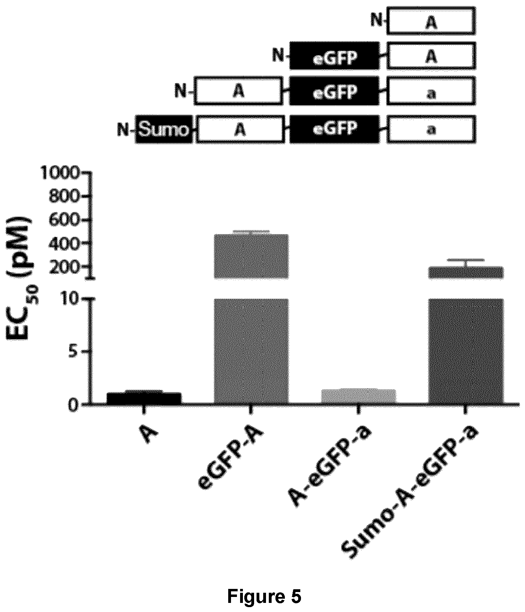

**Figure 5** depicts the construct and shows, remarkably, that A-eGFP-a-B inhibited protein synthesis such that it was indistinguishable from wildtype-like toxin. This shows that the shifts in potency are due to proximity effects on dtA activity. Further, Figure 5 shows that addition of Sumo onto the amino terminus of this construct shifted the apparent activity back to levels observed with amino-terminal cargo constructs. Bars represent average EC₅₀ ± SD (n = 3).

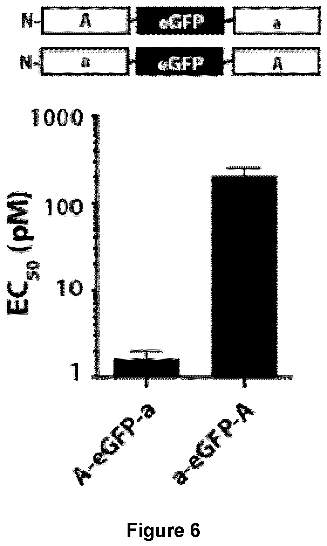

**Figure 6** corroborates certain findings depicted in Figure 5. To rule out the possibility that the amino terminal dtA fragment was affecting translocation, it is shown that a-eGFP-A is shifted similar to eGFP-A. Bars represent average EC₅₀ ± SD (n = 3).

To show that this phenomenon was not specific to eGFP, a similar set of constructs were generated, using α-amylase as the passenger domain.

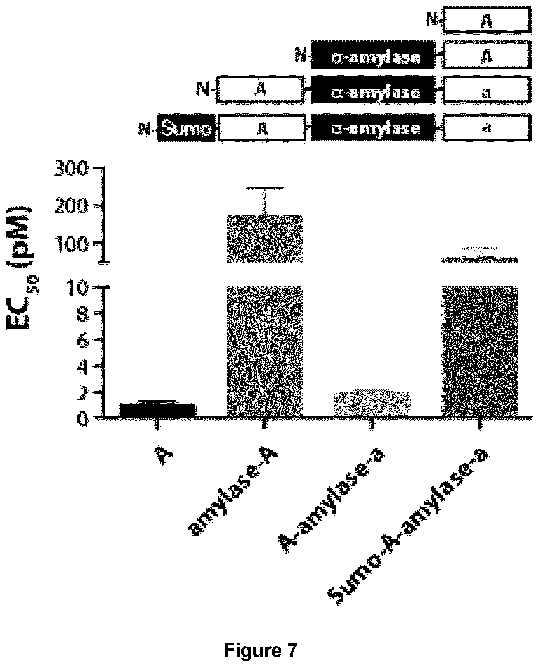

**Figure 7** shows that the same positional dependence of dtA on activity was observed when using amylase as the passenger protein. Bars represent average EC₅₀ ± SD (n = 3).

The 'wildtype-like' potencies observed for A-cargo-a-B constructs have several important implications for DT delivery. In addition to strongly supporting the hypothesis that amino-terminal passenger proteins affect dtA activity after they reach the cytosol, rather than impeding receptor binding or translocation, these data indicate that passenger proteins are virtually invisible to the translocation machinery of DT. Also, because translocation initiates with the C-terminal end of the A-domain that is adjacent to the B-moiety and proceeds such that the amino terminus is last to enter the cytosol¹¹, these findings show unequivocally that passenger proteins fully enter the cytosol. Finally, these constructs eliminate any possibility that the inhibition of protein synthesis observed for chimeric toxins is from breakdown products in which cargo was removed prior to or during intoxication, since amino terminal truncations would result in the loss of dtA and would be nontoxic.

Building on these findings, the predictable shifts observed with amino terminal fusions to dtA were exploited, and the unique properties of ubiquitin and deubiquitinating enzymes found only in the cytosol, to demonstrate intracellular delivery through an independent measure. Since cytosolic deubiquitinating enzymes cleave at the C-terminus of ubiquitin (Ub), Ub was inserted between passenger proteins and dtA in two different contexts so that the amino terminus of dtA will be liberated only if the entire payload was translocated into the cytosol.

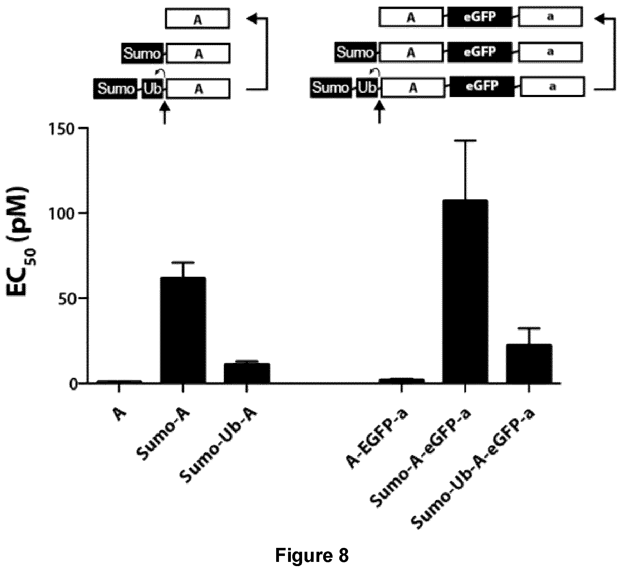

**Figure 8** depicts these constructions, and shows that both Ub-containing constructs were more potent on cells than their des-Ub counterparts, albeit not back to wildtype levels, which may reflect the kinetics of removal of Ub by deubiquitinating enzymes (DUBs). Ub was placed between Sumo and dtA (left panel of Figure 8) and was found to be more potent on cells than Sumo-A, consistent with deubiquitinating enzymes removing amino terminal cargo and relieving the proximity effect on dtA activity. Using more extensive cargo, the ubiquitin entry assay confirms that large protein cargo enter the cytosol (right panel of Figure 8).

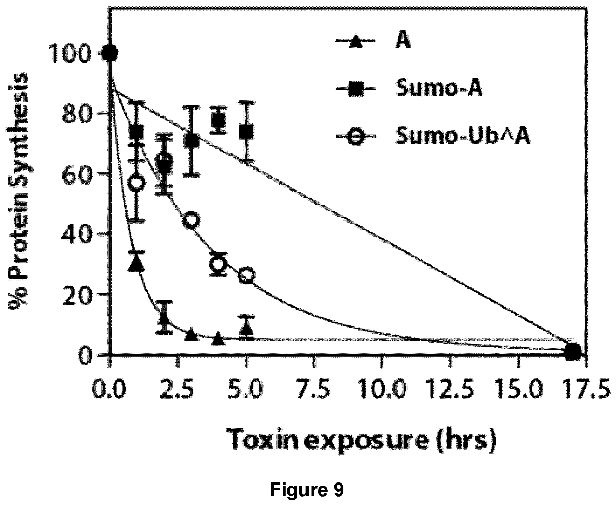

**Figure 9** depicts a time course of inhibition of protein synthesis of all three constructs using 1nM of each toxin. Symbols ± SD (n = 3) are shown.

In addition to demonstrating that passenger proteins are in the cytosolic compartment, these data show that DT can simultaneously deliver multiple different proteins, akin to beads on a string - that combined, are over 100-kDa in size - into the cytosol *en masse.*

### Example 8

### The DT translocation machinery can deliver a folded protein into cells

The unexpected plasticity of the DT translocation machinery observed with large and diverse protein cargo prompted the question of whether DT could transport stably folded proteins into cells. To this end, the fluorescent protein variant derived from *Discosoma* sp. "DsRed" called monomeric Cherry (mCherry) was used. Though similar in size and structure to eGFP, mCherry been shown to possess dramatically increased conformational stability relative to eGFP *in vitro* and *in vivo.* Constructs were generated per Example 1.

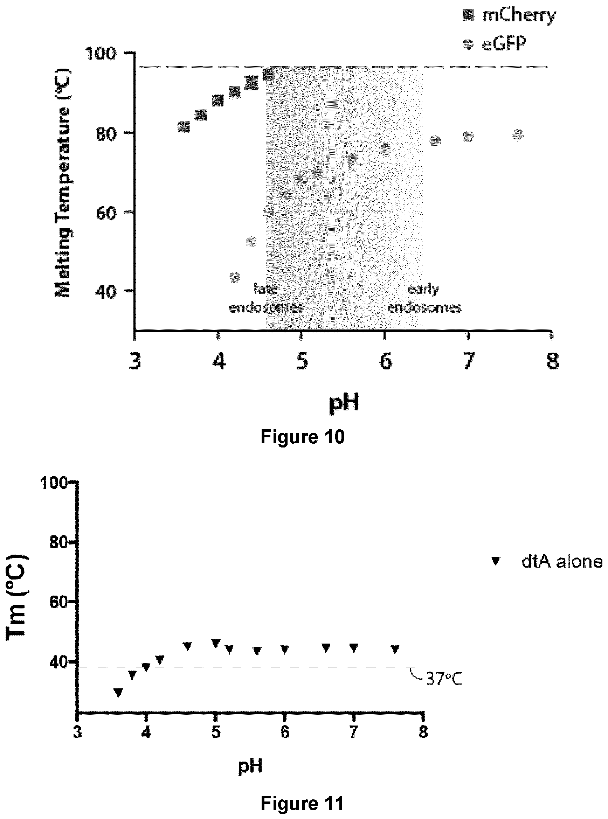

**Figure 10** shows that, using differential scanning fluorimetry, mCherry was indeed dramatically more stable to thermal- and pH-induced unfolding than eGFP. Shaded region shows pH levels within early to late endosomes where translocation takes place. Above pH 4.6, mCherry does not unfold up to 95°C. Symbols represent average Tₘ ± SD (n = 3).

**Figure 11** shows that mCherry was also more stable than dtA.

In fact, an unfolding transition for mCherry could only begin to be measured below pH 4.6, strongly suggesting that unfolding of mCherry is not likely to occur within endosomal compartments, where membrane translocation occurs. Zornetta et al.²² recently investigated anthrax toxin translocation in cells and found that whereas eGFP fusions to lethal factor (LF) were efficiently transported through the narrow and fixed protective antigen (PA) pore, similar fusions with mCherry were unable to translocate into cells, supporting the notion that eGFP, but not mCherry unfolds in early endosomes prior to translocation.

To test whether the DT translocation apparatus could deliver stably folded mCherry into cells, mCherry-DT chimeras were generated using the same platform designs as above, in Example 1.

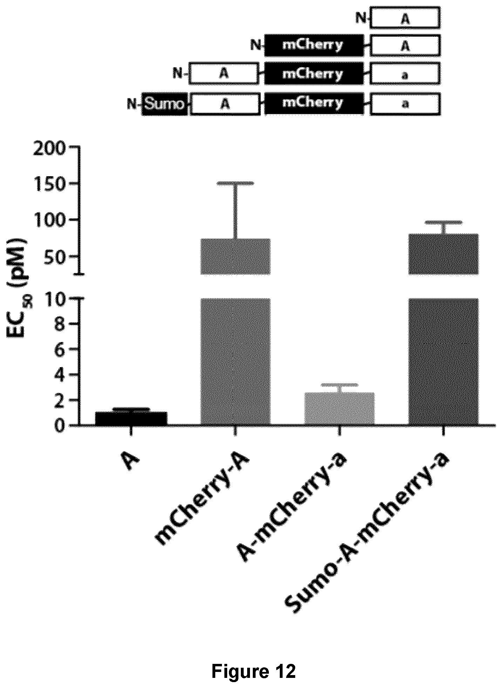

**Figure 12** depicts the results of cell toxicity assays indicating that mCherry is efficiently delivered into cells by DT. Surprisingly, unlike the anthrax toxin translocation system, diphtheria toxin was able to deliver mCherry into cells with wildtype like efficiency. mCherry, like eGFP and amylase are invisible to the DT translocation machinery. Though the possibility cannot be excluded that mCherry is somehow mechanically unfolded immediately prior to, or during translocation, these data indicate, at the very least that DT can accommodate and transport hyper-stable proteins. Furthermore these findings show that DT is distinct from and has a broader substrate profile than the anthrax toxin pore suggesting that not all toxin translocation systems are alike.

### EXAMPLE 9

### Comparison and characterization of the DT delivery platform

With the observed differentiation from anthrax toxin, further benchmarking of DT against a non-toxin derived protein delivery platform was sought. To this end, the ability of the cell-penetrating TAT peptide from HIV-1¹ to deliver dtA into the cytosol was evaluated. Given the effects observed here on dtA activity in the presence of amino-terminal extensions, both TAT-dtA and dtA-TAT were generated and compared their ability to inhibit protein synthesis with the translocation machinery of DT (i.e., dtB).

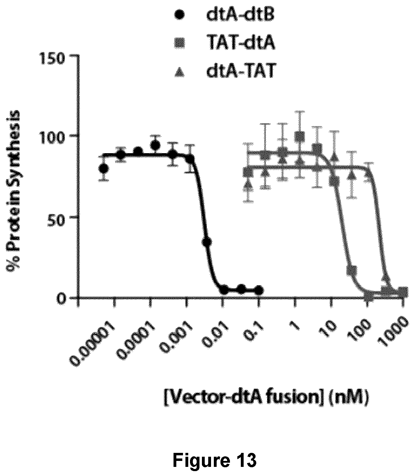

**Figure 13** shows results comparing the delivery of dtA by dtB and TAT peptides, and shows that both Tat-dtA and dtA-Tat were able to penetrate cells and inhibit protein synthesis. Symbols ± SD (n = 3) are shown. However, as reported previously for TAT-dtA, the concentrations required for both TAT constructs were at least four orders-of-magnitude higher than those required for DT. Beyond this clear efficiency advantage for DT, an important conceptual advantage of the DT system over existing protein delivery platforms such as TAT, is the target-cell specificity conferred by a receptor-binding domain.

To confirm that cargo translocation by DT was receptor-dependent, a competition experiment between A-eGFP-a-B and catalytically inactive DT was performed.

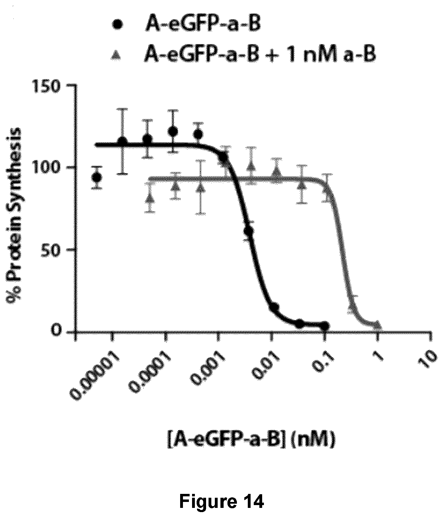

**Figure 14** shows that, in the presence of 1 nM nontoxic DT, the potency of A-eGFP-a-B was shifted from 3.8 pM to 215 pM, confirming that cargo delivery was receptor-dependent, and that the cargo itself did not mediate its own uptake. Symbols ± SD (n = 3) are shown.

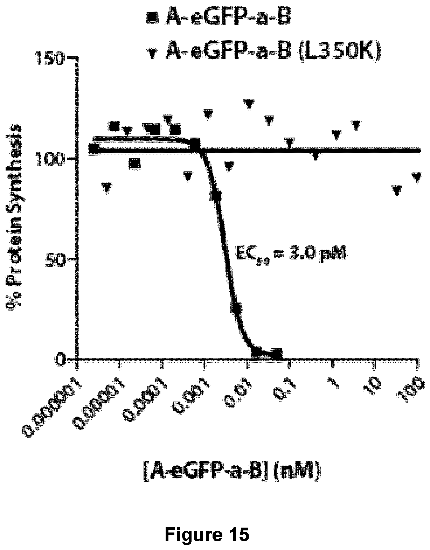

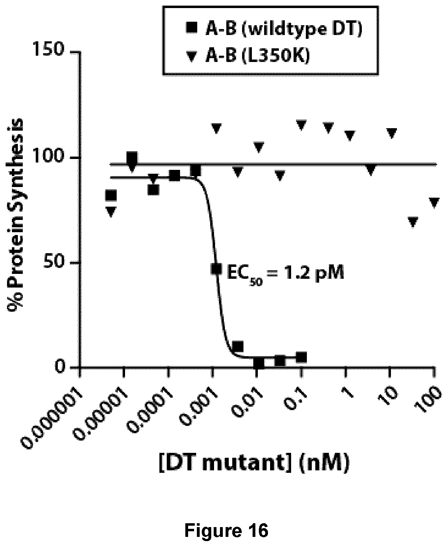

**Figure 15** and **Figure 16** show that, using a pore-formation mutant in the translocation domain of DT, which prevents translocation, it is shown that cargo delivery into cells requires a functional translocation domain, and that cargo did not mediate its own entry. Symbols ± SD (n = 3) are shown.

### EXAMPLE 10

### Direct evidence of functional entry of α-amylase by DT

Having demonstrated that passenger proteins are delivered into cells by DT in a receptor- and translocation-dependent manner with high efficiency, it was next desirable to test whether the delivered cargo was folded and functional within the cytosol. Rather than use the more qualitative measurements of intracellular fluorescence using eGFP or mCherry as cargo, it was desirable to measure the ability of delivered α-amylase to enzymatically digest cytosolic glycogen. An amino-terminal extension of nontoxic DT (i.e., α-amylase-dta-dtB) was prepared in general accordance with Example 1.

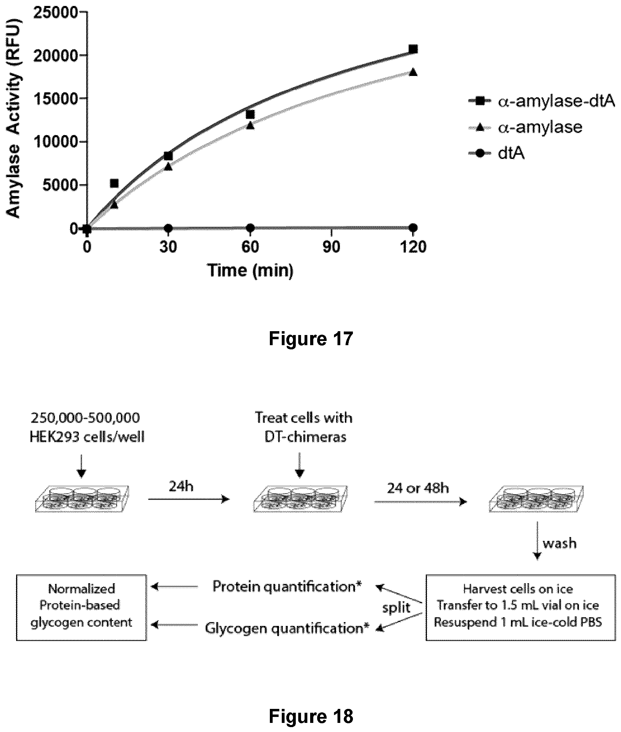

**Figure 17** confirms that the specific activity of α-amylase was equivalent to α-amylase alone using a quenched fluorescence substrate-based assay. The EnzChek^{™} Ultra Amylase Assay was used to measure the activity of α-amylase-dtA (curve marked with square data points), α-amylase (curve marked with triangular data points), and dtA alone (curve marked with circular data points) over 2 hours.

**Figure 18** depicts the experimental design for α-amylase-DT treatment of HEK 293 cells. HEK293 cells were treated for 24 or 48 h with α-amylase-dta-dtB at two different concentrations to establish conditions where decreases in protein-based glycogen could be detected.

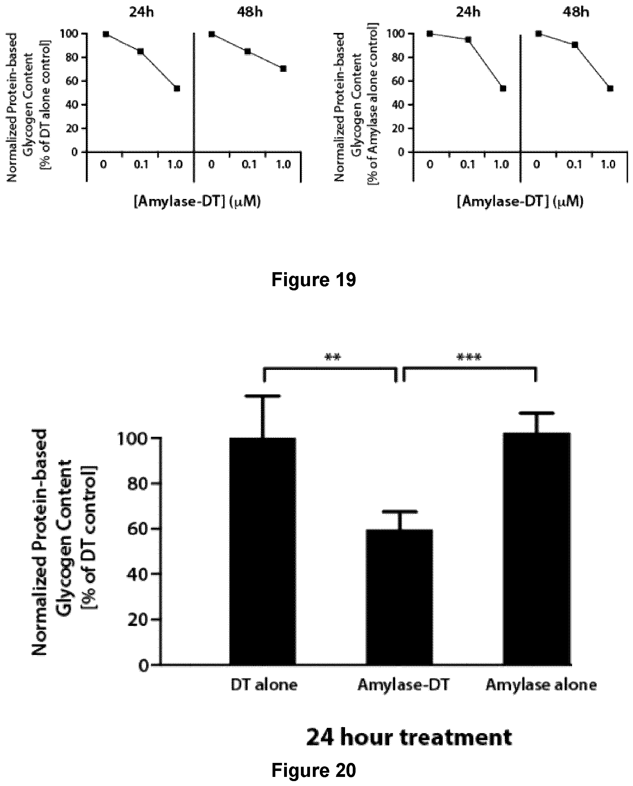

**Figure 19** shows protein-based glycogen content in HEK cells after 24 h or 48 h treatment normalized on content in cells treated with either DT alone or amylase alone, respectively (n=1). Using DT alone or α-amylase as controls, dose-dependent decreases in protein-based glycogen content in cells were observed at both time points, with a slightly more pronounced effect apparent at 24 h.

**Figure 20** shows protein-based glycogen content in HEK cells after 24 h treatment with 1.0 uM DT, amylase-DT, or amylase alone. Error bars, SD (n=4; Significance as determined with STUDENT t-test (p<0.01, **; p<0.001, ***). A highly significant decrease in glycogen observed was using 1 µM α-amylase-dta-dtB, which demonstrates that the translocated α-amylase-dta is folded and active in the cytosol, and shows that the amounts delivered are sufficient not only to degrade existing glycogen, but also to compete with on-going cellular glycogen synthesis. The measured breakdown of glycogen is thus likely an underestimation of the intracellular activity. Moreover, because maltose or any longer glucose oligomer - though also products of the amylase-mediated glycogen degradation - would still be determined as 'glycogen' in our biochemical glycogen quantification method, the possibility also exists that even greater amounts of glycogen were degraded by the delivered α-amylase. Nevertheless, these results provide an important proof-of-delivery of a large and functionally active protein into cells by the diphtheria toxin platform.

### EXAMPLE 11

### Discussion

In its 'protective' role, the plasma membrane that encases all human cells unwittingly excludes proteins from entering that might otherwise be effective therapeutics. Though several vectors for intracellular protein delivery into cells have been described, few if any combine the attributes of efficiency, target-cell specificity and low toxicity into a single platform. In this study, a protein-delivery vector is described, which based on the versatile diphtheria toxin that is capable of translocating proteins of varying structural motifs, stabilities and sizes including those that are over 100-kDa in size, with high efficiency into specific receptor-bearing cells (see **Table 2,** above, for a list of chimeric constructs with molecular weights). Engineering protein toxins to create 'designer chimeras' is not a new concept, however, efforts thus far have largely focused on delivering the toxic A-fragment enzymes into specific target cells through modifications to the receptor-binding domain of toxins. Denileukin difitox (Ontak^{®}), an FDA-approved antineoplastic agent created through the fusion of human Interleukin-2 (IL2) to a truncated form of DT (*viz.* dtA-dtB₃₈₉-IL2), binds the IL2 receptor that is overexpressed in various malignancies and translocates dtA into the cytosol to ultimately specifically ablate cancerous cells^{12,13}. Recent breakthroughs that serve to improve the safety profile and specificity of immunotoxins have intensified interest in these therapeutics with several now in clinical development, largely for cancer-related indications¹³⁻¹⁵.

This study investigated creating a different type of toxin-based 'designer chimera', taking advantage of the cell-penetrating properties of DT, rather than its cytotoxic features. Previous studies investigating passenger protein delivery with toxins have had mixed success. Using anthrax toxin, there have been a number of reports of successful delivery of peptide and protein cargo into cells¹⁶⁻¹⁹. A limitation of anthrax toxin as a delivery system - aside from it being composed of two separate proteins that must find each other on the cell surface to become delivery competent - is the rigid β-barrel translocation pore^{20,21} of the B-fragment of anthrax toxin²² that necessitates complete unfolding of passenger proteins. It has been shown here that DT, on the other hand, can deliver large and structurally diverse proteins into cells with relative ease. Previous studies investigating the utility of DT as a protein delivery vector were inconclusive, in large part because the addition of cargo to the amino terminus decreased the apparent efficiency of intracellular delivery as determined using the 'gold-standard' measure of protein synthesis inhibition. An important discovery here is that the translocation step itself was not impeded for all passenger proteins tested, but rather the activity of dtA was diminished by the juxtaposition of amino terminal proteins. Of equal importance is the finding that DT was able to transport hyper stable, potentially folded, proteins into cells.

An important consideration for future development of DT- and other toxin-based protein delivery vectors is the extent to which immunogenicity can be addressed for *in vivo* applications. Recent breakthroughs in identifying and eliminating both B-cell²³ and T-cell^{15,24} epitopes on toxin-based proteins suggest that there are manageable strategies to redesign the surfaces of toxins without disrupting their cell-penetrating properties. With the growing library of chimeric toxins targeting different cell types, next generation toxin designs incorporating different features that target particular proteins to specific cells/tissues will serve to further expand the potential of DT-based systems as intracellular protein delivery platforms, ultimately for therapeutic delivery of proteins into cells *in vivo.*

### EXAMPLE 12

### Delivery of Additional Cargo

Additional constructs were made to test the ability of the DT platform to delivery other proteins, including cargo of therapeutic significance (e.g. MecP2, SMN, FMRP, FMRP), cargo for genome editing applications (e.g. Cas9), and an auto-processing release domain (e.g. CPD).

### Materials and Methods

### Cell lines

Vero cells are grown in DMEM with 10% fetal bovine serum and 1% penicillin/streptomycin. Vero-NlucP cells are Vero cells stably expressing a Nanoluciferase-PEST fusion protein (NlucP; Promega) delivered via lentiviral vector and subsequent puromycin selection and clonal selection. Both WT and RTTΔ3-4 were derived from fibroblasts of a single Rett Syndrome patient (Cheung et al 2011). RTTΔ3-4 neurons lack exons 3 and 4 of the MeCP2 gene, resulting in a MeCP2-null cell line. WT neurons are isogenic controls. Neurons were kindly provided by Dr. James Ellis.

### Expression and Purification of fusion proteins

Recombinant DT fusion proteins were expressed as N-terminal His-tagged proteins using the Champion pET-SUMO expression system (Invitrogen), except Myc-MeCP2-DT, which does not have either an N-terminal His or SUMO tag. Fusion proteins were expressed in *E*. *coli* BL21(DE3) cells. Cells were transformed with the individual plasmids and grown to an OD of ~ 0.6. Myc-MeCP2-DT was induced with 0.5 mM IPTG and expressed at 28°C for 6 hours. Myc-SMN-DT and Myc-FMRP-DT were induced with 1 mM IPTG and expressed at 16°C for 18 hours. PNP-DT was expressed with 1 mM IPTG and expressed for 4 hours at 21°C. Cas9-DT was induced with 0.2 mM IPTG and expressed at 18°C for 18 hours. eGFP-CPD_{Vc}-DT was induced with 1mM IPTG and expressed at 21°C for 5 hours. All lysates were purified on HisTrap FF Crude (GE Healthcare) chromatography columns. Cas9-DT was further purified on a GE Heparin FF column, while eGFP-CPD_{Vc}-DT was further purified on a GE Superdex pg75 gel filtration column. All SUMO-tagged proteins were treated with 1U of SUMO protease (Life Sensor) per 90 µg of purified protein in 20 mM Tris-HCl pH 8 containing 150 mM NaCl and 2 mM DTT. The cleavage reaction was incubated at 30°C for 1 hour followed by purification with His-Pure Ni-NTA resin (Thermo Scientific) to remove the His-SUMO tag and SUMO protease from the purified fusion proteins.

### DT Toxicity Assays

### Cell Viability Assay

Vero cells were plated at 4000 cells/well in a 96-well cell culture plate and allowed to attach overnight at 37°C and 5% C0₂. The next day, fusion toxins were added at various concentrations in DMEM (10% FBS, 1% penicillin/streptomycin). After 48 hours, 100 µl of Presto-Blue (Life Technologies) cell viability dye was added to all wells and incubated at 37°C for 2 hours. Fluoresence was measured in a SpectraMax M5e microplate reader (Molecular Devices) (Ex/Em 555/585 nm). Results were quantified and fit to a sigmoidal function in GraphPad Prism.

### ³H-Leucine Incorporation Assay

Vero cells or neurons were plated as above (neurons were used after 2 weeks at 30,000 cells/well). Cells were treated with various concentrations of fusion toxins in either DMEM (10% FBS, 1% penicillin/streptomycin [cDMEM]) (Vero cells) or neurobasal media supplemented with cAMP (1 µM), BDNF (10 ng/ml) GDNF (10 ng/ml) and ascorbic acid (200 ng/ml) (neurons). After 15 hours, cells were washed with 200 µl leucine-free cDMEM then incubated in 50 µl leucine-free cDMEM supplemented with 5 µCi/ml of tritiated leucine for 2 hours at 37°C. Cells were washed with 200 µl of ice-cold PBS, and cellular protein was precipitated with 100 µl ice-cold 10% TCA for 10 minutes at room temperature. Cells were then washed with 100 µl of ice-cold 5% TCA and dissolved in 0.1N NaOH before being transferred into a 96-well polystyrene plate and mixed with 200 µl of scintillation fluid. Total incorporated ³H-leucine was measured by scintillation counting using a TopCount NXT.

### NanoGlo Assay

Vero cells were transduced with a lentivirus containing the Nanoluc luciferase gene (Promega) fused to a C-terminal PEST degradation domain. Positive clones were selected by puromycin selection followed by clonal selection to make Vero-NlucP cells. These cells were treated with various concentrations of fusion toxins as above for 15 hours. NanoGlo assay (Promega) was carried out per the manufacturer's instructions. Luminescence was read on a SpectraMax M5e microplate reader and data was fit to a sigmoidal function (GraphPad Prism).

### Results and Discussion

### Cargo of Therapeutic Significance

A primary theme of the development of diphtheria toxin (DT) as a protein delivery platform is the delivery of proteins implicated in recessive monogenic disorders, especially those with a neurological component, as a form of enzyme replacement therapy (ERT). Typically, ERT regimens rely on proteins that are active in the extracellular environment or in the endosomal/lysosomal pathway due to their inability to penetrate the cellular plasma membrane. Others rely on cell-penetrating peptides (CPP) such as the HIV-derived TAT peptide, but these suffer from a lack of specificity, and typically do not cross the blood-brain-barrier (BBB).

The fundamental platform on which all fusion proteins are built is the dtA-dtB, wildtype diphtheria toxin. The dtB domain is composed of the translocation (dtT) domain, and the receptor-binding (dtR) domain. Two inactivating mutations in dtA (K51E and E148K) render the toxin completely non-toxic (referred to as dta herein). All DT fusion proteins were also created with these inactivating mutations as non-toxic versions. A further mutation in the dtT domain (L350K) abrogates toxicity by preventing pore-formation and translocation. All fusion proteins are expressed with an N-terminal polyhistidine tag and a SUMO tag. Removal of the His-SUMO tag is accomplished during purification with treatment with SUMO protease.

Four proteins implicated in childhood genetic brain disorders have been cloned, expressed and purified. Namely, methyl-CpG-Binding Protein 2 (MeCP2; Rett Syndrome), Survival of Motor Neuron (SMN; Spinal Muscular Atrophy), Fragile X Mental Retardation Protein (FMRP; Fragile X Syndrome), and Purine Nucleoside Phosphorylase (PNP; PNP-deficiency). Cloned, expressed and purified are alpha-amylase from *Bacillus megaterium* as a therapeutic treatment for Lafora Disease, the Cas9 nuclease from *Streptococcus pyogenes,* as well as the fluorescent proteins eGFP and mCherry. Cytoplasm-sensing autorelease domains have been engineered into the DT platform in the form of cysteine protease domains from both *Clostridium difficile* toxin B and *Vibrio cholerae* MARTX toxin.

### MecP2

The primary cause of Rett Syndrome, mutations in the MeCP2 gene result in a non-functional protein product. MeCP2 is a DNA-binding protein and acts as a global transcriptional regulator. Myc-MeCP2-dtA-dtB has been expressed and purified. The DNA sequence for MeCP2e1-dtA-dtB was synthesized and codon optimized for *E*. *coli* expression from GenScript. The Myc tag is linked to MeCP2 with a GSG linker. MeCP2 is linked to dtA with a (G4S)2 linker. MeCP2 can exist in two main isoforms, e1 and e2.

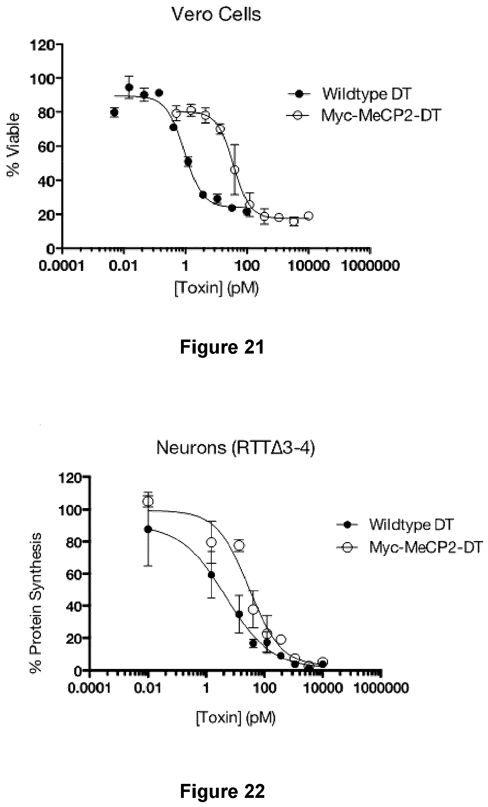

**Figure 21** demonstrates proof of cystosolic delivery of MeCP2e1 into cells by fusion protein toxicity in Vero cells. Vero cell toxicity based on Presto-Blue cell viability assay. EC₅₀ values for WT DT and Myc-MeCP2-DT were 0.93 ± 1.16 and 37.33 ± 1.13 pM, respectively.

**Figure 22** shows proof of cystosolic delivery of MecP2e1 into iPSC-derived neurons from Rett Syndrome patient fibroblasts. It shows the effect of Myc-MeCP2-DT on protein synthesis in 2 week old RTTΔ3-4 neurons as measured by ³H-leucine incorporation assay. EC₅₀ values for WT and Myc-MeCP2-DT were 4.72 ± 1.71 and 29.56 ± 1.39 pM, respectively.

### SMN1

Spinal Muscular Atrophy (SMA) is caused by mutations in the SMN1 gene, resulting in a defective or missing protein product. Disease severity is moderated by a gene duplication event unique to humans that resulted in SMN2, a gene identical to SMN1 except for a C to T transition resulting in alternative splicing and exclusion of exon 7 from most SMN2 transcripts. Myc-SMN-dtA-dtB has been expressed and purified. The N-terminal Myc tag is linked to SMN with a GSG linker.

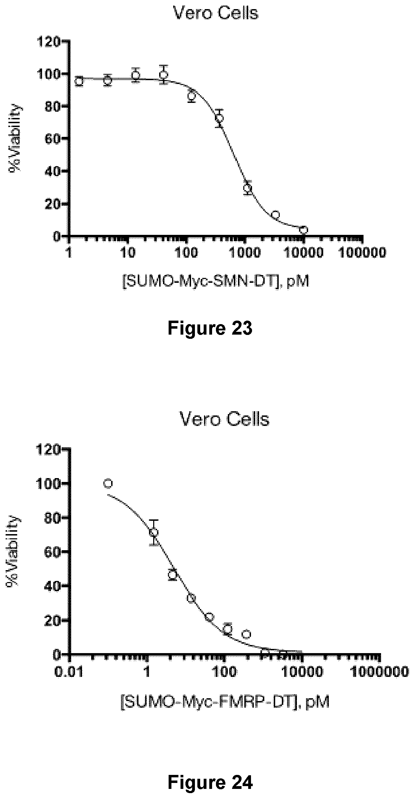

**Figure 23** depicts results of fusion protein toxicity assays indicating that SMN is delivered into the cytosol Vero cells. Vero cell toxicity was based on the Presto-Blue cell viability assay. The EC₅₀ value for SUMO-Myc-SMN-DT was 648.2 ± 1.09 pM. Constructs can also contain the inactivating mutations K51E and E148K in dtA, when a non-toxic version is desirable.

### FMRP

Fragile X Syndrome is characterized by the lack of the FMRP protein product due to the expansion of a CGG trinucleotide repeat region in the 5'UTR of the FMR1 gene. This results in hypermethylation and silencing of FMR1. FMRP is a translational regulator with many downstream gene targets. Myc-FMRP-dtA-dtB was expressed and purified. The N-terminal Myc tag is linked to FMRP with a GSG linker.

**Figure 24** depicts results of fusion protein toxicity assays indicating that SMN is delivered into the cytosol Vero cells. Vero cell toxicity was based on the Presto-Blue cell viability assay. The EC₅₀ value for SUMO-Myc-FMRP-DT was 4.95 ± 1.15 pM.

### PNP

PNP-deficiency is a metabolic disorder that results in immunodeficiency, as well as neurological symptoms such as developmental decline and mental retardation. The PNP enzyme catalyzes the conversion of inosine and guanosine into hypoxanthine. PNP-dtA-dtB has been expressed and purified, wherein PNP is linked to dtA with a (G4S)₂ linker.

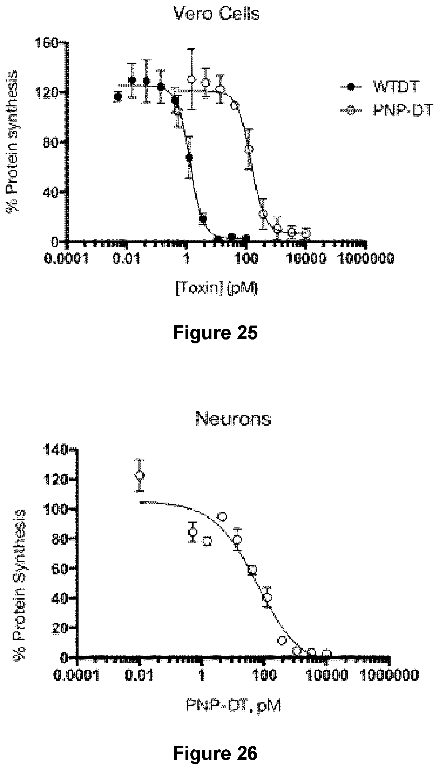

**Figure 25** depicts results indicating that PNP fusion proteins have been delivered into the cytosol of Vero cells. Vero cell toxicity was based on the ³H-leucine incorporation assay. EC50 values for WT DT and PNP-DT were 1.31 ± 1.11 and 145.8 ± 1.15 pM, respectively.

**Figure 26** depicts results indicating that PNP fusion proteins have been delivered into the cytosol of two-week old wild type (WT) neurons. Toxicity was again based on the ³H-leucine incorporation assay. The EC₅₀ value for PNP-DT was 61.34 ± 1.59 pM.

The construct dtA-PNP-dta-dtB has also been expressed and purified, wherein dtA is linked to PNP via a GSG linker and PNP is linked to dta using a GSG linker.

### Cargo for Genome Editing

### Cas9

The recent application of the Clustered Regularly Interspaced Short Palindromic Repeats (CRISPR) system to targeted genome editing has radically changed the field of molecular biology. The system involves the DNA endonuclease CRISPR-associated protein 9 (Cas9), which is guided to its DNA target sequence by a -97nt RNA molecule (gRNA). *In vitro,* both Cas9 and gRNA are delivered on a plasmid by transfection, while *in vivo,* the use of viral vectors is the preferred delivery method. There have also been reports of other delivery methods for Cas9, such as CPPs, or lipid-based methods. The construct Cas9-dtA-dtB has been expressed and purified. The Cas9 sequence is from *S*. *pyogenes.* Cas9 is linked to dtA with a (G4S)₂ linker. Cas9 is flanked on both sides by SV40 nuclear localization sequences (NLS). Cas9 is preceded by an N-terminal His tag (6xHis).

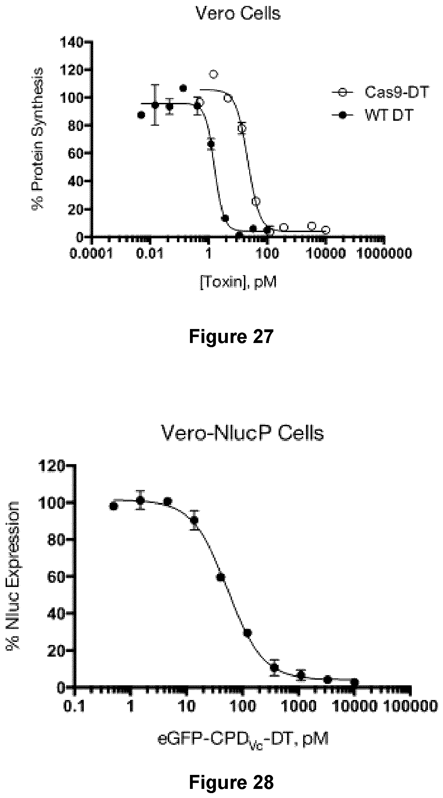

**Figure 27** demonstrates delivery of Cas9 into the cytosol of Vero cells by fusion protein toxicity, as above. Vero cell toxicity was based on the 3H-leucine incorporation assay. The EC₅₀ values for WT DT and Cas9-DT was 1.63 ± 1.10 and 21.74 ± 1.09 pM, respectively.

A further version of this construct contains the inactivating mutations K51E and E148K. Cas9 is the largest cargo protein yet delivered (160 kDa).

### Cargo Release

### CPD

In order to release the native cargo protein upon delivery into the cytosol, a cysteine protease domain (CPD) from each of *Clostridium difficile* toxin B (CPD_{Cd}) and *Vibrio cholera* MARTX toxin (CPD_{Vc}) has been employed. The construct eGFP-CPDₓₓ-dtA-dtB as been expressed and purified, wherein CPDxx is either CPD_{Vc} or CPD_{Cd}. CPD is linked to dtA with a GSG linker. Both domains undergo self-cleavage at their own N-termini upon binding of the small molecule inositol hexakisphosphate (IP6), which is exclusively located in the cytoplasm of mammalian cells. They are autoprocessing, and "self-clearing".

**Figure 28** depicts delivery of eGFP-CPD_{Vc}-DT into the cytosol of Vero-NlucP cells by fusion protein toxicity. Vero-NlucP cell toxicity was based on the NanoGlo assay. The EC₅₀ values for eGFP-CPD_{Vc}-DT was 54.44 ± 1.06 pM.

Similar release constructs can be made for other cargo proteins, for example with either CPD_{Vc} or CPD_{Cd} and the cargo proteins described above. To date, the following CPD constructs have been made:

CPD_{cd}-(G4S)₂-dtA-dtB;

eGFP-CPD_{vc}-(GSG)-dtA-dtB;

and

eGFP-CPD_{cd}-(GSG)-dtA-dtB;

Data generated to date indicates that the presence of CPD results in release of (eGFP) in the presence of InsP6 *in vitro* (data not shown). Western blotting data indicates that that cargo is similarly released *in vivo* (data not shown).

Other constructs that have been made to date include:

MeCP2-CPD_{vc}-(G4S)₂-dtA-dtB;

and

PNP-(G4S)₂-CPD_{cd}-(G4S)₃-dtA-dtB.

These constructs are expected to be capable of releasing their cargo upon delivery to the cytosol. Non-toxic variants can be readily made, using dta in place of dtA.

### EXAMPLE 13

For some therapeutic applications, it may be desirable to reduce the immunogenicity of DT domains. To this end, DT domains are mutated, e.g., to reduce their antigenicity, for example by removing T-cell epitopes.

### EXAMPLE 14

For some applications, it may be advantageous to reduce the size of the construct, e.g. to provide a smaller construct and/or to reduce potential for antigenicity. Experiments were conducted to assess the function of the DTA domain.

### Materials and Methods

### Constructs

The glucosyl transferase domain (GTD; SEQ ID NO: 28) from *Clostridium difficile* toxin B was linked to dta or Δdta-dtB via a GSG linker to generate the constructs GTD-dta-dtB and GTD-Δdta-dtB. The latter retains a small DTA fragment (Δdta); most of the functional domain proper has been deleted, leaving SEQ ID NO: 26. Δdta thus extends from a cysteine corresponding to position 186 of SEQ ID NO: 1 through its C-terminus. This cysteine residue was retained as it is involved in disulphide bond formation. The CPD domain from *Vibrio cholerae* was subsequently cloned between GTD and dtB upstream of the linker yielding the construct GTD-CPD-Δdta-dtB with no linker sequence between the GTD and CPD domains. All three constructs were cloned with an N-terminal polyhistidine tag and a C-terminal Strep-tag^{™} II sequence (SEQ ID NO: 27) for affinity purification using the GE-Healthcare StrepTactin^{™} purification system.

### Expression and Purification

GTD DT chimeras were expressed as N-terminal His-tagged proteins in *E*. *coli* BL21(DE3) cells, induced with 1 mM isopropyl-β-d-1-thiogalactopyranoside (IPTG) for 4 hours at 21°C. Cells were harvested by centrifugation, re-suspended in lysis buffer (20 mM Tris-HCl pH 8.0, 0.5 M NaCl, 20 mM imidazole, benzonase, lysozyme and Protease inhibitor cocktail) and lysed by an EmulsiFlex C3 microfluidizer (Avestin) at 15,000 psi. The lysates were centrifuged at 18,000 × g for 20 minutes. His -tagged proteins were purified by Ni-affinity chromatography using a His-Trap FF column (GE-Healthcare). Protein was eluted in 20 mM Tris-HCl pH 8.0, 0.5 M NaCl and 125 mM imidazole and loaded directly onto a 5 mL StrepTrap HP column (GE-Healthcare). Pure protein was then eluted from the StrepTrap HP column in 20 mM Tris-HCl pH 8.0, 150 mM NaCl and 2.0 mM desthiobiotin.

### Cell Viability Assay

Vero cells were plated at 4000 cells/well in a 96-well cell culture plate and allowed to attach overnight at 37°C and 5% C0₂. The next day, fusion toxins were added at various concentrations in DMEM (10% FBS, 1% penicillin/streptomycin). After 48 hours, 100 µl of Presto-Blue (Life Technologies) cell viability dye was added to all wells and incubated at 37°C for 2 hours. Fluorescence was measured in a SpectraMax M5e microplate reader (Molecular Devices) (Ex/Em 555/585 nm). Results were quantified and fit to a sigmoidal function in GraphPad Prism.

### Results and Discussion

### Delivery of cargo in the absence of the catalytic A domain of DT

Inhibition of protein synthesis by DTA has been a useful tool to demonstrate delivery of cargo-DTA chimeras to the cytosol, but tethering to DTA may interfere with certain cargo protein's activity through steric interference and/or cellular localization in some applications. While inclusion of a cysteine protease domain between cargo and DTA would allow for release of native cargo protein, a question remained of whether cargo could be fused more directly to the DTB domain, i.e. with less of DTA, thereby decreasing the size and complexity of cargo being delivered into the cell. Reducing or eliminating the A domain would have the additional benefit of reducing the potential for immunogenicity in future *in vivo* applications of this technology. Fusion proteins containing the glucosyltransferase domain (GTD) from *Clostridium difficile* fused to dta-dtB, CPD-Δdta -dtB or simply Δdta-dtB were cloned, expressed and purified. Upon reaching the cytosol, the GTD inactivates small Rho family GTPases (Rac1, RhoA, Cdc42), thereby disrupting actin cytoskeleton organization resulting in an acute rounding phenotype and eventual apoptosis (Just 1995). In the absence most of the DTA domain, the cytotoxicity of the GTD was used to compare its cytosolic entry in these three different delivery paradigms.

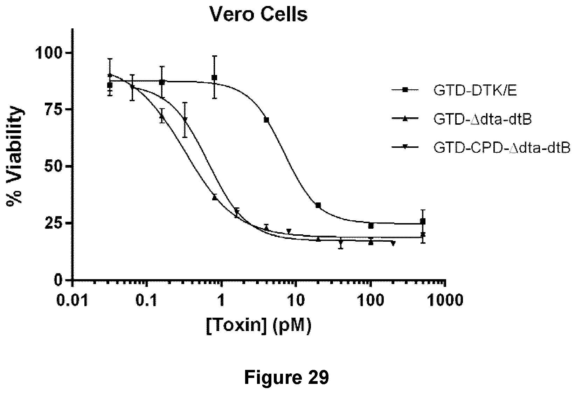

**Figure 29** shows the effect of removing most of the A domain, which appears to have no effect on the ability of the GTD cargo to reach the cytosol. Remarkably, there is a small increase in toxicity of the two constructs lacking most of the DTA domain, however, this difference is small and could be due to an effect on the enzymatic activity of GTD as both GTD-Δdta-CPD-dtB and GTD-Δdta-dtB result in delivery of free GTD while GTD-dta remain fused upon delivery.

The dispensable nature of the DTA domain in cargo translocation has important implications for the DT delivery platform and speaks to the versatility and modularity of this system. This finding also deviates significantly from the widely accepted model of DT translocation, in which DTA is absolutely required and is thought to make up part of the translocation machinery.

### EXAMPLE 15

### Ras-Targeting

### Introduction

Since the discovery of first mutations in Ras genes in various human cancers in 1982, Ras has been the major focus in cancer research. It is now well known that the three Ras genes (KRas, NRas and HRas) constitute the most frequently mutated oncogene family in human cancer and mutations in these Ras genes are found in 20-30% of all human cancers, placing the Ras variants among the most prevalent drivers of cancer^{26, 27}. Despite the frequent involvement of Ras in the onset and progression of cancer, efficient inhibition of oncogenic Ras with small molecules has been very difficult due to their relatively smooth, unpocketed surface and high affinity for its substrate. Moreover, attempts to inhibit downstream effector pathways showed only limited success owing to development of drug resistance and complex feedback mechanisms²⁸. To date, clinically effective anti-Ras therapies remain elusive, prompting a perception that Ras may be undruggable.

Recently, a bacterially-derived enzyme was identified that specifically degrades Ras proteins in human cells. Ectopic expression of this enzyme, RRSP (Ras/Rap1-specific endopeptidase), was shown to cleave mutant KRas, NRas and HRas and inhibit cell growth²⁹, leading to the hypothesis that this enzyme could halt cancer progression in cells with unregulated Ras activity. However, this enzyme would not be an effective anti-cancer biologic unless it is able to reach its target - Ras - which is tethered in the inner leaflet of plasma membranes in the cytosol of cells. Herein is described an intracellular protein delivery platform, which introduces impermeable proteins into the cytosol of target cells. The protein delivery platform is based on a bacterial toxin, called diphtheria toxin (DT) which has an intrinsic mechanism to cross plasma membranes and reach the cytosol of specific cells with high efficiency.

The ability of DT to cross cell membranes and deliver proteins with varying structures, sizes and stabilities as N-terminal fusions into mammalian cells using a detoxified toxin, rendered it an ideal delivery vector for protein therapeutics. The goal of this example is to use the nontoxic variant of DT (denoted as dta-dtB, where dta contains two mutations, K51E and E148K in the dtA domain) as a cytosolic delivery platform to deliver RRSP into the cytosol of cancer cells and inhibit cancer growth and progression.

### Materials and Methods

### Cell lines and media

HeLa epithelial cells were cultured in Eagle's Minimum Essential Medium (EMEM, Wisent Bioproducts). HCT116 colorectal cancer cells were cultured in McCoy's 5A medium (Wisent Bioproducts). BxPC3, CFPACI, and HPAFII cells were cultured in RPMI1640. All media were supplemented with 10% fetal bovine serum, 100 U/mL penicillin, and 100 µg/mL streptomycin (Wisent Bioproducts). RealTime-Glo MT Cell Viability Assay was obtained from Promega Corporation.

### Generation of RRSP-DT Chimeras

Point mutations were made in the DT E148S plasmid using QuikChange Lightning Multi Site-Directed Mutagenesis Kit (Agilent Technologies) to prepare catalytically inactive DT (dta-dtB). A plasmid containing RRSP DNA sequences was a kind gift of Dr. Karla J. F. Satchell (Northwestern University, Chicago, IL). RRSP DNA sequences were amplified (see, e.g. SEQ ID NO: 26 for the coding sequence) and integrated into the dta-dtB plasmid using Gibson Assembly (New England BioLabs) to generate RRSP-dta-dtB. RRSP fused to dtB (RRSP-dtB - elsewhere termed RRSP-Δdta-dtB) and to the receptor domain alone (RRSP-dtR) (i.e., without the translocation domain in the B domain) were generated in the same manner as RRSP-dta-dtB.

### Expression and Purification of RRSP-DT Chimera

RRSP (SEQ ID NO: 30) and RRSP fusion proteins were expressed as N-terminal His-Sumo-tagged and C-terminal Strep-tagged II proteins in *E. coli* NiCo21 (DE3). Overnight cultures were diluted in 1:30 in fresh Terrific Broth containing 50 µg/ml kanamycin and grown to OD600 = 0.8 at 37°C before inducing the cultures with 1 mM isopropyl-β -D -1-thiogalactopyranoside (IPTG) for 5 h at 25°C. *E*. *coli* cells were harvested by centrifugation at 5,500 rpm and resuspended in lysis buffer (20 mM Tris-HCl pH 8.0, 500 mM NaCl, 20 mM imidazole, benzonase, lysozyme and protease inhibitor cocktail) and lysed by an EmulsiFlex C3 microfluidizer (Avestin) at 15,000 psi. The lysates were centrifuged at 14,000 g for 20 mins. His-Sumo-tagged proteins were purified by Ni-affinity chromatography using a His-Trap Crude FF column (GE Healthcare). Proteins were further purified using a StrepTrap HP column (GE Healthcare). After the second purification, the His-Sumo tag was removed by adding 1U of Sumo Protease to 90 µg of purified proteins in 20 mM Tris-HCl pH 8.0, 150 mM NaCl and 10 mM imidazole. The cleavage reaction mixture was incubated at 30°C for 1 h followed by purification using His-Pure Ni-NTA resin (Thermo Scientific) to remove the His-Sumo protease and His-Sumo tag from the purified RRSP and RRSP fusion proteins. Purified proteins were concentrated using Millipore Amicon Ultra 50K spin concentrators and glycerol was added so that the final buffer was 20 mM Tris-HCl pH 8.0, 150 mM NaCl, 10 mM imidazole, 8% glycerol. Protein concentration was determined using a nanophotometer (Implen) and purity was estimated using SDS-PAGE. Proteins were stored at -80°C until used.

### Intoxication of cells with RRSP-DT chimeras

HeLa cells were seeded at 2 × 10⁵ cells/well overnight into a 6-well plate. HCT116 cells were seeded at 3 × 10⁵ cells/well for 48 h. Before intoxication, the media was exchanged for fresh media and then indicated toxin concentrations were added to the media and incubated for the times indicated in the legend at 37°C with 5% CO2.

### Western blotting

A total of 3-4 × 10⁵ treated cells were washed with PBS and resuspended in 80 µl of lysis buffer (20 mM Tris-HCl pH 8.0, 150 mM NaCl, 1% Triton X-100). 30 µl of lysate was resuspended in 10 µl of 4 × Laemmli sample buffer and boiled for 10 min. Forty microliters of lysate were separated by SDS-PAGE and transferred to nitrocellulose (Amersham), blocked with 5% milk/Tris-buffered saline containing 0.1% Tween-20 (TBST) for 1 h at room temperature, and probed with a 1/1,000 dilution of pan-Ras mouse mAb RAS10 (05-516, EMD Millipore) and actin mouse mAb AC-40 (Sigma-Aldrich). Following an overnight incubation with the primary antibodies, the blot was washed with TBS-T and incubated with a 1/5,000 dilution of anti-mouse horseradish peroxidase for 1 hr. After the final washes in TBST, chemiluminescent detection was carried out using SuperSignal West Femto substrate (Thermo Pierce) and imaged in ChemiDoc MP Imaging System (BioRad).

### RealTime-Glo assay

BxPC3 pancreatic adenocarcinoma cells (625 cells/well) were plated in 40-ul media containing 2 × RealTime-Glo reagents in a 96-well white, clear flat bottom plate (Corning). RRSP-Δdta-dtB titration was prepared at 2 × concentrations in media and added to the plate at an equal volume. Luminescence was read at 1, 4, 9, 24, 48 and 72 h post toxin addition on a SpectraMax M5 plate reader.

### Results

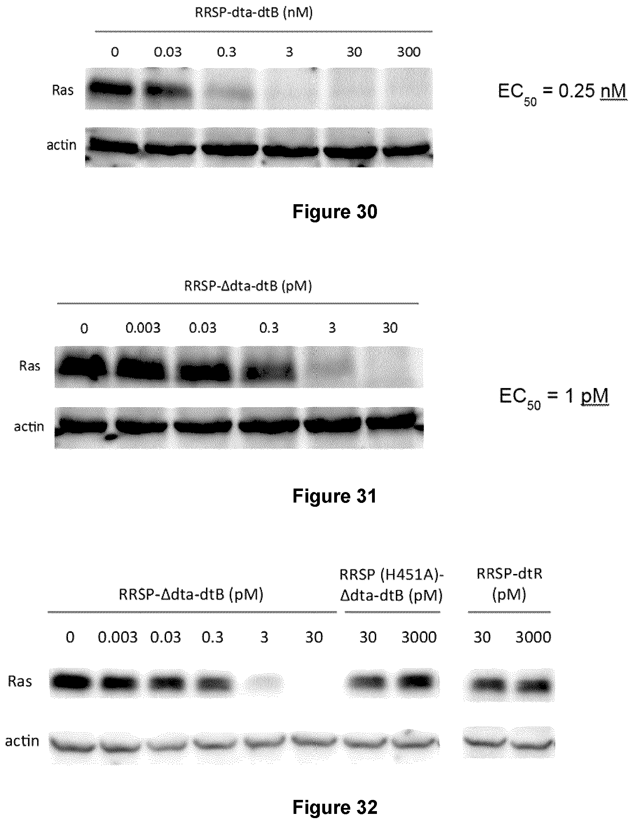

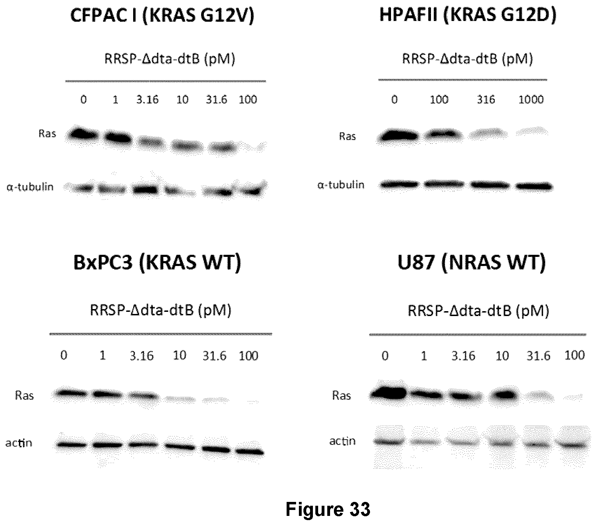

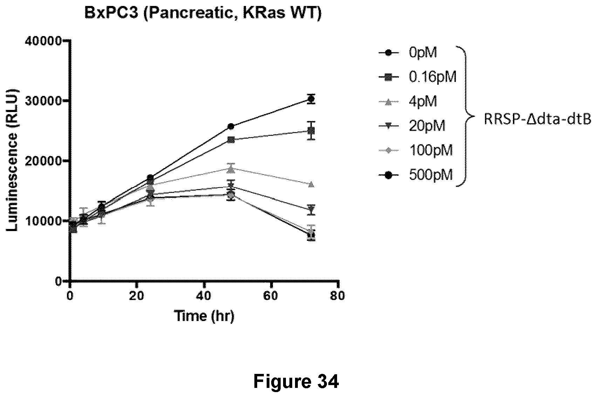

A chimeric protein consisting of RRSP fused to a nontoxic variant of DT (RRSP-dta-dtB) with an intervening (G₄S)₂ linker was generated. Upon 24 h incubation of RRSP-dta-dtB on HeLa cells, a complete loss of total RAS proteins inside cells was detected at a sub-nanomolar concentration of the enzyme (EC50 = 0.25 nM), as measured by immunoblotting with anti-RAS antibody (**Figure 30**). To improve the RAS cleavage efficiency, the delivery platform was optimized by either removing the dta domain or inserting the Auto-Processing Domain (APD) in between RRSP and dtB. In both cases, 14 amino acid residues from the C-terminus of the dta domain, denoted as Δdta, were maintained to conserve the disulfide bond and the furin cleavage site, essential for toxin entry into cells. The resulting constructs were represented as RRSP-Δdta-dtB and RRSP-APD-Δdta-dtB, respectively, and a (G₄S)₂ linker was introduced between RRSP and Δdta and between RRSP and APD. Remarkably, these second generation constructs were 300 times more potent than the first generation construct with an EC50 of 1 pM in HeLa cells (**Figure 31**). The results demonstrated for the first time that the dtB domain with virtually all of the dta domain removed is sufficient to deliver active RRSP inside cells. As further demonstration that RRSP-Δdta-dtB could be used as a potential cancer therapeutic, the ability of RRSP-Δdta-dtB to degrade RAS in cancer cell lines bearing common RAS point mutations was tested. Intoxicating HCT116 colorectal carcinoma cells, which express KRAS with a G13D mutation, with RRSP-Δdta-dtB resulted in undetectable levels of RAS at a picomolar concentration of the enzyme (**Figure 32**). The effect of RAS cleavage was solely due to the activity of RRSP, as its inactive mutant control (RRSP_H451A) did not affect intracellular RAS levels. Moreover, as expected, RRSP fused to the receptor-binding domain of DT (RRSP-dtR) did not cleave RAS in HCT116 cells as it lacks the ability to translocate RRSP into the cytosol. Mutant KRAS proteins in cancer cell lines (CFPACI - G12V and HPAFII - G12D) were likewise degraded, demonstrating that RRSP can effectively intoxicate cells with activating RAS mutations (**Figure 33**). To investigate whether the cleavage of RAS in RRSP-Δdta-dtB-treated cells would lead to disruption in cell proliferation, a cell proliferation assay was performed. BxPC3 pancreatic cancer cells were intoxicated at varying concentrations of RRSP-Δdta-dtB and luminescence emitted by live cells was recorded at 1, 4, 9, 24, 48, or 72 h post toxin addition. As shown in **Figure 34****,** untreated cells showed steady increase in luminescence over the course of 72 hours, indicating cellular proliferation. In contrast, a decrease in luminescence was observed in RRSP-Δdta-dtB-treated cells, and the most significant impairment in proliferation was found in cells treated with the highest enzyme concentration, indicating that the cleavage of RAS led to reduced cellular proliferation.

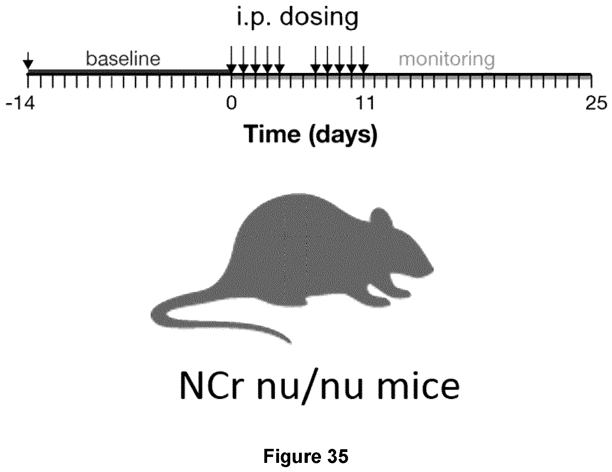

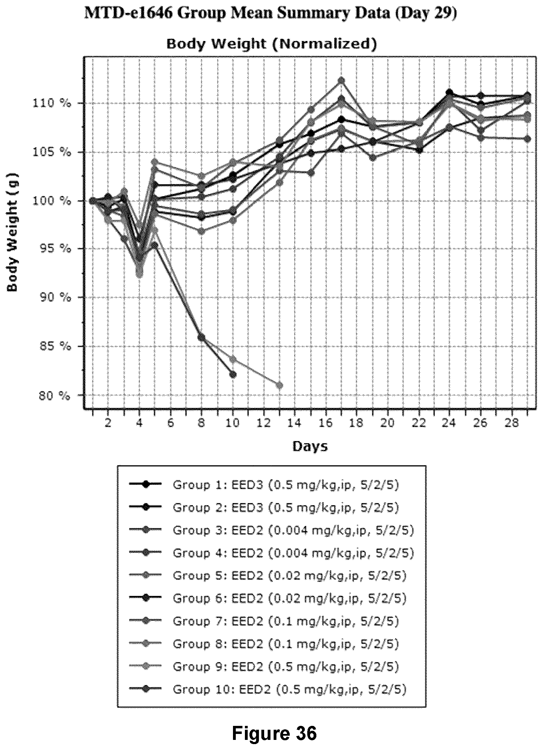

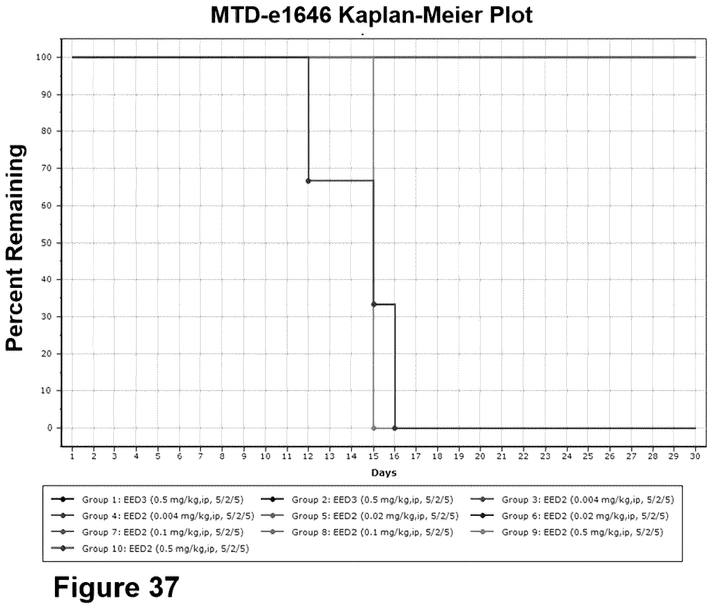

To determine the maximum tolerated dose (MTD) for RRSP-Δdta-dtB and an enzymatically inactivated form of RRSP (RRSP_H451A), an MTD study was conducted in NCr nu/nu mice. Each group contained 3 mice. To mice that were aged between 8-12 weeks, we administered 10 doses intraperitoneally (i.p.), once per day during the week Monday to Friday for two weeks with a two day break on the weekend (**Figure 35**). We injected 15 female and 15 male mice and monitored their body weight as shown in Figure 36. Any individual animal with a single observation of > than 30% body weight loss or three consecutive measurements of >25% body weight loss was euthanized (**Figures 36** **and** **37**). Below 0.5mpk, no significant weight loss was observed, and thus all mice survived the duration of the study (**Figures 36** **and** **37**).

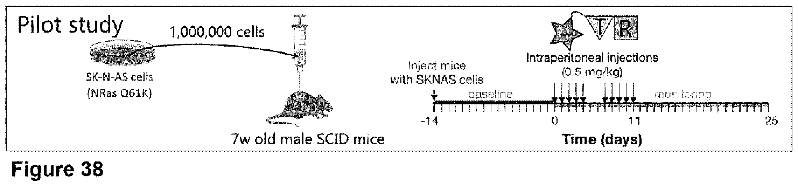

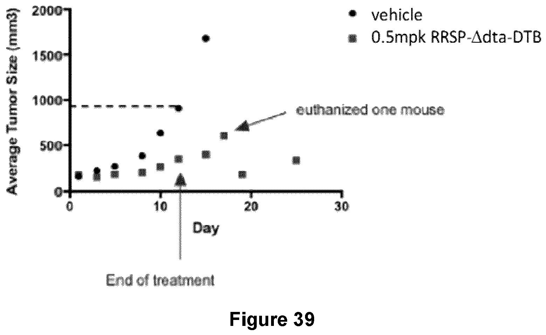

Further to this, a small study was conducted to test whether RRSP-Δdta-dtB could shrink a human xenograft (see schematic in **Figure 38**). To this end, 1 million SK-N-AS cells bearing a mutation in NRas (Q61K) were implanted into 7-week-old male SCID mice. The xenografts were allowed to grow for approximately 2 weeks after which a 0.5 mpk of RRSP-Δdta-dTB or vehicle was injected i.p. into mice once a day (q.d.) for 10 days with a two day break in the middle. Each data point represents the mean of 3 mice per group. Compared to vehicle, 0.5mpk of RRSP-Δdta-dTB treatment reduced tumor xenograft growth (**Figure 39**).

The ability of DT to deliver RRSP was compared to a previously described anthrax toxin-based delivery system (see c). To this end, an anthrax toxin lethal factor-RRSP fusion protein (LF_{N}-RRSP) was constructed, and a receptor binding moiety of the toxin, PA83. Consistent with the previously reported data, 3 nM LF_{N}-RRSP in the presence of 7 nM PA83, resulted in partial cleavage of Ras in HCT116 cells after 24 hours incubation (**Figure 40****, Panel A**). Subsequent one in five dilutions of the LF_{N}-RRSP + PA83 mixture resulted in no Ras degradation (**Figure 40****, Panel A**)**.** Under the same experimental conditions, RRSP-Δdta-dtB degraded Ras down to single-digit picomolar concentrations (**Figure 40****, Panel B**). Comparing the effective concentration at which 50% of Ras is cleaves as an index of delivery, these data show that for equivalent efficacy, the DT-based delivery system requires nearly 1000-fold less delivery platform than the anthrax delivery platform (**Figure 40****, Panel C**).

### Discussion

RAS proteins are small GTPases, which serve as a crucial signaling hub that regulates various cellular processes, including proliferation, survival and differentiation. The discovery of constitutively activating RAS mutations in human tumors has initiated intense research on RAS, yet, to date, none of the RAS-targeted drugs have shown clinical efficacy. Recently, an effector domain of the multifunctional-autoprocessing repeats-in-toxin (MARTX) toxin from *Vibrio vulnificus* that specifically cleaves the Switch I region of all three isoforms of RAS proteins has been identified, and the enzyme has been named RRSP. In order to achieve its full therapeutic efficacy, however, RRSP must reach the cytosol of cancer cells where mutant RAS proteins are present.

Here, it was demonstrated that using an engineered DT, active RRSP was successfully delivered into the cytosol of cancer cells and degraded RAS with extremely high efficiency. It was also demonstrated that the optimization of the DT-based delivery platform resulted in ~300-fold increase in potency. Importantly, several cancer cell lines carrying common RAS mutations were also efficiently cleaved by the intracellularly-delivered RRSP. A safe dose was determined for the DT-based delivery platform, and RRSP-Δdta-dTB was subsequently shown to be able to reduce tumor xenograph growth at this dose. The DT-base delivery platform was shown to be approximately 1000-fold better than a previously described anthrax toxin delivery system at delivering RRSP to cells, reducing Ras to single-digit picomolar concentrations.

Collectively, the results demonstrate the great potential of DT-based delivery platforms for delivery of RRSP as an anti-RAS cancer therapy. In particular the surprising increase in potency achieved is such that this construct is expected to be amenable to *in vivo* and/or therapeutic applications.

In the preceding description, for purposes of explanation, numerous details are set forth in order to provide a thorough understanding of the embodiments. However, it will be apparent to one skilled in the art that these specific details are not required. In other instances, well-known electrical structures and circuits are shown in block diagram form in order not to obscure the understanding. For example, specific details are not provided as to whether the embodiments described herein are implemented as a software routine, hardware circuit, firmware, or a combination thereof.

The above-described embodiments are intended to be examples only. Alterations, modifications and variations can be effected to the particular embodiments by those of skill in the art. The scope of the claims should not be limited by the particular embodiments set forth herein, but should be construed in a manner consistent with the specification as a whole.

### REFERENCES

1 Williams, D. P. et al. Diphtheria toxin receptor binding domain substitution with interleukin-2: genetic construction and properties of a diphtheria toxin-related interleukin-2 fusion protein. Protein engineering 1, 493-498 (1987).
2 Jean, L. F. & Murphy, J. R. Diphtheria toxin receptor-binding domain substitution with interleukin 6: genetic construction and interleukin 6 receptor-specific action of a diphtheria toxin-related interleukin 6 fusion protein. Protein engineering 4, 989-994 (1991).
3 Aullo, P. et al. A recombinant diphtheria toxin related human CD4 fusion protein specifically kills HIV infected cells which express gp120 but selects fusion toxin resistant cells which carry HIV. The EMBO journal 11, 575-583 (1992).
4 Madshus, I. H., Olsnes, S. & Stenmark, H. Membrane translocation of diphtheria toxin carrying passenger protein domains. Infection and immunity 60, 3296-3302 (1992).
5 Stenmark, H., Moskaug, J. O., Madshus, I. H., Sandvig, K. & Olsnes, S. Peptides fused to the amino-terminal end of diphtheria toxin are translocated to the cytosol. The Journal of cell biology 113, 1025-1032 (1991).
6 Wiedlocha, A., Madshus, I. H., Mach, H., Middaugh, C. R. & Olsnes, S. Tight folding of acidic fibroblast growth factor prevents its translocation to the cytosol with diphtheria toxin as vector. The EMBO journal 11, 4835-4842 (1992).
7 Klingenberg, O. & Olsnes, S. Ability of methotrexate to inhibit translocation to the cytosol of dihydrofolate reductase fused to diphtheria toxin. The Biochemical journal 313 ( Pt 2), 647-653 (1996).
8 Ainavarapu, S. R., Li, L., Badilla, C. L. & Fernandez, J. M. Ligand binding modulates the mechanical stability of dihydrofolate reductase. Biophysical journal 89, 3337-3344, doi:10.1529/biophysj.105.062034 (2005).
9 Francis, J. W. et al. A survival motor neuron:tetanus toxin fragment C fusion protein for the targeted delivery of SMN protein to neurons. Brain research 995, 84-96 (2004).
10 Fu, H., Blanke, S. R., Mattheakis, L. C. & Collier, R. J. Selection of diphtheria toxin active-site mutants in yeast. Rediscovery of glutamic acid-148 as a key residue. Advances in experimental medicine and biology 419, 45-52 (1997).
11 Murphy, J. R. Mechanism of diphtheria toxin catalytic domain delivery to the eukaryotic cell cytosol and the cellular factors that directly participate in the process. Toxins 3, 294-308, doi:10.3390/toxins3030294 (2011).
12 Kiyokawa, T., Williams, D. P., Snider, C. E., Strom, T. B. & Murphy, J. R. Protein engineering of diphtheria-toxin-related interleukin-2 fusion toxins to increase cytotoxic potency for high-affinity IL-2-receptor-bearing target cells. Protein engineering 4, 463-468 (1991).
13 Choudhary, S., Mathew, M. & Verma, R. S. Therapeutic potential of anticancer immunotoxins. Drug discovery today 16, 495-503, doi:10.1016/j.drudis.2011.04.003 (2011).
14 Alewine, C., Hassan, R. & Pastan, I. Advances in Anticancer Immunotoxin Therapy. The oncologist, doi:10.1634/theoncologist.2014-0358 (2015).
15 Mazor, R. et al. Identification and elimination of an immunodominant T-cell epitope in recombinant immunotoxins based on Pseudomonas exotoxin A. Proc Natl Acad Sci U S A 109, E3597-3603, doi:10.1073/pnas.1218138109 (2012).
16 Ballard, J. D., Collier, R. J. & Starnbach, M. N. Anthrax toxin-mediated delivery of a cytotoxic T-cell epitope in vivo. Proceedings of the National Academy of Sciences of the United States of America 93, 12531-12534 (1996).
17 Leppla, S. H., Arora, N. & Varughese, M. Anthrax toxin fusion proteins for intracellular delivery of macromolecules. Journal of applied microbiology 87, 284 (1999).
18 Bachran, C. et al. Anthrax toxin-mediated delivery of the Pseudomonas exotoxin A enzymatic domain to the cytosol of tumor cells via cleavable ubiquitin fusions. mBio 4, e00201-00213, doi:10.1128/mBio.00201-13 (2013).
19 Liao, X., Rabideau, A. E. & Pentelute, B. L. Delivery of antibody mimics into mammalian cells via anthrax toxin protective antigen. Chembiochem : a European journal of chemical biology 15, 2458-2466, doi:10.1002/cbic.201402290 (2014).
20 Benson, E. L., Huynh, P. D., Finkelstein, A. & Collier, R. J. Identification of residues lining the anthrax protective antigen channel. Biochemistry 37, 3941-3948, doi:10.1021/bi972657b (1998).
21 Krantz, B. A. et al. A phenylalanine clamp catalyzes protein translocation through the anthrax toxin pore. Science 309, 777-781, doi:10.1126/science. 1113380 (2005).
22 Zornetta, I. et al. Imaging the cell entry of the anthrax oedema and lethal toxins with fluorescent protein chimeras. Cellular microbiology 12, 1435-1445, doi:10.1111/j.1462-5822.2010.01480.x (2010).
23 Nagata, S. & Pastan, I. Removal of B cell epitopes as a practical approach for reducing the immunogenicity of foreign protein-based therapeutics. Advanced drug delivery reviews 61, 977-985, doi:10.1016/j.addr.2009.07.014 (2009).
24 King, C. et al. Removing T-cell epitopes with computational protein design. Proc Natl Acad Sci U S A 111, 8577-8582, doi:10.1073/pnas.1321126111 (2014).
25. Just I, Selzer J, Wilm M, von Eichel-Streiber C, Mann M, Aktories K (1995) Glucosylation of Rho proteins by Clostridium difficile toxin B. (1995) Nature 8: 500-503.
26. Forbes, S.A., et al., COSMIC: mining complete cancer genomes in the Catalogue of Somatic Mutations in Cancer. Nucleic Acids Res, 2011. 39(Database issue): p. D945-50.
27. Prior, I.A., P.D. Lewis, and C. Mattos, A comprehensive survey of Ras mutations in cancer. Cancer Res, 2012. 72(10): p. 2457-67.
28. Lito, P., N. Rosen, and D.B. Solit, Tumor adaptation and resistance to RAF inhibitors. Nat Med, 2013. 19(11): p. 1401-9.
29. Antic, I., et al., Site-specific processing of Ras and Rap1 Switch I by a MARTX toxin effector domain. Nat Commun, 2015. 6: p. 7396.

## Claims

1. A recombinant molecule for use in treatment of cancer comprising increased RAS activity in cells compared to a corresponding healthy state, the recombinant molecule comprising a cargo polypeptide (C), a diphtheria toxin enzymatic fragment (DTA), and a diphtheria toxin translocation fragment (DTB), having a general structure:
x-C-y-DTA-DTB
wherein:
x is a polypeptide or absent,
C comprises a Ras/Rap1-specific endopeptidase (RRSP) from *Vibrio vulnificus* having the sequence set forth in SEQ ID No: 30,
y comprises a linker comprising (G₄S)₂ (SEQ ID No: 11),
DTA consists of amino acids having the sequence set forth in SEQ ID No: 26, and
DTB comprises amino acids having the sequence set forth in SEQ ID No: 3,
wherein the DTA is linked to the DTB by way of a disulphide linkage.

2. The recombinant molecule for use according to claim 1, wherein said increased RAS activity is caused by an activating mutation.

3. The recombinant molecule for use according to claim 1 or 2, wherein the cancer is a pancreatic cancer.

4. The recombinant molecule for use according to any of claims 1 to 3, wherein the use is intraperitoneal.

## Patentansprüche

1. Rekombinantes Molekül zur Verwendung bei der Behandlung von Krebs, der eine erhöhte RAS-Aktivität in Zellen im Vergleich zu einem entsprechenden gesunden Zustand umfasst, wobei das rekombinante Molekül ein Frachtpolypeptid (C), ein Diphtherietoxin-Enzymfragment (DTA) und ein Diphtherietoxin-Translokationsfragment (DTB) umfasst und eine folgende allgemeine Struktur aufweist:
x-C-y-DTA-DTB
wobei:
x ein Polypeptid ist oder fehlt,
C eine Ras/Rap1-spezifische Endopeptidase (RRSP) aus *Vibrio vulnificus* mit der Sequenz, wie festgelegt in SEQ ID No: 30, umfasst,
y einen Linker umfasst, der (G₄S)₂ (SEQ ID No: 11) umfasst,
DTA aus Aminosäuren besteht, die die Sequenz aufweisen, wie festgelegt in SEQ ID No: 26, und
DTB Aminosäuren umfasst, die eine Sequenz aufweisen, wie festgelegt in SEQ ID No: 3,
wobei das DTA über eine Disulfidbindung mit dem DTB verbunden ist.

2. Rekombinantes Molekül zur Verwendung nach Anspruch 1, wobei die erhöhte RAS-Aktivität durch eine aktivierende Mutation verursacht wird.

3. Rekombinantes Molekül zur Verwendung nach Anspruch 1 oder 2, wobei der Krebs ein Bauchspeicheldrüsenkrebs ist.

4. Rekombinantes Molekül zur Verwendung nach einem der Ansprüche 1 bis 3, wobei die Verwendung intraperitoneal erfolgt.

## Revendications

1. Molécule recombinante destinée à être utilisée dans le traitement d'un cancer comprenant une activité RAS accrue dans les cellules comparée à un état sain correspondant, la molécule recombinante comprenant un polypeptide cargo (C), un fragment enzymatique de toxine diphtérique (DTA) et un fragment de translocation de toxine diphtérique (DTB), comportant la structure générale :
x-C-y-DTA-DTB
dans laquelle :
x représente un polypeptide ou est absent,
C comprend une endopeptidase spécifique à Ras/Rap1 (RRSP) provenant de *Vibrio vulnificus* comportant la séquence décrite dans la SEQ ID n° : 30,
y comprend un lieur comprenant (G₄S)₂ (SEQ ID n° : 11),
le DTA consiste en des acides aminés comportant la séquence décrite dans la SEQ ID n° : 26, et
le DTB comprend des acides aminés comportant la séquence décrite dans la SEQ ID n° : 3,
ledit DTA étant lié au DTB par une liaison disulfure.

2. Molécule recombinante destinée à être utilisée selon la revendication 1, ladite activité RAS accrue étant provoquée par une mutation d'activation.

3. Molécule recombinante destinée à être utilisée selon la revendication 1 ou 2, ledit cancer étant un cancer du pancréas.

4. Molécule recombinante destinée à être utilisée selon l'une quelconque des revendications 1 à 3, ladite utilisation étant intrapéritonéale.
